# EUROPEAN PATENT APPLICATION

(11) **EP 3 136 462 A1**
(43) Date of publication of application: **01.03.2017**
(21) Application number: 15782884.9
(22) Date of filing: 13.04.2015
(51) Int. Cl.: H01L 51/50, C07D 213/06, C07F 15/00, C09K 11/06, H05B 33/12

(54) **LIGHT-EMITTING ELEMENT**

(30) Priority: 25.04.2014 JP 2014091611
(71) Applicant: Sumitomo Chemical Company Limited, Tokyo 104-8260 (JP); Cambridge Display Technology Limited, Cardinal Way Godmanchester Cambridgeshire PE29 2XG (GB)
(72) Inventor: MATSUMOTO, Ryuji, Tsukuba-shi Ibaraki 300-3294 (JP); STEUDEL, Regine, Annette, D-01309 Dresden (DE); WILSON, Richard, Godmanchester Cambridgeshire PE2 92XG (GB)
(74) Representative: Newman, Alastair James Morley
(86) International application number: PCT/JP2015/061318
(87) International publication number: WO 2015/163174

(57) **Abstract**

Provided is a light emitting device which is excellent in external quantum efficiency. The light emitting device comprises an anode, a cathode, a first light-emitting layer provided between the anode and the cathode, and a second light-emitting layer provided between the anode and the cathode. The first light-emitting layer is a layer obtained by using a polymer compound comprising a constitutional unit having a cross-linkable group and a phosphorescent constitutional unit, and the second light-emitting layer is a layer obtained by using a composition comprising a non-phosphorescent low molecular weight compound having a heterocyclic structure and at least two phosphorescent compounds.

## Description

### TECHNICAL FIELD

The present invention relates to a light emitting device and a polymer compound used in the light emitting device.

### BACKGROUND ART

A light emitting device is expected to be applied to a display or an illuminator. Regarding application to an illuminator, it has been known that a light emitting device to emit a white light can be produced by using at a predetermined ratio a blue light-emitting material, a green light-emitting material and a red light-emitting material (Patent Documents 1 and 2).

For manufacturing a large-area display or an illuminator at low cost, a technique of forming a functional layer necessary for the light emitting device by a wet process is required. For example, Patent Document 2 describes an all-phosphorescent white-light emitting device comprising a hole injection layer, a light-emitting layer and an electron transport layer all of which are formed by an application method.

### RELATED ART DOCUMENTS

### Patent Document

Patent Document 1: JP-A No. 2001-185357
Patent Document 2: International Publication WO 2007/114244

### DISCLOSURE OF INVENTION

### PROBLEM TO BE SOLVED BY THE INVENTION

However, regarding the conventional light emitting device, the external quantum efficiency has not been necessarily sufficient. An object of the present invention is to provide a light emitting device which is excellent in external quantum efficiency.

### MEANS FOR SOLVING PROBLEM

First, the present invention provides a light emitting device comprising:
an anode;
a cathode;
a first light-emitting layer provided between the anode and the cathode; and
a second light-emitting layer provided between the anode and the cathode, wherein
the first light-emitting layer is a layer obtained by using a polymer compound comprising a constitutional unit having a cross-linkable group and a phosphorescent constitutional unit, and
the second light-emitting layer is a layer obtained by using a composition comprising a non-phosphorescent low molecular weight compound having a heterocyclic structure and at least two phosphorescent compounds.

Second, the present invention provides a polymer compound comprising a constitutional unit having a group represented by the formula (13) and a phosphorescent constitutional unit: 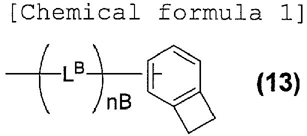 [wherein,
nB represents an integer of 1 to 5.
L^{B} represents an alkylene group, a cycloalkylene group, an arylene group, a divalent heterocyclic group, a group represented by -NR'-, an oxygen atom or a sulfur atom, and these groups optionally have a substituent. R' represents a hydrogen atom, an alkyl group, a cycloalkyl group, an aryl group or a monovalent heterocyclic group, and these groups optionally have a substituent. When a plurality of L^{B} are present, they may be the same or different.

The benzocyclobutene ring optionally has a substituent. When a plurality of the substituents are present, they may be the same or different, and they may be combined together to form a ring together with the carbon atoms to which they are attached.]

### EFFECT OF THE INVENTION

The present invention can provide a light emitting device which is excellent in external quantum efficiency. Moreover, a preferred embodiment of the present invention can provide a light emitting device which is excellent in luminous efficiency.

### Modes for Carrying Out the Invention

Suitable embodiments of the present invention will be illustrated in detail below.

### <Explanation of common term>

Terms commonly used in the present specification described below have the following meanings unless otherwise stated.

The term "obtained by using" referring to a relationship between a certain layer and a material for forming the layer denotes that the layer is formed by using the material, but it does not necessarily denotes that the material is contained while maintaining its state in the layer. For example, in the light emitting device of the present invention, the first light-emitting layer is formed by using a polymer compound comprising a constitutional unit having a cross-linkable group and a phosphorescent constitutional unit, but there is no necessity of containing the polymer compound as it is, because the polymer compound is crosslinked during layer formation, thereby forming a crosslinked polymer.

Regarding a group, a ring or a compound, the term "optionally have a substituent" denotes both a case where a hydrogen atom in the group, the ring or the compound is not substituted by a substituent, and a case where a part or all of hydrogen atoms in the group, the ring or the compound have been substituted by a substituent.

Me represents a methyl group, Et represents an ethyl group, Bu represents a butyl group, i-Pr represents an isopropyl group, and t-Bu represents a tert-butyl group.

The hydrogen atom may be a heavy hydrogen atom or a light hydrogen atom.

A solid line representing a bond to a central metal in a structural formula representing a metal complex denotes a covalent bond or a coordinate bond.

"Polymer compound" denotes a polymer having molecular weight distribution and having a polystyrene-equivalent number average molecular weight of 1×10³ to 1×10⁸.

A polymer compound may be any of a block copolymer, a random copolymer, an alternating copolymer and a graft copolymer, and may also be another embodiment.

An end group of a polymer compound is preferably a stable group because if a polymerization-active-group remains intact at the end, when the polymer compound is used for fabrication of a light emitting device, the light emitting property or luminance life possibly becomes lower. This end group is preferably a group having a conjugated bond to the main chain, and examples thereof include groups bonding to an aryl group or a monovalent heterocyclic group via a carbon-carbon bond.

"Low molecular weight compound" denotes a compound having no molecular weight distribution and having a molecular weight of 1×10⁴ or less.

"Constitutional unit" denotes a unit structure found once or more in a polymer compound.

"Halogen atom" denotes a fluorine atom, a chlorine atom, a bromine atom or an iodine atom.

"Alkyl group" may be one of linear or branched. The number of carbon atoms of the linear alkyl group is, not including the number of carbon atoms of a substituent, usually 1 to 50, preferably 3 to 30, more preferably 4 to 20 or 4 to 10. The number of carbon atoms of the branched alkyl groups is, not including the number of carbon atoms of a substituent, usually 3 to 50, preferably 3 to 30, more preferably 4 to 20 or 4 to 10. The alkyl group optionally has a substituent, and examples thereof include a methyl group, an ethyl group, a propyl group, an isopropyl group, a butyl group, an isobutyl group, a tert-butyl group, a pentyl group, an isoamyl group, 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group, a dodecyl group, and groups obtained by substituting a part or all of hydrogen atoms in these groups with a substituent. Although the substituent is described later, the preferable substituent which the alkyl group optionally has includes a cycloalkyl group, an alkoxy group, a cycloalkoxy group, an aryl group, a fluorine atom or the like, and these groups optionally further have a substituent. The alkyl group having a substituent includes a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-n-hexylphenyl) propyl group and a 6-ethyloxyhexyl group.

The number of carbon atoms of "Cycloalkyl group" is, not including the number of carbon atoms of a substituent, usually 3 to 50, preferably 3 to 30, more preferably 4 to 20 or 4 to 10. The cycloalkyl group optionally has a substituent, and examples thereof include a cyclopropyl group, a cyclobutyl group, a cyclopentyl group, a cyclohexyl group, and groups obtained by substituting a part or all of hydrogen atoms in these groups with a substituent. Although the substituent is described later, the preferable substituent which the cycloalkyl group optionally has includes a alkyl group, an alkoxy group, a cycloalkoxy group, an aryl group, a fluorine atom or the like, and these groups optionally further have a substituent. The cycloalkyl group having a substituent includes a methylcyclohexyl group and a ethylcyclohexyl group.

"Aryl group" denotes an atomic group remaining after removing from an aromatic hydrocarbon one hydrogen atom linked directly to a carbon atom constituting the ring. The number of carbon atoms of the aryl group is, not including the number of carbon atoms of a substituent, usually 6 to 60, preferably 6 to 30, more preferably 6 to 20, further preferably 6 to 18, 6 to 14 or 6 to 10. The aryl group optionally has a substituent, and examples thereof include a phenyl group, a 1-naphthyl group, a 2-naphthyl group, a 1-anthracenyl group, a 2-anthracenyl group, a 9-anthracenyl group, a 1-pyrenyl group, a 2-pyrenyl group, a 4-pyrenyl group, a 2-fluorenyl group, a 3-fluorenyl group, a 4-fluorenyl group, and groups obtained by substituting a part or all of hydrogen atoms in these groups with a substituent. Although the substituent is described later, the preferable substituent which the aryl group optionally has includes an alkyl group, a cycloalkyl group, an alkoxy group, a cycloalkoxy group, an aryl group, a fluorine atom or the like, and these groups optionally further have a substituent. The aryl group having a substituent includes a 2-phenylphenyl group, a 3-phenylphenyl group and a 4-phenylphenyl group.

"Alkoxy group" may be one of linear or branched. The number of carbon atoms of the linear alkoxy group is, not including the number of carbon atoms of a substituent, usually 1 to 40, preferably 1 to 20, more preferably 4 to 10. The number of carbon atoms of the branched alkoxy group is, not including the number of carbon atoms of a substituent, usually 3 to 40, preferably 3 to 20, more preferably 4 to 10. The alkoxy group optionally has a substituent, and examples thereof include a methoxy group, an ethoxy group, a propyloxy group, an isopropyloxy group, a butyloxy group, an isobutyloxy group, a tert-butyloxy group, a pentyloxy group, a hexyloxy group, a heptyloxy group, a octyloxy group, a 2-ethylhexyloxy group, a nonyloxy group, a decyloxy group, a 3,7-dimethyloctyloxy group, a lauryloxy group, and groups obtained by substituting a part or all of hydrogen atoms in these groups with a substituent, and these groups optionally further have a substituent. Although the substituent is described later, the preferable substituent which the alkoxy group optionally has includes a cycloalkyl group, an alkoxy group, a cycloalkoxy group, an aryl group, a fluorine atom or the like.

The number of carbon atoms of "Cycloalkoxy group" is, not including the number of carbon atoms of a substituent, usually 3 to 40, preferably 3 to 20, more preferably 4 to 10. The cycloalkoxy group optionally has a substituent, and examples thereof include a cyclopropyloxy group, a cyclobutyloxy group, a cyclopentyloxy group, cyclohexyloxy group, and groups obtained by substituting a part or all of hydrogen atoms in these groups with a substituent. Although the substituent is described later, the preferable substituent which the cycloalkoxy group optionally has includes an alkyl group, an alkoxy group, a cycloalkyl group, an aryl group, a fluorine atom or the like, and these groups optionally further have a substituent.

The number of carbon atoms of "Aryloxy group" is, not including the number of carbon atoms of a substituent, usually 6 to 60, preferably 6 to 48, more preferably 6 to 20, further preferably 6 to 18, 6 to 14 or 6 to 10. The aryloxy group optionally has a substituent, and examples thereof include a phenoxy group, a 1-naphthyloxy group, a 2-naphthyloxy group, a 1-anthracenyloxy group, a 9-anthracenyloxy group, a 1-pyrenyloxy group, and groups obtained by substituting a part or all of hydrogen atoms in these groups with a substituent. Although the substituent is described later, the preferable substituent which the aryloxy group optionally has includes an alkyl group, a cycloalkyl group, an alkoxy group, a cycloalkoxy group, a fluorine atom or the like, and these groups optionally further have a substituent.

"p-valent heterocyclic group" (p represents an integer of 1 or more) denotes an atomic group remaining after removing from a heterocyclic compound the p-number of hydrogen atoms linked directly to carbon atoms or hetero atoms constituting the ring. "Heterocyclic compound" denotes a non-aromatic heterocyclic compound such as oxirane, aziridine, azetidine, oxetane, thietane, pyrrolidine, tetrahydrofuran, dioxolane, imidazolidine, oxazolidine and piperidine, and an aromatic heterocyclic compound described later. Among the p-valent heterocyclic groups, a "p-valent aromatic heterocyclic group" is preferable. It is an atomic group remaining after removing from an aromatic heterocyclic compound the p-number of hydrogen atoms linked directly to carbon atoms or hetero atoms constituting a ring. "Aromatic heterocyclic compound" denotes a compound in which a heterocyclic ring itself exhibits aromaticity, and examples thereof include azole, diazole, triazole, oxadiazole, thiadiazole, thiazole, oxazole, thiophene, pyrrole, phosphole, furan, pyridine, diazabenzene (pyridazine, pyrimidine, pyrazine), triazine, azanaphthalene (quinoline, isoquinoline), diazanaphthalene (quinoxaline, quinazoline, etc.), carbazole, dibenzofuran, dibenzothiophene, dibenzosilole and acridine, and a compound in which although a heterocyclic ring does not exhibit aromaticity in itself, an aromatic ring is condensed with the heterocyclic ring, and examples thereof include phenoxazine, phenothiazine, dibenzoborole, dibenzosilole and benzopyran. The number of carbon atoms of the p-valent heterocyclic group is, not including the number of carbon atoms of a substituent, usually 2 to 60, preferably 3 to 20, and further preferably 3 to 15.

The monovalent heterocyclic group optionally has a substituent, and examples thereof include a thienyl group, a pyrrolyl group, a furyl group, a pyridyl group, a piperidyl group, a quinolyl group, an isoquinolyl group, a pyrimidinyl group, a triazinyl group, and groups obtained by substituting a part or all of hydrogen atoms in these groups with a substituent. Although the substituent is described later, the preferable substituent which the monovalent heterocyclic group optionally has includes an alkyl group, a cycloalkyl group, an alkoxy group, a cycloalkoxy group or the like, and these groups optionally further have a substituent.

"Substituted amino group" denotes an amino group obtained by substituting one or two hydrogen atoms with a substituent. Although the substituent is described later, the substituent which the amino group has is preferably an alkyl group, a cycloalkyl group, an aryl group or a monovalent heterocyclic group, and these groups optionally further have a substituent. The substituted amino group includes, for example, a dialkylamino group, a dicycloalkylamino group and a diarylamino group, and specific examples thereof include a dimethylamino group, a diethylamino group, a diphenylamino group, a bis(4-methylphenyl)amino group, a bis(4-tert-butylphenyl)amino group and a bis(3,5-di-tert-butylphenyl)amino group.

"Alkenyl group" may be one of linear or branched. The number of carbon atoms of the linear alkenyl group, not including the number of carbon atoms of the substituent, is usually 2 to 30, preferably 2 to 20, more preferably 3 to 20 or 3 to 10. The number of carbon atoms of the branched alkenyl group, not including the number of carbon atoms of the substituent, is usually 3 to 30, preferably 2 to 20, more preferably 4 to 20 or 4 to 10. The alkenyl group optionally has a substituent, and examples thereof include a vinyl group, a 1-propenyl group, a 2-propenyl group, a 2-butenyl group, a 3-butenyl group, a 3-pentenyl group, a 4-pentenyl group, a 1-hexenyl group, a 5-hexenyl group, a 7-octenyl group, and groups obtained by substituting a part or all of hydrogen atoms in these groups with a substituent. Although the substituent is described later, the preferable substituent which the alkenyl group optionally has includes a halogen atom, an aryl group, a monovalent heterocyclic group or the like, and these groups optionally further have a substituent.

The number of carbon atoms of "Cycloalkenyl group", not including the number of carbon atoms of the substituent, is usually 3 to 30, preferably 3 to 20, more preferably 4 to 20 or 4 to 10. The cycloalkenyl group optionally has a substituent, and examples thereof include a cyclopropenyl group, a cyclobutenyl group, a cyclopentenyl group, a cyclohexenyl group, a cyclooctenyl group, and groups obtained by substituting a part or all of hydrogen atoms in these groups with a substituent. Although the substituent is described later, the preferable substituent which the cycloalkenyl group optionally has includes an alkyl group, a halogen atom, an aryl group, a monovalent heterocyclic group or the like, and these groups optionally further have a substituent.

"Alkynyl group" may be any of linear or branched. The number of carbon atoms of the alkynyl group, not including the number of carbon atoms of the substituent, is usually 2 to 20, preferably 3 to 20, more preferably 3 to 10. The number of carbon atoms of the branched alkynyl group, not including the number of carbon atoms of the substituent, is usually 4 to 30, preferably 4 to 20, more preferably 4 to 10. The alkynyl group optionally has a substituent, and examples thereof include an ethynyl group, a 1-propynyl group, a 2-propynyl group, a 2-butynyl group, a 3-butynyl group, a 3-pentynyl group, a 4-pentynyl group, a 1-hexenyl group, a 5-hexenyl group, and groups obtained by substituting a part or all of hydrogen atoms in these groups with a substituent. Although the substituent is described later, the preferable substituent which the alkynyl group optionally has includes a halogen atom, an aryl group, a monovalent heterocyclic group or the like, and these groups optionally further have a substituent.

The number of carbon atoms of "Cycloalkynyl group", not including the number of carbon atoms of the substituent, is usually 3 to 30, preferably 4 to 20, more preferably 4 to 10. The cycloalkynyl group optionally has a substituent, and examples thereof include a cyclopropynyl group, a cyclobutynyl group, a cyclopentynyl group, a cyclohexenyl group, and groups obtained by substituting a part or all of hydrogen atoms in these groups with a substituent. Although the substituent is described later, the preferable substituent which the cycloalkynyl group optionally has includes an alkyl group, a halogen atom, an aryl group, a monovalent heterocyclic group or the like, and these groups optionally further have a substituent.

"Arylene group" denotes an atomic group remaining after removing from an aromatic hydrocarbon two hydrogen atoms linked directly to carbon atoms constituting the ring. The number of carbon atoms of the arylene group is, not including the number of carbon atoms of a substituent, usually 6 to 60, preferably 6 to 30, more preferably 6 to 20, further preferably 6 to 18, 6 to 14 or 6 to 10. The arylene group optionally has a substituent, and examples thereof include a phenylene group, a naphthalenediyl group, an anthracenediyl group, a phenanthrenediyl group, a dihydrophenanthrenediyl group, a naphthacenediyl group, a fluorenediyl group, a pyrenediyl group, a perylenediyl group, a chrysenediyl group, and groups obtained by substituting a part or all of hydrogen atoms in these groups with a substituent. Although the substituent is described later, the preferable substituent which the aryl group optionally has includes an alkyl group, a cycloalkyl group, an aryl group, a monovalent heterocyclic group or the like, and these groups optionally further have a substituent. The preferable example of the arylene group includes groups represented by the formulae (A-1) to (A-20). The group obtained by directly linking at least two of these groups is also preferable as the arylene group. 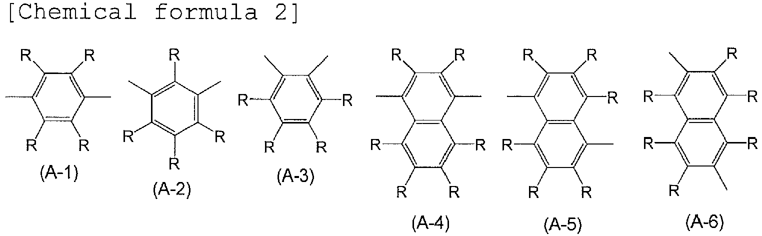 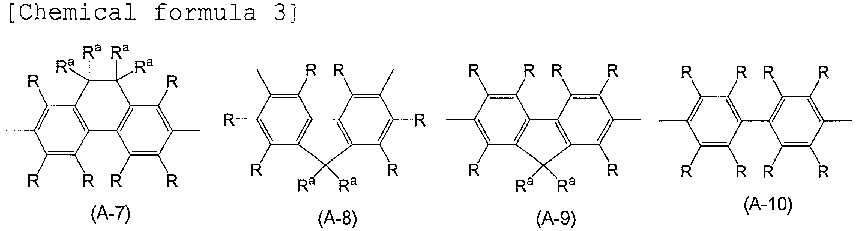 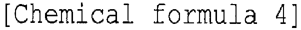 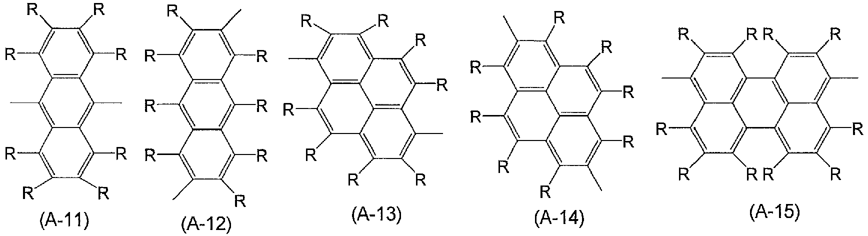 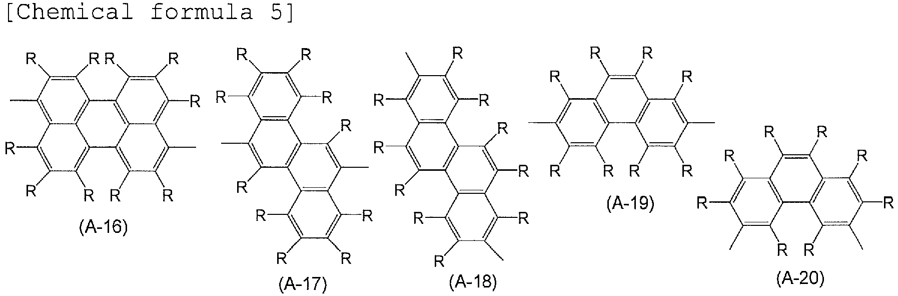 [wherein, R and R^{a} each independently represent a hydrogen atom, an alkyl group, a cycloalkyl group, an alkoxy group, a cycloalkoxy group, an aryl group or a monovalent heterocyclic group, and these groups optionally further have a substituent. The plurality of R and R^{a} each may be the same or different, and groups R^{a} may be combined together to form a ring together with the atoms to which they are attached.]

The divalent heterocyclic group optionally has a substituent, and examples thereof include a pyridinediyl group, a diazabenzenediyl group (pyridazinediyl group, pyrimidinediyl group, pyrazinediyl group), a triazinediyl group, an azanaphthalenediyl group (quinolinediyl group, isoquinolinediyl group), a diazanaphthalenediyl group (quinoxalinediyl group, quinazolinediyl group etc.), a carbazolediyl group, a dibenzofurandiyl group, a dibenzothiophenediyl group, a dibenzosilolediyl group, a phenoxazinediyl group, a phenothiazinediyl group, an acridinediyl group, dihydroacridinediyl group, a furandiyl group, a thiophenediyl group, an azolediyl group, a diazolediyl group, a triazolediyl group, and groups obtained by substituting a part or all of hydrogen atoms in these groups with a substituent. Although the substituent is described later, the preferable substituent which the divalent heterocyclic group optionally has include an alkyl group, a cycloalkyl group, aryl group, a monovalent heterocyclic group or the like, and these groups optionally further have a substituent. The preferable example of the divalent heterocyclic group includes groups represented by the formulae (AA-1) to (AA-34). The group directly linking at least two of these groups is also preferable as the divalent heterocyclic group. 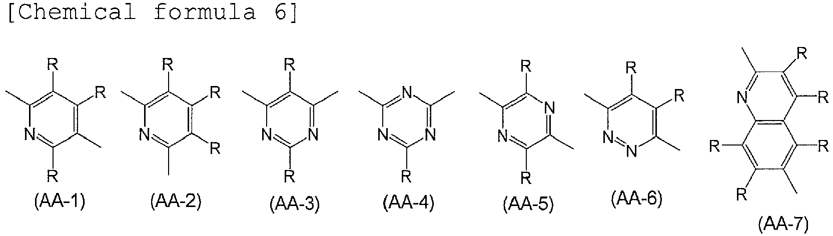 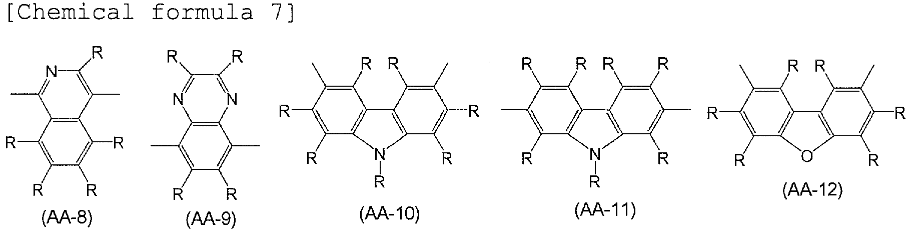 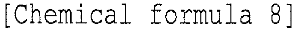 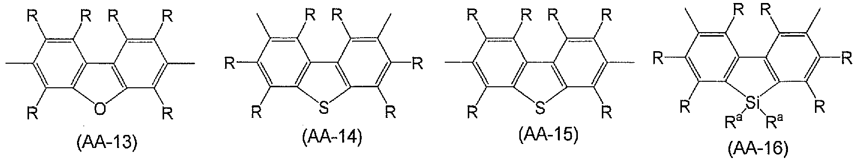 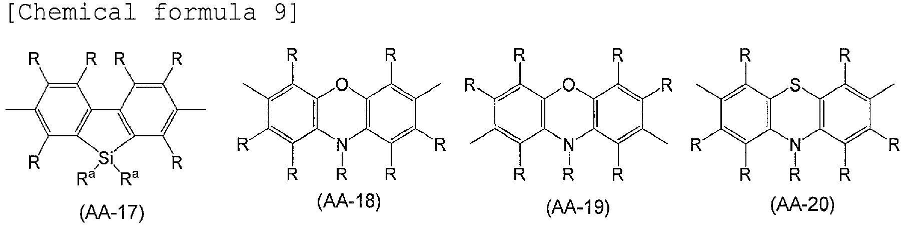 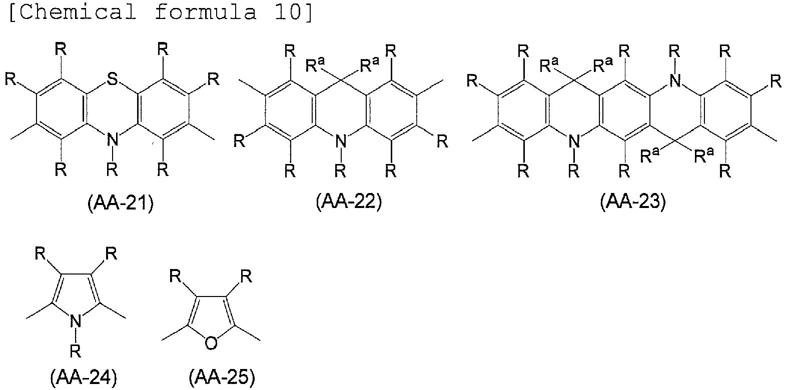 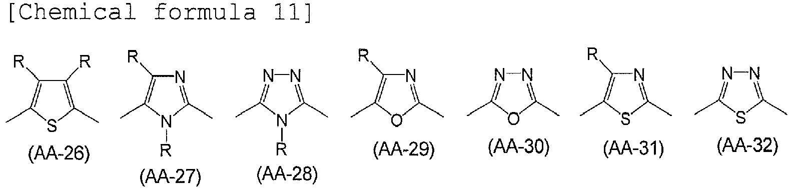 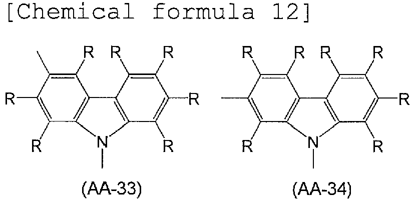 [wherein, R and R^{a} represent the same meaning as described above.]

"q-valent aromatic hydrocarbon group" (q represents an integer of 1 or more) denotes an atomic group remaining after removing from an aromatic hydrocarbon two hydrogen atoms linked directly to carbon atoms constituting the ring. The monovalent aromatic hydrocarbon group and the divalent aromatic hydrocarbon group each may also be called an aryl group and an arylene group, which are described above in detail. The number of carbon atoms of the q-valent aromatic hydrocarbon group is, not including the number of carbon atoms of a substituent, usually 6 to 60, preferably 6 to 30, more preferably 6 to 20 further preferably 6 to 18, 6 to 14, or 6 to 10.

"Cross-linkable group" is a group capable of forming a new bond by being subjected to a heating treatment, an ultraviolet irradiation treatment, a radical reaction and the like, and is preferably a group represented by the formulae (XL-1) to (XL-17) in the Group A of cross-linkable group.

"Substituent" represents a halogen atom, a cyano group, an alkyl group, a cylcoalkyl group, an aryl group, an alkoxy group, a cycloalkoxy group, an aryloxy group, a monovalent heterocyclic group, an amino group, a substituted amino group, an alkenyl group, a cycloalkenyl group, an alkynyl group or a cycloalkynyl group. The substutuent may also be a cross-linkable group. These groups optionally further have a substituent.

"Dendron" denotes a group having a regular dendritic branched structure having a branching point at an atom or a ring (in other words, a dendrimer structure). A compound having a dendron (hereinafter, referred to as "dendrimer") includes, for example, structures described in literatures such as International Publication WO 02/067343, JP-A No. 2003-231692, International Publication WO 2003/079736, International Publication WO 2006/097717 and the like.

The dendron is preferably a group represented by the formula (D-A) or (D-B). 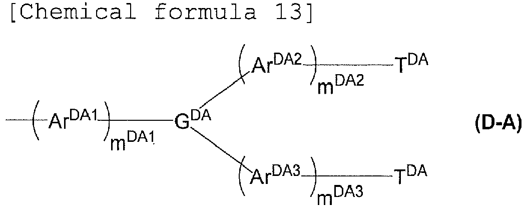 [wherein,
m^{DA1}, m^{DA2} and M^{DA3} each independently represent an integer of 0 or more.
G^{DA} represents a nitrogen atom, a trivalent aromatic hydrocarbon group or a trivalent heterocyclic group, and these groups optionally have a substituent.
Ar^{DA1}, Ar^{DA2} and Ar^{DA3} each independently represent an arylene group or a divalent heterocyclic group, and these groups optionally have a substituent. When a plurality of Ar^{DA1}, Ar^{DA2} and Ar^{DA3} are present, they may be the same or different at each occurrence.
T^{DA} represents an aryl group or a monovalent heterocyclic group, and these groups optionally have a substituent. The plurality of T^{DA} may be the same or different.] 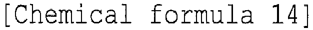 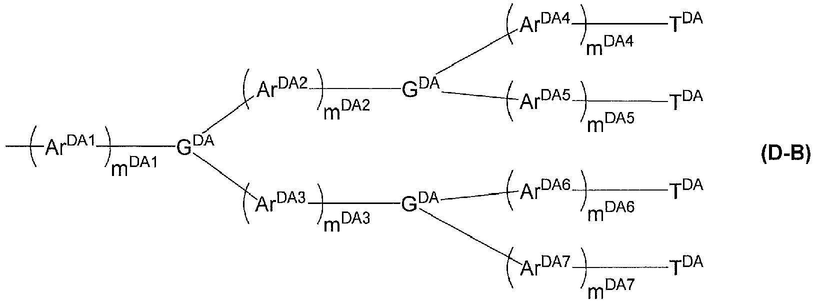
[wherein,
m^{DA1}, m^{DA2}, m^{DA3}, m^{DA4}, m^{DA5}, m^{DA6} and m^{DA7} each independently represent an integer of 0 or more.
G^{DA} represents a nitrogen atom, a trivalent aromatic hydrocarbon group or a trivalent heterocyclic group, and these groups optionally have a substituent. The plurality of G^{DA} may be the same or different.
Ar^{DA1}, Ar^{DA2}, Ar^{DA3} Ar^{DA4}, Ar^{DA5}, Ar^{DA6} and Ar^{DA7} each independently represent an arylene group or a divalent heterocyclic group, and these groups optionally have a substituent. When a plurality of Ar^{DA1}, Ar^{DA2}, Ar^{DA3}, Ar^{DA4}, Ar^{DA5}, Ar^{DA6} and Ar^{DA7} are present, they may be the same or different at each occurrence.
T^{DA} represents an aryl group or a monovalent heterocyclic group, and these groups optionally have a substituent. The plurality of T^{DA} may be the same or different.]

In the formula (D-A) and the formula (D-B), at least one of the plurality of T^{DA} is preferably an aryl group having as a substituent an alkyl group with the carbon number of 4 or more or a cycloalkyl group with the carbon number of 4 or more, or a monovalent heterocyclic group having as a substituent an alkyl group with the carbon number of 4 or more or a cycloalkyl group with the carbon number of 4 or more. More preferably, all of the plurality of T^{DA} are aryl groups each having as a substituent an alkyl group with the carbon number of 4 or more or a cycloalkyl group with the carbon number of 4 or more, or monovalent heterocyclic groups each having as a substituent an alkyl group with the carbon number of 4 or more or a cycloalkyl group with the carbon number of 4 or more.

m^{DA1} m^{DA2}, m^{DA3}, m^{DA4}, m^{DA5}, m^{DA6} and m^{DA7} are usually an integer of 10 or less, preferably an integer of 5 or less, more preferably 0 or 1. It is preferable that m^{DA1}, m^{DA2}, m^{DA3}, m^{DA4}, m^{DA5}, m^{DA6} and m^{DA7} are the same m^{DA1}, m^{DA2},m^{DA6} and m^{DA7} are the same integer.

A trivalent aromatic hydrocarbon group and a trivalent heterocyclic group represented by G^{DA} optionally have a substituent. The substituent is the same as described above. Examples of preferable substituents which the trivalent aromatic hydrocarbon group and the trivalent heterocyclic group represented by G^{DA} optionally have include an alkyl group, a cycloalkyl group, an alkoxy group, a cycloalkoxy group, an aryl group, a monovalent heterocyclic group and the like, and these groups optionally further have a substituent.

G^{DA} is preferably a trivalent aromatic hydrocarbon group or a trivalent heterocyclic group, and these groups optionally have a substituent. G^{DA} is more preferably a group represented by the formulae (GDA-11) to (GDA-15). 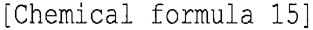 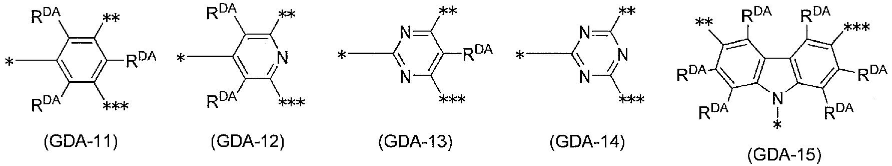 [wherein,
* represents a linkage to Ar^{DA1} in the formula (D-A), Ar^{DA1} in the formula (D-B), Ar^{DA2} in the formula (D-B) or Ar^{DA3} in the formula (D-B).
** represents a linkage to Ar^{DA2} in the formula (D-A), Ar^{DA2} in the formula (D-B), Ar^{DA4} in the formula (D-B) or Ar^{DA6} in the formula (D-B).
*** represents a linkage to Ar^{DA3} in the formula (D-A), Ar^{DA3} in the formula (D-B), Ar^{DA5} in the formula (D-B) or Ar^{DA7} in the formula (D-B).
R^{DA} represents a hydrogen atom, an alkyl group, a cycloalkyl group, an alkoxy group, a cycloalkoxy group, an aryl group or a monovalent heterocyclic group, and these groups optionally further have a substituent. When a plurality of R^{DA} are present, they may be the same or different.]

R^{DA} is preferably a hydrogen atom, an alkyl group, a cycloalkyl group, an alkoxy group or a cycloalkoxy group, more preferably a hydrogen atom, an alkyl group or cycloalkyl group, and these groups optionally have a substituent.

It is preferable that Ar^{DA1}, Ar^{DA2}, Ar^{DA3}, Ar^{DA4}, Ar^{DA5}, Ar^{DA6} and Ar^{DA7} are groups represented by the formulae (ArDA-1) to (ArDA-3). 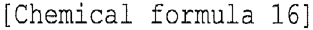 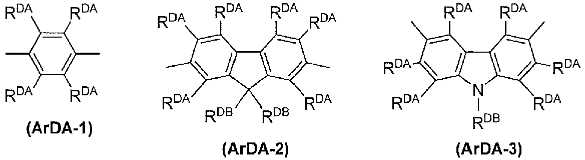 [wherein,
R^{DA} represents the same meaning as described above.
R^{DB} represents a hydrogen atom, an alkyl group, a cycloalkyl group, an aryl group or a monovalent heterocyclic group, and these groups optionally have a substituent. When a plurality of R^{DB} are present, they may be the same or different.]

R^{DB} is preferably an alkyl group, a cycloalkyl group, an aryl group or a monovalent heterocyclic group, more preferably an aryl group or a monovalent heterocyclic group, further preferably an aryl group, and these groups optionally have a substituent.

T^{DA} is preferably groups represented by the formulae (TDA-1) to (TDA-3). 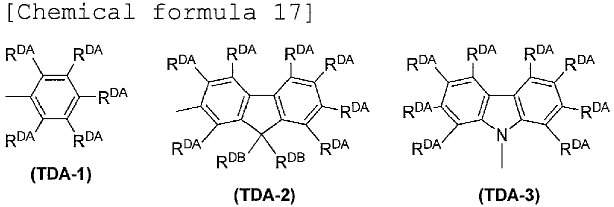 [wherein, R^{DA} and R^{DB} represent the same meaning as described above.]

The group represented by the formula (D-A) is preferably a group represented by the formulae (D-A1) to (D-A3). 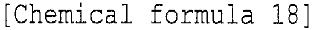 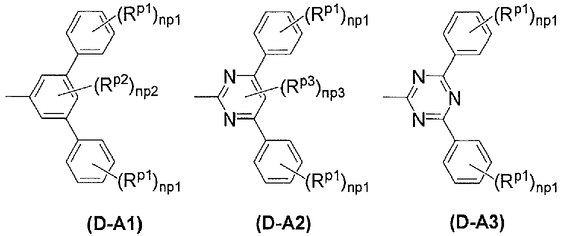 [wherein,
R^{p1}, R^{p2} and R^{p3} each independently represent an alkyl group, a cycloalkyl group, an alkoxy group, a cycloalkoxy group or a halogen atom, and these groups optionally have a substituent. When a plurality of R^{p1} and R^{p2} are present, they may be the same or different at each occurrence.
np1 represents an integer of 0 to 5, np2 represents an integer of 0 to 3, and np3 represents 0 or 1. The plurality of np1 may be the same or different.]

The group represented by the formula (D-B) is preferably a group represented by the formulae (D-B1) to (D-B3). 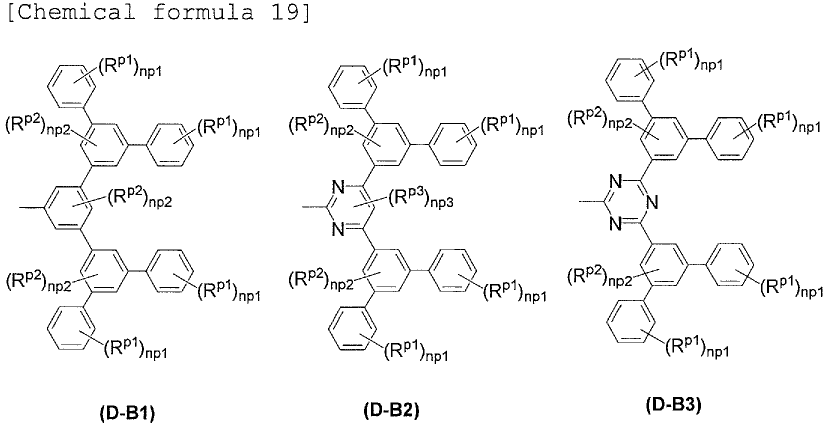 [wherein,
R^{p1}, R^{p2} and R^{p3} each independently represent an alkyl group, a cycloalkyl group, an alkoxy group, a cycloalkoxy group or a halogen atom, and these groups optionally have a substituent. When a plurality of R^{p1} and R^{p2} are present, they may be the same or different at each occurrence.
np1 represents an integer of 0 to 5, np2 represents an integer of 0 to 3, and np3 represents 0 or 1. When a plurality of np1 and np2 are present, they may be the same or different at each occurrence.]

np1 is preferably 0 or 1, more preferably 1. np2 is preferably 0 or 1, more preferably 0. np3 is preferably 0.

R^{p1}, R^{p2} and R^{p3} are preferably an alkyl group or a cycloalkyl group, and these groups optionally have a substituent.

Among the formulae (D-A1) to (D-A3) and the formulae (D-B1) to (D-B3), at least one of the plurality of R^{p1} is preferably an alkyl group with the carbon number of 4 or more or a cycloalkyl group with the carbon number of 4 or more. It is preferable that all of the plurality of R^{p1} are alkyl groups with the carbon number of 4 or more or cycloalkyl groups with the carbon number of 4 or more.

"r-membered aromatic carbon ring" (r is 5 or 6) denotes an aromatic carbon ring including an r-membered ring in its structure. For example, a six-membered aromatic carbon ring may be a monocyclic aromatic carbon ring (benzene ring) or a polycyclic aromatic carbon ring (naphthalene ring, anthracene ring, phenanthrene ring, fluorene ring or the like) as long as it contains a six-membered ring. This holds true for an "r-membered aromatic heterocyclic ring".

### [Light emitting device]

A light emitting device of the present invention is characterized in that it comprises an anode, a cathode, a first light-emitting layer provided between the anode and the cathode, and a second light-emitting layer provided between the anode and the cathode. The first light-emitting layer is a layer obtained by using a polymer compound comprising a constitutional unit having a cross-linkable group and a phosphorescent constitutional unit, and the second light-emitting layer is a layer obtained by using a composition comprising a non-phosphorescent low molecular weight compound having a heterocyclic structure and at least two phosphorescent compounds.

### <First light-emitting layer>

A first light-emitting layer is a layer obtained by using a polymer compound comprising a constitutional unit having a cross-linkable group and a phosphorescent constitutional unit.

The constitutional unit having a cross-linkable group is preferably a constitutional unit having at least one kind of cross-linkable group selected from the Group A of cross-linkable group.

### (Group A of cross-linkable group)

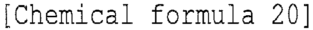

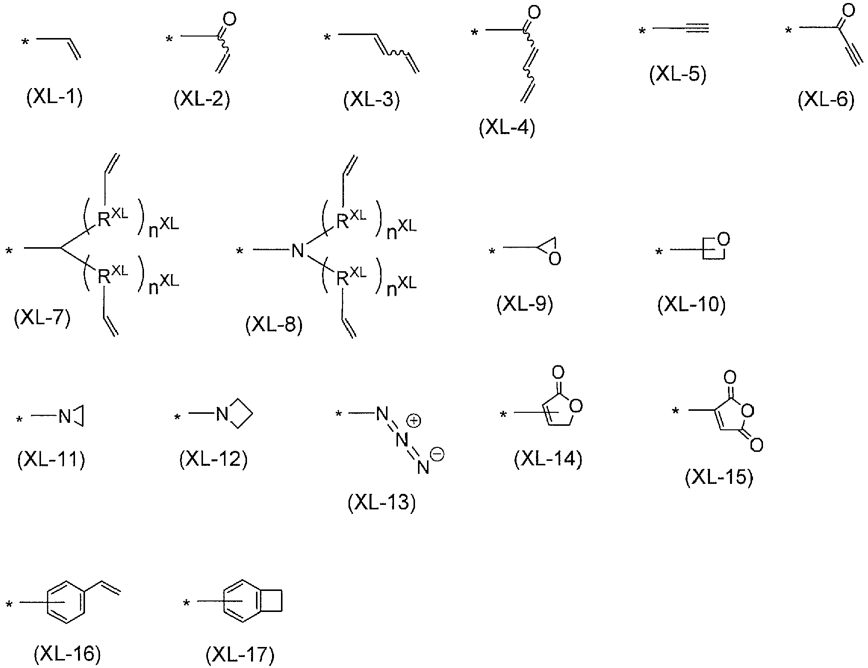

[wherein, R^{XL} represents a methylene group, an oxygen atom or a sulfur atom, and n^{XL} represents an integer of 0 to 5. When a plurality of R^{XL} are present, they may be the same or different. * represents a linkage position. These cross-linkable groups optionally have a substituent.]

For the cross-linkable group, a cross-linkable group represented by the formula (XL-1), the formula (XL-3), the formula (XL-5), the formula (XL-7), the formula (XL-16) or the formula (XL-17) is preferable, and a cross-linkable group represented by the formula (XL-1) or the formula (XL-17) is further preferable, because a polymer compound which is excellent in crosslinkability can be obtained. Though the constitutional unit having a cross-linkable group is preferably at least one constitutional unit selected from the group consisting of a constitutional unit represented by the formula (11) and a constitutional unit represented by the formula (12) described later, it may be a constitutional unit represented by the following formulae. The constitutional units represented by the following formulae optionally have a substituent.

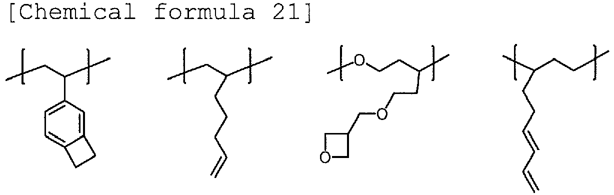

The constitutional unit having a cross-linkable group is preferably at least one type of constitutional unit selected from the group consisting of a constitutional unit represented by the formula (11) and a constitutional unit represented by the formula (12). In particular, the constitutional unit having a cross-linkable group is preferably at least one type of constitutional unit selected from the group consisting of the constitutional unit represented by the formula (11).

### -Constitutional unit represented by the formula (11)-

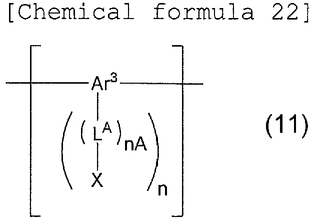

[wherein,
nA represents an integer of 0 to 5, n represents 1 or 2. When a plurality of nA are present, they may be the same or different.
Ar³ represents a (n+2)-valent aromatic hydrocarbon group or a (n+2)-valent heterocyclic group, and the groups optionally have a substituent.
L^{A} represents an alkylene group, a cycloalkylene group, an arylene group, a divalent heterocyclic group, a group represented by -NR'-, an oxygen atom or a sulfur atom, and these groups optionally have a substituent. R' represents a hydrogen atom, an alkyl group, a cycloalkyl group, an aryl group or a monovalent heterocyclic group, and these groups each optionally have a substituent. When a plurality of L^{A} are present, they may be the same or different.
X represents a cross-linkable group selected from the Group A of cross-linkable group. When a plurality of X are present, they may be the same or different.]

nA is preferably an integer of 1 to 5, and more preferably 1 or 2, because a light emitting device which is more excellent in the luminance life can be produced.

n is preferably 2, because a light emitting device which is more excellent in the luminance life can be produced.

Ar³ is preferably a (n+2)-valent aromatic hydrocarbon group optionally having a substituent, because a light emitting device which is more excellent in the luminance life can be produced.

The (n+2)-valent aromatic hydrocarbon group and the (n+2)-valent heterocyclic group each represented by Ar³ optionally have a substituent. The preferable substituents may be an alkyl group, a cycloalkyl group, an alkoxy group, a cycloalkoxy group, an aryl group, an aryloxy group, a halogen atom, a monovalent heterocyclic group or a cyano group, and these groups optionally further have a substituent.

The (n+2)-valent aromatic hydrocarbon group represented by Ar³ is preferably a group represented by the formulae (A-1) to (A-20) (the n-number of R and R^{a} are atomic bonding), more preferably a group represented by the formula (A-1), the formula (A-2), the formulae (A-6) to (A-10), the formula (A-19) or the formula (A-20) (the n-number of R and R^{a} are atomic bonding), and further preferably a group represented by the formula (A-1), the formula (A-2), the formula (A-7), the formula (A-9) or the formula (A-19) (the n-number of R and R^{a} are atomic bonding).

The (n+2)-valent heterocyclic group represented by Ar³ is preferably a group represented by the formulae (AA-1) to (AA-34) (the n-number of R and R^{a} are atomic bonding).

The number of carbon atoms of the alkylene group represented by L^{A} is, not including the number of carbon atoms of a substituent, usually 1 to 10, preferably 1 to 5, and more preferably 1 to 3. The number of carbon atoms of the cycloalkylene group represented by L^{A} is, not including the number of carbon atoms of a substituent, usually 3 to 10. The alkylene group and the cycloalkylene group optionally have a substituent, and the examples include a methylene group, an ethylene group, a propylene group, a butylene group, a hexylene group, a cyclohexylene group, an octylene group, and groups obtained by substituting a part or all of hydrogen atoms in these groups with a substituent. Although the substituent is the same as described above, examples of preferable substituents which the alkylene group and the cycloalkylene group each represented by L^{A} optionally have include an alkyl group, a cycloalkyl group, an alkoxy group, a cycloalkoxy group, a halogen atom and a cyano group, and these groups optionally further have a substituent.

Preferable examples of the arylene group represented by L^{A} include o-phenylene, m-phenylene, p-phenylene, and groups obtained by substituting a part or all of hydrogen atoms in these groups with a substituent. Although the substituent is the same as described above, examples of the preferable substituent which the arylene group represented by L^{A} optionally has include an alkyl group, a cycloalkyl group, an alkoxy group, a cycloalkoxy group, an aryl group, an aryloxy group, a monovalent heterocyclic group, a halogen atom, a cyano group and a cross-linkable group selected from the Group A of cross-linkable group, and these groups optionally further have a substituent.

L^{A} is preferably an alkylene group or an arylene group, more preferably an alkylene group or a phenylene group, and these groups each optionally have a substituent, because the polymer compound is manufactured easily.

The cross-linkable group represented by X is preferably a cross-linkable group represented by the formula (XL-1), the formula (XL-3), the formula (XL-5), the formula (XL-7), the formula (XL-16) or the formula (XL-17), and more preferably a cross-linkable group represented by the formula (XL-1) or the formula (XL-17), because a polymer compound which is excellent in crosslinkability can be obtained.

The constitutional unit represented by the formula (11) includes, for example, a constitutional unit represented by the formulae (11-1) to (11-30). Among them, preferable examples may be a constitutional unit represented by the formula (11-1) to the formula (11-15), the formula (11-19), the formula (11-20), the formula (11-23), the formula (11-25) or the formula (11-30), more preferably a constitutional unit represented by the formulae (11-1) to (11-9) or the formula (11-30), because a polymer compound which is excellent in crosslinkability can be obtained.

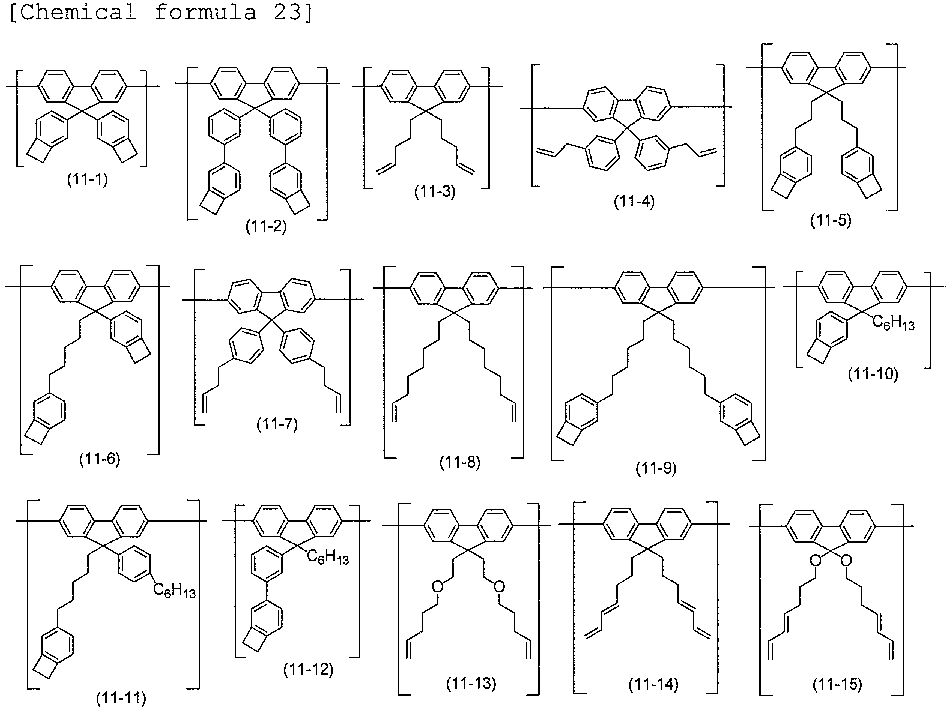

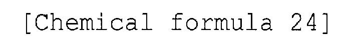

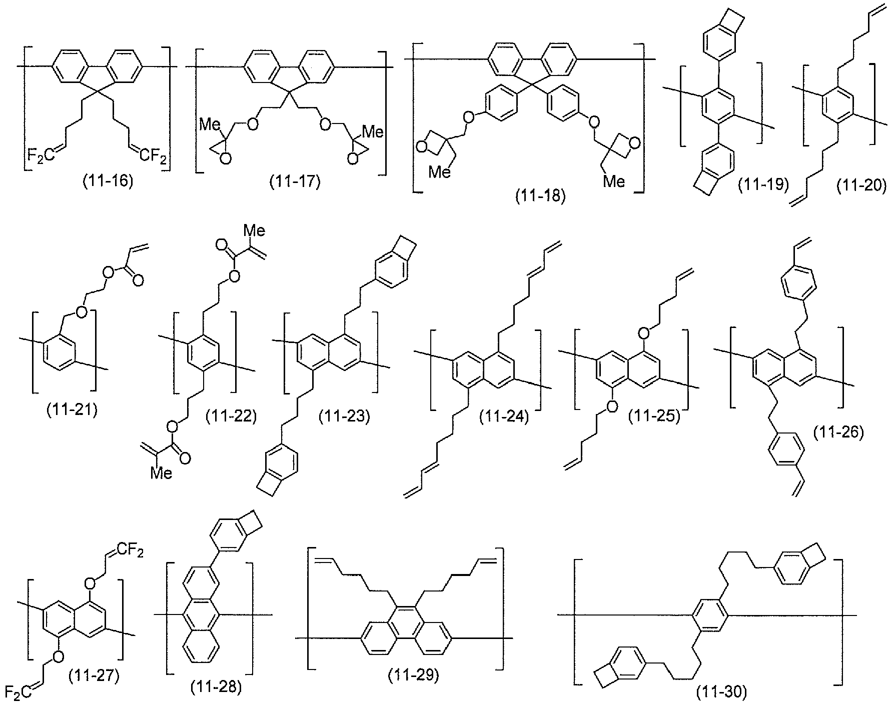

The amount of the constitutional unit represented by the formula (11) contained in the polymer compound is preferably 0.5 to 40 mol%, more preferably 5 to 40 mol%, further preferably 10 to 40 mol% with respect to the total amount of 100 mol% of the constitutional units contained in the polymer compound, because a polymer compound which is excellent in stability and crosslinkability can be obtained.

The constitutional unit represented by the formula (11) may be contained only singly or two or more units thereof may be contained in the polymer compound.

### -Constitutional unit represented by the formula (12)-

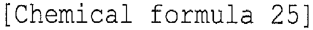

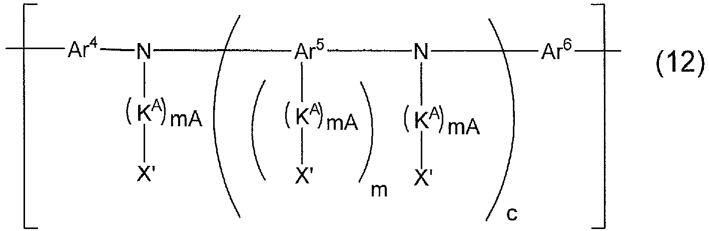

[wherein,
mA represents an integer of 0 to 5, m represents an integer of 1 to 4, and c represents an integer of 0 or 1. When a plurality of mA are present, they may be the same or different.
Ar⁵ represents a (m+2)-valent aromatic hydrocarbon group, a (m+2)-valent heterocyclic group or a (m+2)-valent group in which at least one aromatic carbon ring and at least one heterocyclic ring are bonded directly to each other, and these groups each optionally have a substituent.
Ar⁴ and Ar⁶ each independently represent an arylene group or a divalent heterocyclic group, and these groups optionally further have a substituent.
Each of Ar⁴, Ar⁵ and Ar⁶ each may be bonded directly or via an oxygen atom or a sulfur atom to a group that is different from that group and that is attached to the nitrogen atom to which that group is attached, thereby forming a ring.
K^{A} represents an alkylene group, a cycloalkylene group, an arylene group, a divalent heterocyclic group, a group represented by -NR'-, an oxygen atom or a sulfur atom, and these groups optionally have a substituent. R' represents the same meaning as described above. When a plurality of K^{A} are present, they may be the same or different.
X' represents a cross-linkable group selected from the Group A of cross-linkable group, a hydrogen atom, an alkyl group, a cycloalkyl group, an aryl group or a monovalent heterocyclic group, and these groups optionally have a substituent. When a plurality of X' are present, they may be the same or different. At least one X' is a cross-linkable group selected from the Group A of cross-linkable group.]

mA is preferably 0 or 1, and more preferably 0, because a light emitting device which is more excellent in the luminance life can be produced.

m is preferably 2, because a light emitting device which is more excellent in the luminance life can be produced.

c is preferably 0, because a polymer compound is easily produced and the light emitting device which is more excellent in the luminance life can be produced.

Ar⁵ is preferably a (m+2)-valent aromatic hydrocarbon group optionally having a substituent, because a light emitting device which is more excellent in the luminance life can be produced.

The group represented by Ar⁵ optionally has a substituent. Examples of the preferable substituent include an alkyl group, a cycloalkyl group, an alkoxy group, a cycloalkoxy group, an aryl group, an aryloxy group, a halogen atom, a monovalent heterocyclic group and a cyano group, and these groups optionally further have a substituent.

The (m+2)-valent aromatic hydrocarbon group represented by Ar⁵ is preferably a group represented by the formula (A-1), the formula (A-6), the formula (A-7), the formulae (A-9) to (A-11) or a group represented by the formula (A-19) (the m-number of R and R^{a} are atomic bonding).

The (m+2)-valent heterocyclic group represented by Ar⁵ is preferably a group represented by the formula (AA-1), the formula (AA-2), or the formulae (AA-7) to (AA-26) (the m-number of R and R^{a} are atomic bonding).

The number of carbon atoms of the (m+2)-valent group in which at least one aromatic carbon ring and at least one heterocyclic ring are bonded directly to each other represented by Ar⁵ is, not including the number of carbon atoms of a substituent, usually 10 to 80, preferably 10 to 60, and more preferably 12 to 28. The (m+2)-valent group is a group in which at least one of the groups represented by the formula (A-1) and the formula (A-9) and at least one of the groups represented by the formula (AA-1), the formula (AA-2), the formula (AA-4) and the formulae (AA-7) to (AA-26) are bonded directly to each other (in the thus formed group, the m-number of R and R^{a} are atomic bonding).

The Ar⁴ and Ar⁶ each are preferably an arylene group optionally having a substituent, because a light emitting device which is more excellent in the luminance life can be produced.

The group represented by Ar⁴ and Ar⁶ optionally have a substituent. Examples of preferable substituent include an alkyl group, a cycloalkyl group, an alkoxy group, a cycloalkoxy group, an aryl group, an aryloxy group, a halogen atom, a monovalent heterocyclic group and a cyano group, and these groups optionally further have a substituent.

The arylene group represented by Ar⁴ and Ar⁶ is preferably a group represented by the formula (A-1) or the formula (A-9), and more preferably a group represented by the formula (A-1).

The divalent heterocyclic groups represented by Ar⁴ and Ar⁶ are preferably groups represented by the formula (AA-1), the formula (AA-2), the formula (AA-7) to the formula (AA-26).

The alkylene group, the cycloalkylene group and the arylene group each represented by K^{A} are the same as described above in the explanation of L^{A}.

K^{A} is preferably an alkylene group or an arylene group, and more preferably a methylene group or a phenylene group, because the polymer compound is produced easily, and these groups each optionally have a substituent.

The cross-linkable group represented by X' is preferably a cross-linkable group represented by the formula (XL-1), the formula (XL-3), the formula (XL-5), the formula (XL-7), the formula (XL-16) or the formula (XL-17), and more preferably a cross-linkable group represented by the formula (XL-1) or the formula (XL-17), because a polymer compound which is excellent in crosslinkability can be obtained.

The constitutional unit represented by the formula (12) includes, for example, the constitutional units represented by the formula (12-1) to the formula (12-13).

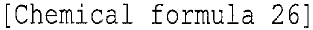

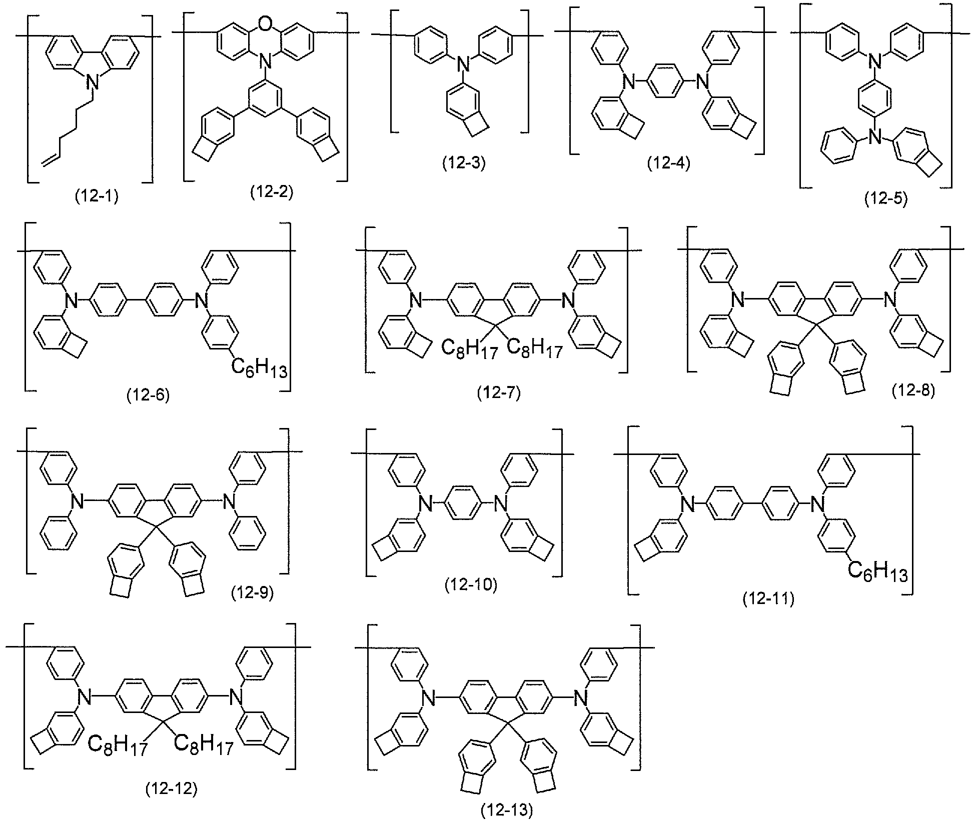

The constitutional unit represented by the formula (12) may be contained only singly or two or more units thereof may be contained in the polymer compound.

The polymer compound used in the first light-emitting layer comprises a phosphorescent constitutional unit together with a constitutional unit having the above-described cross-linkable group. The phosphorescent constitutional unit denotes a constitutional unit having a group that is formed by removing from the phosphorescent compound a hydrogen atom linked directly to a carbon atom or a hetero atom constituting the compound.

The phosphorescent constitutional unit is preferably at least one unit selected from the group of constitutional units represented by the formula (1G), the formula (2G), the formula (3G) and the formula (4G). 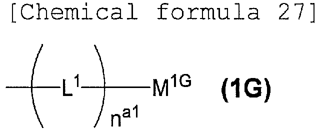 [wherein,
M^{1G} represents a group formed by removing from a phosphorescent compound one hydrogen atom linked directly to a carbon atom or a hetero atom constituting the compound.
L¹ represents an oxygen atom, a sulfur atom, a group represented by -N(R^{A})-, a group represented by -C(R^{B})2-_{,} a group represented by -C(R^{B})=C(R^{B})-, a group represented by -C=C-, an arylene group or a divalent heterocyclic group, and these groups optionally have a substituent. R^{A} represents a hydrogen atom, an alkyl group, a cycloalkyl group, an aryl group or a monovalent heterocyclic group, and these groups optionally have a substituent. R^{B} represents a hydrogen atom, an alkyl group, a cycloalkyl group, an alkoxy group, a cycloalkoxy group, an aryl group or a monovalent heterocyclic group, and these groups optionally have a substituent. The plurality of R^{B} may be the same or different, and they may be combined together to form a ring together with the carbon atoms to which they are attached. When a plurality of L¹ are present, they may be the same or different.
n^{a1} represents an integer of 0 or more.]

R^{A} is preferably an aryl group or a monovalent heterocyclic group, more preferably an aryl group, and these groups optionally have a substituent.

R^{B} is preferably an alkyl group, a cycloalkyl group, an aryl group or a monovalent heterocyclic group, more preferably a hydrogen atom, an alkyl group, a cycloalkyl group or an aryl group, further preferably a hydrogen atom or an alkyl group, and particularly preferably a hydrogen atom, and these groups optionally have a substituent.

L¹ is preferably a group represented by -C(R^{B})₂-, an arylene group or a divalent heterocyclic group, more preferably a group represented by -C(R^{B})₂- or an arylene group, further preferably an arylene group, and these groups optionally have a substituent. In particular, a group represented by the formula (A-1) or the formula (A-2) is preferable.

Examples of preferable substituents which R^{A}, R^{B} and L¹ optionally have include an alkyl group, a cycloalkyl group, an alkoxy group, a cycloalkoxy group, an aryl group, an aryloxy group, a monovalent heterocyclic group, a halogen atom or the like, and these groups optionally further have a substituent.

n^{a1} is usually an integer of 0 to 10, preferably an integer of 0 to 5, more preferably an integer of 0 to 2, further preferably 0 or 1, and particularly preferably 0.

When the polymer compound comprises a constitutional unit represented by the formula (1G), the constitutional unit represented by the formula (1G) is a terminal constitutional unit.

"Terminal constitutional unit" denotes a terminal constitutional unit of a polymer compound, and the terminal constitutional unit is preferably a constitutional unit derived from a terminal blocking agent in production of the polymer compound.

M^{1G} is preferably a group represented by the formula (GM-1). 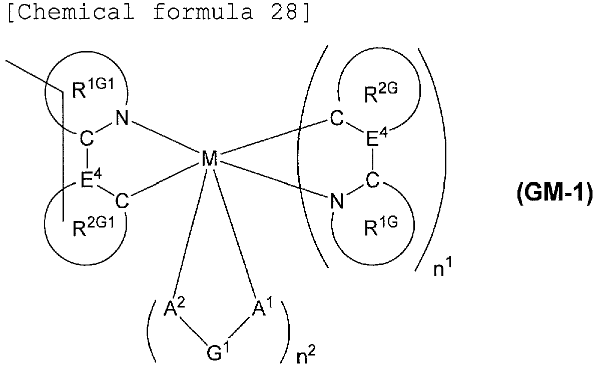 [wherein,
M represents a ruthenium atom, a rhodium atom, a palladium atom, an iridium atom or a platinum atom.
n¹ represents an integer of 1 or more. n² represents an integer of 0 or more. n¹+n² is 1 or 2. When M is a ruthenium atom, a rhodium atom or an iridium atom, n¹+n² is 2. When M is a palladium atom or a platinum atom, n¹+n² is 1.
E⁴ represents a carbon atom or a nitrogen atom. The plurality of E⁴ may be the same or different.

A ring R^{1G} and a ring R^{1G1} represent a six-membered aromatic heterocyclic ring, and these rings optionally have a substituent. When a plurality of the substituents are present, they may be the same or different, and they may be combined together to form a ring together with the atoms to which they are attached. When a plurality of rings R^{1G} are present, they may be the same or different.

The ring R^{2G} and a ring R^{2G1} represent a five-membered or six-membered aromatic carbon ring, or a five-membered or six-membered aromatic heterocyclic ring, and these rings optionally have a substituent. When a plurality of the substituents are present, they may be the same or different, and they may be combined together to form a ring together with the atoms to which they are attached. When a plurality of rings R^{2G} are present, they may be the same or different. E⁴ is a carbon atom when the ring R^{2G} is a six-membered aromatic heterocyclic ring.

Either the ring R^{1G1} or the ring R^{2G1} has one atomic bonding.

A¹-G¹-A² represents an anionic bidentate ligand. A¹ and A² each independently represent a carbon atom, an oxygen atom or a nitrogen atom, and these atoms may be atoms constituting a ring. G¹ represents a single bond or an atomic group constituting the bidentate ligand together with A¹ and A². When a plurality of A¹-G¹-A² are present, they may be the same or different.]

When M is a ruthenium atom, a rhodium atom or an iridium atom, n² is 0 or 1, and more preferably 0.

When M is a palladium atom or a platinum atom, n² is 0.

The ring R^{1G} is preferably a six-membered aromatic heterocyclic ring having 1 to 4 nitrogen atoms as constitutional atoms, more preferably a six-membered aromatic heterocyclic ring having 1 to 2 nitrogen atoms as constitutional atoms, further preferably a pyridine ring, a diazabenzene ring, a quinoline ring or an isoquinoline ring, particularly preferably a pyridine ring, a quinoline ring or an isoquinoline, and these rings optionally have a substituent.

The ring R^{2G} is preferably a six-membered aromatic carbon ring, or a five-membered or six-membered aromatic heterocyclic ring, more preferably a benzene ring, a naphthalene ring, a fluorene ring, a phenanthrene ring, a pyridine ring, a diazabenzene ring, a pyrrole ring, a furan ring or a thiophene ring, further preferably a benzene ring, a naphthalene ring or a fluorene ring, particularly preferably a benzene ring, and these rings optionally have a substituent.

Substituents which the ring R^{1G} and the ring R^{2G} optionally have may be preferably an alkyl group, a cycloalkyl group, an aryl group, a monovalent heterocyclic group, an alkoxy group, a cycloalkoxy group, an aryloxy group, or a dendron, more preferably an alkyl group, a cycloalkyl group, an aryl group, a monovalent heterocyclic group or a dendron, further preferably an alkyl group, a cycloalkyl group, an aryl group or a dendron, particularly preferably an alkyl group, an aryl group or a dendron, and these groups optionally further have a substituent. When a plurality of substituents are present in the ring R^{1G} and the ring R^{2G}, they may be the same or different, and they may be combined together to form a ring together with the atoms to which they are attached.

More preferably at least one ring selected from the group consisting of the ring R^{1G} and the ring R^{2G} is a phosphorescent compound having a dendron.

The number of dendrons which at least one of the ring R^{1G} and the ring R^{2G} has is preferably 1 to 3, more preferably 1 or 2, and further preferably 1.

When the dendron which at least one of the ring R^{1G} and the ring R^{2G} has is a group represented by the formula (D-A) or the formula (D-B) and when m^{DA1}is an integer of 1 or more, Ar^{DA1} to be bonded to the ring R^{1G} and/or the ring R^{2G} is preferably a group represented by the formula (ArDA-1).

When the dendron which at least one of the ring R^{1G} and the ring R^{2G} has is a group represented by the formula (D-A) or the formula (D-B) and when m^{DA1} is 0, G^{DA} bonded to the ring R^{1G} and/or the ring R^{2G} is preferably a group represented by the formula (GDA-11), the formula

(GDA-12), the formula (GDA-14) or the formula (GDA-15), and more preferably a group represented by the formula (GDA-11) or the formula (GDA-15).

The dendron which at least one of the R^{1G} and the ring R^{2G} has is preferably a group represented by the formula (D-A1), the formula (D-A3), the formula (D-B1) or the formula (D-B3), and more preferably a group represented by the formula (D-A1) or the formula (D-A3).

In the formula (GM-1), at least one ligand (ligand represented by ring R^{1G}-R^{2G}) whose number is defined with a subscript n¹ is preferably a ligand represented by the formula (GM-L1) to the formula (GM-L4), and more preferably a ligand represented by the formula (GM-L1) or the formula (GM-L2). 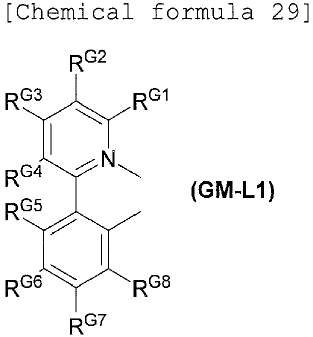 [wherein,
R^{G1} to R^{G8} each independently represent a hydrogen atom, an alkyl group, a cycloalkyl group, an aryl group, a monovalent heterocyclic group, an alkoxy group, a cycloalkoxy group, an aryloxy group or a dendron, and these groups optionally have a substituent. R^{G1} and R^{G2}, R^{G2} and R^{G3}, R^{G3} and R^{G4}, R^{G4} and R^{G5}, R^{G5} and R^{G6}, R^{G6} and R^{G7}, and, R^{G7} and R^{G8} each may be combined together to form a ring together with the atoms to which they are attached.]

R^{G1}, R^{G4} , R^{C5} and R^{G8} are preferably a hydrogen atom, an alkyl group, a cycloalkyl group, an aryl group, a monovalent heterocyclic group, an alkoxy group or a cycloalkoxy group, more preferably a hydrogen atom, an alkyl group or an aryl group, further preferably a hydrogen atom or an alkyl group, particularly preferably a hydrogen atom, and these groups optionally have a substituent.

R^{G2}, R^{G3} , R^{G6} and R^{G7} are preferably a hydrogen atom, an alkyl group, a cycloalkyl group, an aryl group, monovalent heterocyclic group, an alkoxy group, a cycloalkoxy group or a dendron, more preferably a hydrogen atom, an alkyl group, an aryl group, a monovalent heterocyclic group or a dendron, further preferably a hydrogen atom, an alkyl group, an aryl group, a monovalent heterocyclic group or a dendron, particularly preferably a hydrogen atom or a dendron, and these groups optionally have a substituent.

At least one of R^{G1} to R^{G8} is preferably an alkyl group, a cycloalkyl group, an aryl group, a monovalent heterocyclic group, an alkoxy group, a cycloalkoxy group, an aryloxy group or a dendron. More preferably at least one of R^{G2}, R^{G3} , R^{G6} and R^{G7} is an alkyl group, a cycloalkyl group, an aryl group, a monovalent heterocyclic group, an alkoxy group, a cycloalkoxy group, an aryloxy group or a dendron, and these groups optionally have a substituent.

When the ligand represented by the formula (GM-L1) has a dendron, at least one of R^{G2}, R^{G3} , R^{G6} and R^{G7} is preferably the dendron, and more preferably at least one of R^{G2} and R^{G6} is the dendron.

When the ligand represented by the formula (GM-L1) has an alkyl group, a cycloalkyl group, an aryl group, a monovalent heterocyclic group, an alkoxy group, a cycloalkoxy group or an aryloxy group, at least one of R^{G2}, R^{G3}, R^{G6} and R^{G7} is preferably the alkyl group, the cycloalkyl group, the aryl group, the monovalent heterocyclic group, the alkoxy group, the cycloalkoxy group or the aryloxy group, and more preferably at least one of R^{G2} and R^{G6} is the alkyl group, the cycloalkyl group, the aryl group, the monovalent heterocyclic group, the alkoxy group, the cycloalkoxy group or the aryloxy group, and these groups optionally have a substituent.

In the formula (GM-1), when a plurality of ligands represented by the formula (GM-L1) are present, the plurality of R^{G1} to R^{G8} each may be the same or different. 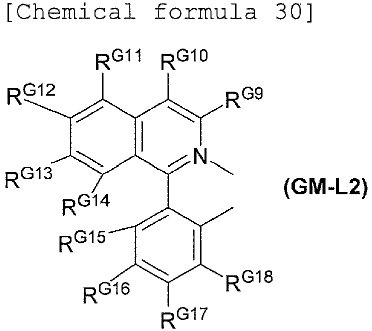 [wherein,
R^{G9} to R^{G18} each independently represent a hydrogen atom, an alkyl group, a cycloalkyl group, an aryl group, a monovalent heterocyclic group, an alkoxy group, a cycloalkoxy group, an aryloxy group or a dendron, and these groups optionally have a substituent. R^{G9} and R^{G10}, R^{G10} and R^{G11}, R^{G11} and R^{G12}, R^{G12} and R^{G13}, R^{G13} and R^{G14}, R^{G14} and R^{G15}, R^{G15} and R^{G16}, R^{G16} and R^{G17}, and R^{G17} and R^{G18} each may be combined together to form a ring together with the atoms to which they are attached.]

R^{G9}, R^{G11} to R^{G15} and R^{G18} are preferably a hydrogen atom, an alkyl group, a cycloalkyl group, an aryl group, a monovalent heterocyclic group, an alkoxy group or a cycloalkoxy group, more preferably a hydrogen atom, an alkyl group or an aryl group, further preferably a hydrogen atom or an alkyl group, particularly preferably a hydrogen atom, and these groups optionally have a substituent.

R^{G10}, R^{G16} and R^{G17} are preferably a hydrogen atom, an alkyl group, a cycloalkyl group, an aryl group, a monovalent heterocyclic group, an alkoxy group, a cycloalkoxy group or a dendron, more preferably a hydrogen atom, an alkyl group, an aryl group, a monovalent heterocyclic group or a dendron, further preferably a hydrogen atom, an alkyl group, an aryl group, a monovalent heterocyclic group or a dendron, particularly preferably a hydrogen atom or a dendron, and these groups optionally have a substituent.

At least one of R^{G9} to R^{G18} is preferably an alkyl group, a cycloalkyl group, an aryl group, a monovalent heterocyclic group, an alkoxy group, a cycloalkoxy group, an aryloxy group or a dendron, more preferably at least one of R^{G10}, R^{G16} and R^{G17} is an alkyl group, a cycloalkyl group, an aryl group, a monovalent heterocyclic group, an alkoxy group, a cycloalkoxy group, an aryloxy group or a dendron, and these groups optionally have a substituent.

When the ligand represented by the formula (GM-L2) has a dendron, preferably at least one of R^{G10}, R^{G16} and R^{G17} is the dendron, more preferably at least one of R^{G16} and R^{G17} is the dendron, and further preferably R^{G16} is the dendron.

When the ligand represented by the formula (GM-L2) has an alkyl group, a cycloalkyl group, an aryl group, a monovalent heterocyclic group, an alkoxy group, a cycloalkoxy group or an aryloxy group, preferably at least one of R^{G10}, R^{G16} and R^{G17} is the alkyl group, the cycloalkyl group, the aryl group, the monovalent heterocyclic group, the alkoxy group, the cycloalkoxy group or the aryloxy group, and more preferably at least one of R^{G16} and R^{G17} is the alkyl group, the cycloalkyl group, the aryl group, the monovalent heterocyclic group, the alkoxy group, the cycloalkoxy group or the aryloxy group.

In the formula (GM-1), when a plurality of ligands represented by the formula (GM-L2) are present, the plurality of R^{G9} to R^{G18} may be the same or different. 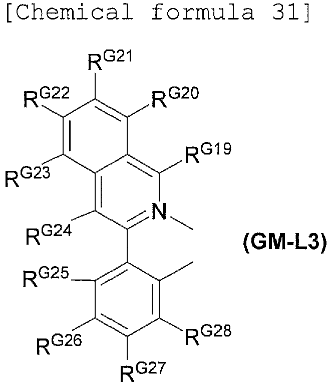 [wherein,
R^{G19} to R^{G28} each independently represent a hydrogen atom, an alkyl group, a cycloalkyl group, an aryl group, a monovalent heterocyclic group, an alkoxy group, a cycloalkoxy group, an aryloxy group or a dendron, and these groups optionally have a substituent. R^{G19} and R^{G20}, R^{G20} and R^{G21}, R^{G21} and R^{G22}, R^{G22} and R^{G23}, R^{G23} and R^{G24}, R^{G24} and R^{G25}, R^{G25}, and R^{G26}, R^{G26} and R^{G27}, and R^{G27} and R^{G28} each may be combined together to form a ring together with the atoms to which they are attached.]

R^{G19} to R^{G25}, and R^{G28} are preferably a hydrogen atom, an alkyl group, a cycloalkyl group, an aryl group, a monovalent heterocyclic group, an alkoxy group or a cycloalkoxy group, more preferably a hydrogen atom, an alkyl group or an aryl group, further preferably a hydrogen atom or an alkyl group, particularly preferably a hydrogen atom, and these groups optionally have a substituent.

R^{G26} and R^{G27} are preferably a hydrogen atom, an alkyl group, a cycloalkyl group, an aryl group, a monovalent heterocyclic group, an alkoxy group, a cycloalkoxy group or a dendron, more preferably a hydrogen atom, an alkyl group, an aryl group, a monovalent heterocyclic group or a dendron, further preferably a hydrogen atom, an alkyl group, an aryl group, a monovalent heterocyclic group or a dendron, particularly preferably a hydrogen atom or a dendron, and these groups optionally have a substituent.

At least one of R^{G19} to R^{G28} is preferably an alkyl group, a cycloalkyl group, an aryl group, a monovalent heterocyclic group, an alkoxy group, a cycloalkoxy group, an aryloxy group or a dendron, more preferably at least one of R^{G26} and R^{G27} is an alkyl group, a cycloalkyl group, an aryl group, a monovalent heterocyclic group, an alkoxy group, a cycloalkoxy group, an aryloxy group or a dendron, and these groups optionally have a substituent.

When the ligand represented by the formula (GM-L3) has a dendron, preferably at least one of R^{G26} and R^{G27} is the dendron, and more preferably R^{G26} is the dendron.

When the ligand represented by the formula (GM-L3) has an alkyl group, a cycloalkyl group, an aryl group, a monovalent heterocyclic group, an alkoxy group, a cycloalkoxy group or an aryloxy group, preferably at least one of R^{G26} and R^{G27} is the alkyl group, the cycloalkyl group, the aryl group, the monovalent heterocyclic group, the alkoxy group, the cycloalkoxy group or the aryloxy group, and these groups optionally have a substituent.

In the formula (GM-1), when a plurality of ligands represented by the formula (GM-L3) are present, the plurality of R^{G19} to R^{G28} each may be the same or different. 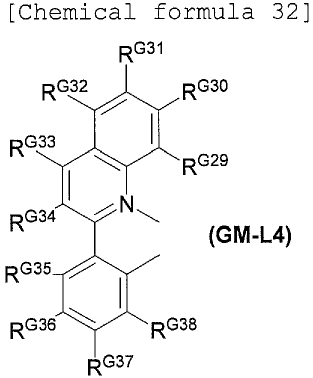 [wherein,
R^{G29} to R^{G38} each independently represent a hydrogen atom, an alkyl group, a cycloalkyl group, an aryl group, a monovalent heterocyclic group, an alkoxy group, a cycloalkoxy group, an aryloxy group or a dendron, and these groups optionally have a substituent. R^{G29} and R^{G30}, R^{G30} and R^{G31}, R^{G31} and R^{G32}, R^{G32} and R^{G33}, R^{G33} and R^{G34}, R^{G34} and R^{G35}, R^{G35} and R^{G36}, R^{G36} and R^{G37}, and R^{G37} and R^{G38} each may be combined together to form a ring together with the atoms to which they are attached.]

R^{G29} to R^{G32}, R^{G34}, R^{G35} and R^{G38} are preferably a hydrogen atom, an alkyl group, a cycloalkyl group, an aryl group, a monovalent heterocyclic group, an alkoxy group or a cycloalkoxy group, more preferably a hydrogen atom, an alkyl group or an aryl group, further preferably a hydrogen atom or an alkyl group, particularly preferably a hydrogen atom, and these groups optionally have a substituent.

R^{G33}, R^{G36} and R^{G37} are preferably a hydrogen atom, an alkyl group, a cycloalkyl group, an aryl group, a monovalent heterocyclic group, an alkoxy group, a cycloalkoxy group or a dendron, more preferably a hydrogen atom, an alkyl group, an aryl group, a monovalent heterocyclic group or a dendron, further preferably a hydrogen atom, an alkyl group, an aryl group, a monovalent heterocyclic group or a dendron, particularly preferably a hydrogen atom or a dendron, and these groups optionally have a substituent.

At least one of R^{G29} to R^{G38} is preferably an alkyl group, a cycloalkyl group, an aryl group, a monovalent heterocyclic group, an alkoxy group, a cycloalkoxy group, an aryloxy group or a dendron, more preferably at least one of R^{G33}, R^{G36} and R^{G37} is an alkyl group, a cycloalkyl group, an aryl group, a monovalent heterocyclic group, an alkoxy group, a cycloalkoxy group, an aryloxy group or a dendron, and these groups optionally have a substituent.

When the ligand represented by the formula (GM-L4) has a dendron, at least one of R^{G36} and R^{G37} is preferably the dendron, and further preferably R^{G36} is the dendron.

When the ligand represented by the formula (GM-L4) has an alkyl group, a cycloalkyl group, an aryl group, a monovalent heterocyclic group, an alkoxy group, a cycloalkoxy group or an aryloxy group, at least one of R^{G33}, R^{G36} and R^{G37} is preferably the alkyl group, the cycloalkyl group, the aryl group, the monovalent heterocyclic group, the alkoxy group, the cycloalkoxy group or the aryloxy group, more preferably at least one of R^{G36} and R^{G37} is the alkyl group, the cycloalkyl group, the aryl group, the monovalent heterocyclic group, the alkoxy group, the cycloalkoxy group or the aryloxy group, and further preferably R^{G36} is the alkyl group, the cycloalkyl group, the aryl group, the monovalent heterocyclic group, the alkoxy group, the cycloalkoxy group or the aryloxy group.

In the formula (GM-1), when a plurality of ligands represented by the formula (GM-L4) are present, the plurality of R^{G29} to R^{G38} each may be the same or different.

When the ring R^{1G1} has no atomic bonding, the definition of the ring R^{1G1} is the same as the definition of the above-described ring R^{1G}.

When the ring R^{1G1} has atomic bonding, the definition of the ring portion of the ring R^{1G1} excepting the atomic bonding is the same as the definition of the above-described ring R^{1G}.

When the ring R^{2G1} has no atomic bonding, the definition of the ring R^{2G1} is the same as the definition of the above-described ring R^{2G}.

When the ring R^{2G1} has atomic bonding, the definition of the ring portion of the ring R^{2G1} excepting the atomic bonding is the same as the definition of the above-described ring R^{2G}.

In the formula (GM-1), the ligand represented by the ring R^{1G1}-ring R^{2G1} is preferably a ligand represented by the formula (GM-L1) to the formula (GM-L4), and more preferably a ligand represented by the formula (GM-L1) or the formula (GM-L2).

When the ligand represented by the ring R^{1G1}-ring R^{2G1} is the formula (GM-L1), one of R^{G1} to R^{G8} represents atomic bonding, preferably R^{G2}, R^{G3}, R^{G6} or R^{G7} is the atomic bonding, more preferably R^{G2}, R^{G6} or R^{G7} is the atomic bonding, further preferably R^{G2} or R^{G6} is the atomic bonding, and particularly preferably R^{G6} is the atomic bonding.

When the ligand represented by the ring R^{1G1}-ring R^{2G1} is the formula (GM-L2), one of R^{G9} to R^{G18} represents atomic bonding, preferably R^{G10}, R^{G16} or R^{G17} is the atomic bonding, and more preferably R^{G16} is the atomic bonding.

When the ligand represented by the ring R^{1G1}-ring R^{2G1} is the formula (GM-L3), one of R^{G19} to R^{G28} represents atomic bonding, preferably R^{G26} or R^{G27} is the atomic bonding, and more preferably R^{G26} is the atomic bonding.

When the ligand represented by the ring R^{1G1}-ring R^{2G1} is the formula (GM-L4), one of the R^{G29} to R^{G38} represents atomic bonding, preferably R^{G36} or R^{G37} is the atomic bonding, and more preferably R^{G36} is the atomic bonding.

The anionic bidentate ligand represented by A¹-G¹-A² includes, for example, ligands represented by the following formulae. 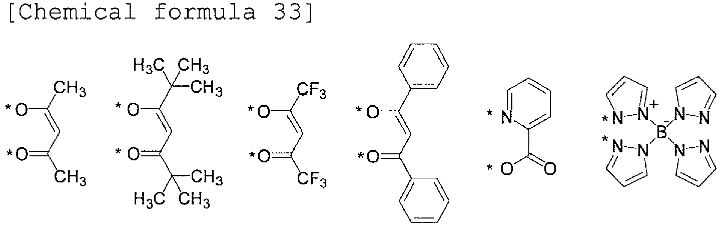 [wherein, * represents a position to be bonded to M. These bidentate ligands optionally have a substituent.] 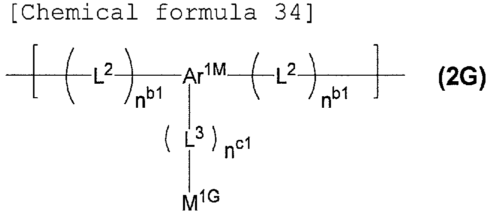 [wherein,
M^{1G} represents the same meaning as described above.
L² and L³ each independently represent an oxygen atom, a sulfur atom, a group represented by -N(RA)-, a group represented by -C(R^{B})2-, a group represented by - C(R^{B})=C(R^{B})-, a group represented by -C=C-, an arylene group or a divalent heterocyclic group, and these groups optionally have a substituent. R^{A} and R^{B} represent the same meaning as described above. When a plurality of L² and L³ present, they may be the same or different at each occurrence.
n^{b1} and n^{c1} each independently represents an integer of 0 or more. The plurality of n^{b1} may be the same or different.
Ar^{1M} represents a trivalent aromatic hydrocarbon group or a trivalent heterocyclic group, and these groups optionally have a substituent.]

L² is preferably a group represented by -C(R^{B})2-_{,} an arylene group or a divalent heterocyclic group, more preferably an arylene group or a divalent heterocyclic group, further preferably an arylene group, and these groups optionally have a substituent. In particular, a group represented by the formula (A-1) or the formula (A-2) is preferable.

L³ is preferably a group represented by -C(RB)2-_{,} an arylene group or a divalent heterocyclic group, more preferably a group represented by -C(R^{B})₂- or an arylene group, further preferably an arylene group, and these groups optionally have a substituent. In particular, a group represented by the formula (A-1) or the formula (A-2) is preferable.

n^{b1} and n^{c1} are usually integers of 0 to 10, preferably integers of 0 to 5, more preferably integers of 0 to 2, further preferably 0 or 1, and particularly preferably 0.

Ar^{1M} is preferably a group formed by removing from a benzene ring, a naphthalene ring, a fluorene ring, a phenanthrene ring, a dihydrophenanthrene ring, a pyridine ring, a diazabenzene ring, a triazine ring, a carbazole ring, a phenoxazine ring or a phenothiazine ring three hydrogen atoms linked directly to carbon atoms or hetero atoms constituting the ring. More preferably, it is a group formed by removing from a benzene ring, a naphthalene ring, a fluorene ring, a phenanthrene ring or a dihydrophenanthrene ring three hydrogen atoms linked directly to carbon atoms constituting the ring, further preferably a group formed by removing from a benzene ring or a fluorene ring three hydrogen atoms linked directly to carbon atoms constituting the ring, particularly preferably a group formed by removing from a benzene ring three hydrogen atoms linked directly to carbon atoms constituting the ring, and these groups optionally have a substituent.

The substituents which L², L³ and Ar^{1M} optionally have are the same as the substituents which the above-described ring R^{1G} and the ring R^{2G} optionally have. 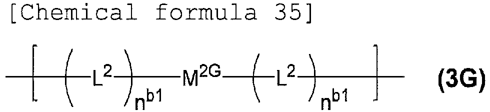 [wherein,
_{L}² and n^{b1} represent the same meaning as described above.
M^{2G} represents a group formed by removing from a phosphorescent compound two hydrogen atoms linked directly to carbon atoms or hetero atoms constituting the compound.]

M^{2G} is preferably a group represented by the formula (GM-2) or the formula (GM-3), and more preferably a group represented by the formula (GM-2). 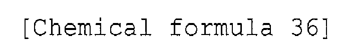 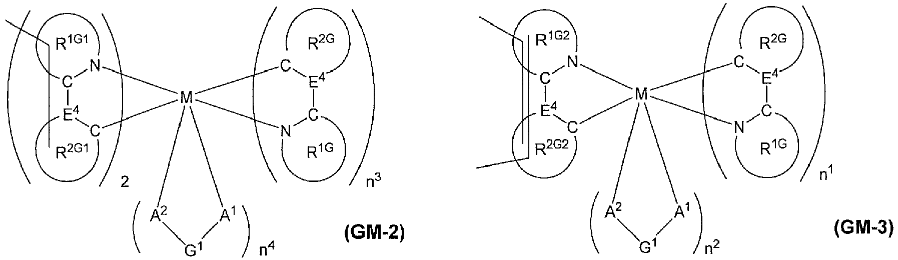 [wherein,
M, E⁴, a ring R^{1G}, a ring R^{2G}, A¹-G¹-A², a ring R^{1G1}, a ring R^{2G1} , n¹ and n² represent the same meaning as described above.
n³ and n⁴ each independently represent an integer of 0 or more. n³+n⁴ is 0 or 1. When M is a ruthenium atom, a rhodium atom or an iridium atom, n³+n⁴ is 1. When M is a palladium atom or a platinum atom, n³+n⁴ is 0.
The ring R^{1G2} represents a six-membered aromatic heterocyclic ring, and these rings optionally have a substituent. When a plurality of the substituents are present, they may be the same or different, and they may be combined together to form a ring together with the atoms to which they are attached.
The ring R^{2G2} represents a five-membered or six-membered aromatic carbon ring, or a five-membered or six-membered aromatic heterocyclic ring, and these rings optionally have a substituent. When a plurality of the substituents are present, they may be the same or different, and they may be combined together to form a ring together with the atoms to which they are attached.
One of the ring R^{1G2} and the ring R^{2G2} has two atomic bonding, or the ring R^{1G2} and the ring R^{2G2} each have one atomic bonding.]

When M is a ruthenium atom, a rhodium atom or an iridium atom, n⁴ is preferably 0.

When the ring R^{1G2} has no atomic bonding, the definition of the ring R^{1G2} is the same as the definition of the above-described ring R^{1G}.

When the ring R^{1G2} has atomic bonding, the definition of the ring portion of the ring R^{1G2} excepting the atomic bonding is the same as the definition of the above-described ring R^{1G}.

When the ring R^{2G2} has no atomic bonding, the definition of the ring R^{1G2} is the same as the definition of the above-described ring R^{2G}.

When the ring R^{2G2} has atomic bonding, the definition of the ring portion of the ring R^{2G2} excepting the atomic bonding is the same as the definition of the above-described ring R^{2G}.

The substituents which the ring R^{1G2} and the ring R^{2G2} optionally have are the same as the substituents which the above-described ring R^{1G} and ring R^{2G} optionally have.

It is preferable that the ring R^{1G2} and ring R^{2G2} each optionally have one atomic bonding.

The ligand represented by the ring R^{1G2}-ring R^{2G2} is preferably a ligand represented by the formula (GM-L1) to the formula (GM-L4), and more preferably a ligand represented by the formula (GM-L1) or the formula (GM-L2).

When the ligand represented by the ring R^{1G2}-ring R^{2G2} is the formula (GM-L1), two of R^{G1} to R^{G8} represent atomic bonding, preferably two of R^{G2} , R^{G3} , R^{G6} and R^{G7} are the atomic bonding, and more preferably R^{G2} and R^{G6}, R^{G2} and R^{G7} , R^{G3} and R^{G6}, or R^{G3} and R^{G7} are the atomic bonding.

When the ligand represented by the ring R^{1G2}-ring R^{2G2} is the formula (GM-L2), two of R^{G9} to R^{G18} represent bond, preferably two of R^{G10}, R^{G16} and R^{G17} are the atomic bonding, and more preferably R^{G10} and R^{G16}, or, R^{G10} and R^{G17} are the atomic bonding.

When the ligand represented by the ring R^{1G2}-ring R^{2G2} is the formula (GM-L3), two of R^{G19} to R^{G28} represent atomic bonding, preferably two of R^{G20} to R^{G23}, R^{G26} and R^{G27} are the atomic bonding, more preferably R^{G26} and any one selected from the group consisting of R^{G20}, R^{G21}_{,} R^{G17} and R^{G17} are the atomic bonding, or R^{G27} and any one selected from the group consisting of R^{G20}, R^{G21}, R^{G22} and R^{G23} are the atomic bonding.

When the ligand represented by ring R^{1G2}-ring R^{G17} is the formula (GM-L4), two of R^{G29} to R^{G38} represent atomic bonding, preferably two of R^{G36}, R^{G37}' and R^{G33} are the atomic bonding, and more preferably R^{G36} and R^{G33}, or R^{G37} and R^{G17} are the atomic bonding.

In the formula (3G), the preferable ranges for L² and n^{b1} are the same as those for L² and n^{b1} in the formula (2G) . 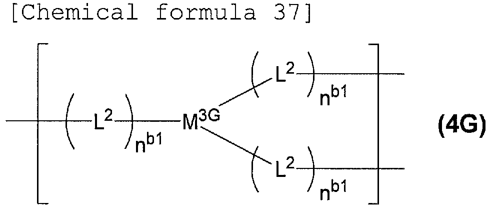 [wherein,
L² and n^{b1} represent the same meaning as described above.
M^{3G} represents a group formed by removing from a phosphorescent compound three hydrogen atoms linked directly to carbon atoms or hetero atoms constituting the compound.]

M^{3G} is preferably a group represented by the formula (GM-4). 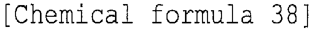 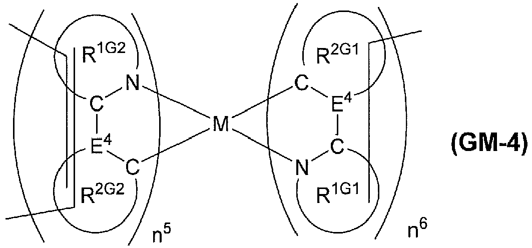 [wherein,
M, E⁴, a ring R^{1G1}, a ring R^{2G1}, a ring R^{1G2} and a ring R^{2G2} represent the same meaning as described above.
n⁵ represents 0 or 1. n⁶ represents 1 or 3. When M is a ruthenium atom, a rhodium atom or an iridium atom, n⁵ is 0, and n⁶ is 3. When M is a palladium atom or a platinum atom, n⁵ is 1, and n⁶ is 1. ]

In the formula (4G), the preferable embodiments for L² and n^{b1} are the same as those for L² and n^{b1} in the formula (2G).

The constitutional unit represented by the formula (1G) may be constitutional units represented by the formula (1G-1) to the formula (1G-12). 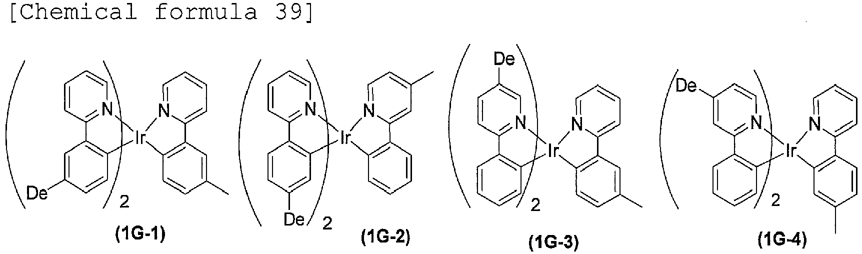 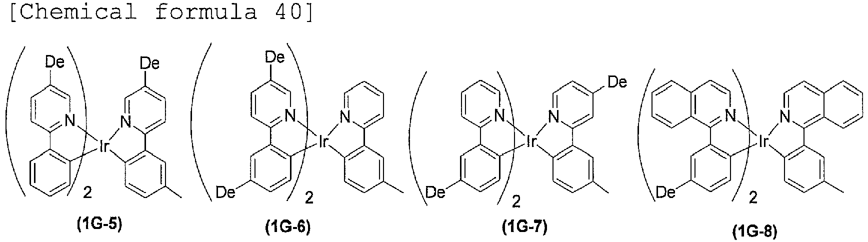 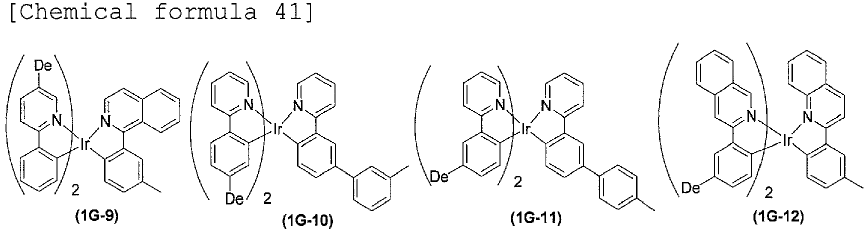 [wherein, De represents a hydrogen atom, a methyl group, an ethyl group, a propyl group, a n-butyl group, a tert-butyl group, a group represented by the formula (D-A) or a group represented by the formula (D-B).]

The constitutional unit represented by the formula (2G) may be constitutional units represented by the formula (2G-1) to the formula (2G-12). 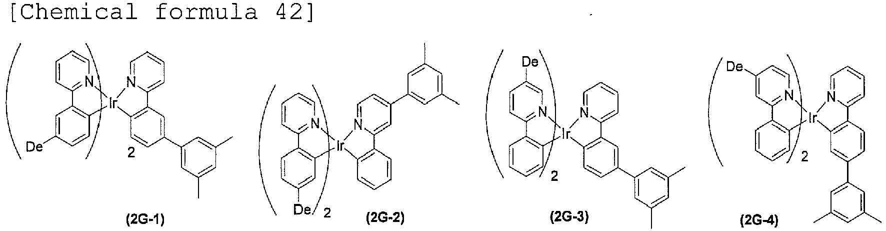 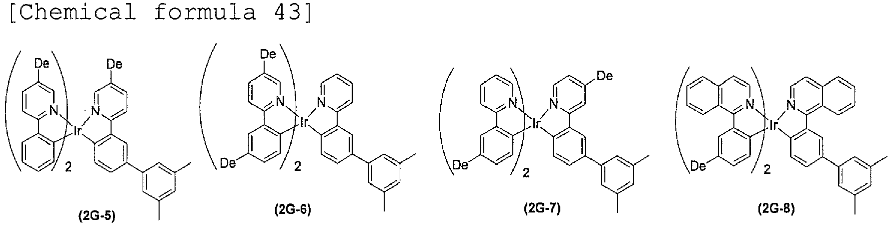 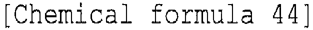 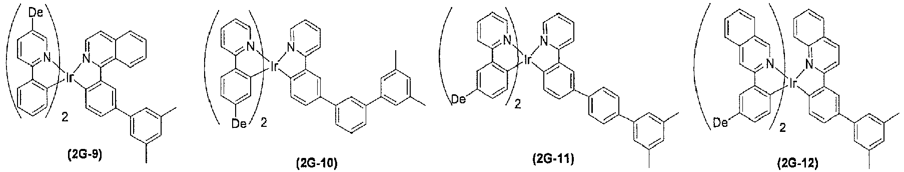 [wherein, De represents the same meaning as described above.]

The constitutional unit represented by the formula (3G) may be constitutional units represented by the formula (3G-1) to the formula (3G-20). 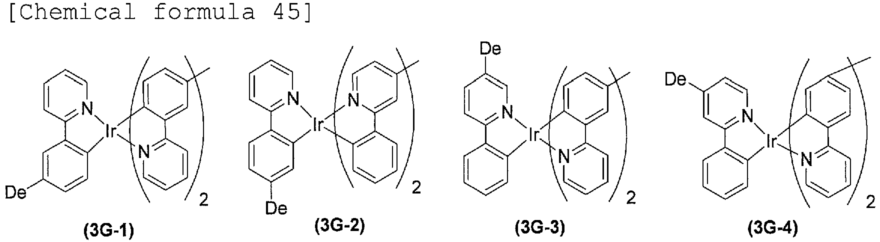 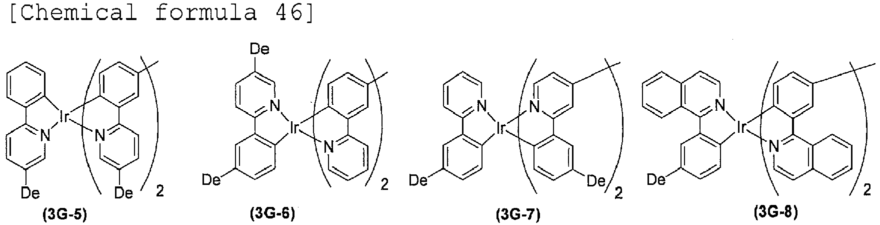 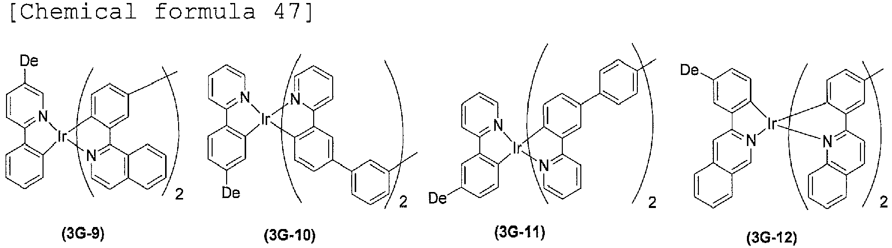 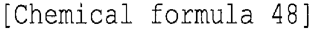 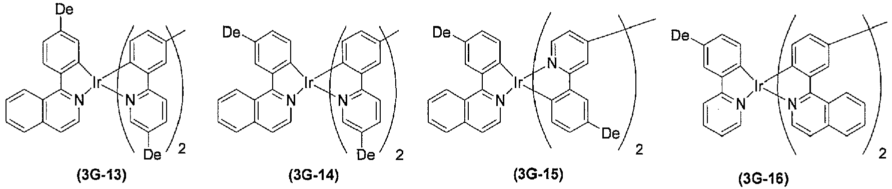 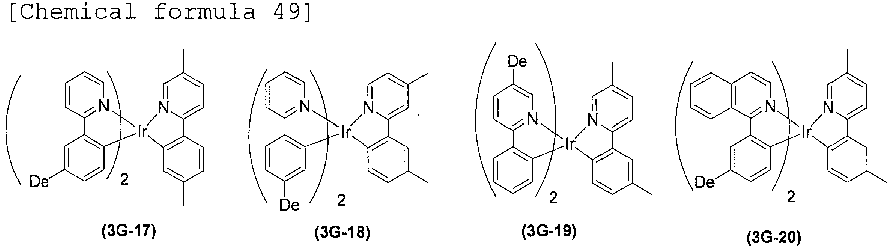 [wherein, De represents the same meaning as described above.]

The constitutional unit represented by the formula (4G) may be constitutional units represented by the formula (4G-1) to the formula (4G-7). 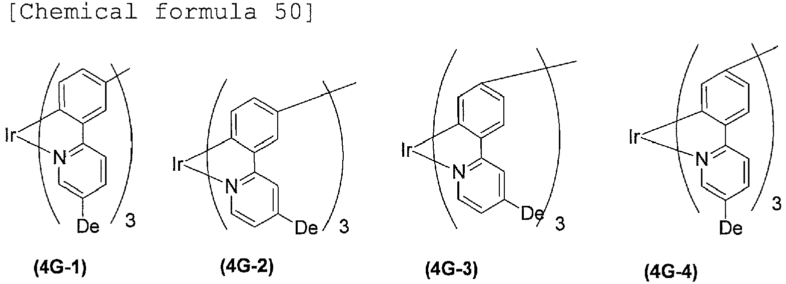 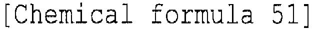 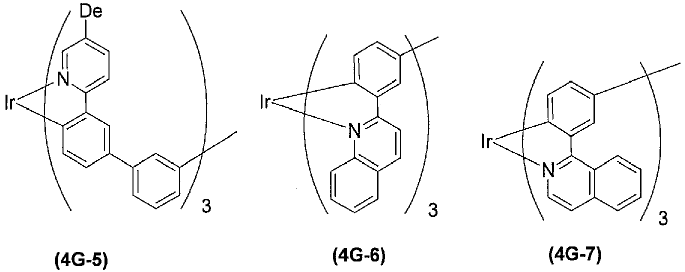 [wherein, De represents the same meaning as described above.]

The amount of the phosphorescent constitutional unit contained in the polymer compound is preferably 0.01 to 30 mol%, more preferably 0.05 to 20 mol%, and further preferably 0.1 to 10 mol% with respect to the total amount of 100 mol% of the constitutional units contained in the polymer compound, because a light emitting device which is excellent in external quantum efficiency can be produced.

The phosphorescent constitutional unit may be contained only singly or two or more units thereof may be contained in the polymer compound.

Preferably a polymer compound comprising a constitutional unit having a cross-linkable group and a phosphorescent constitutional unit further comprises at least one constitutional unit selected from the group consisting of a constitutional unit represented by the formula (X) and a constitutional unit represented by the formula (Y). 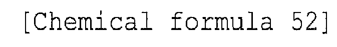 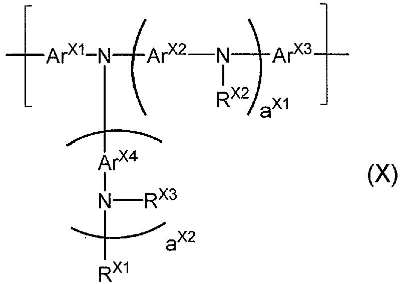 [wherein,
a^{X1} and a^{X2} each independently represent an integer of 0 or more.
Ar^{X1} and Ar^{X3} each independently represent an arylene group or a divalent heterocyclic group, and these groups each optionally have a substituent.
Ar^{X2} and Ar^{X4} each independently represent an arylene group, a divalent heterocyclic group or a divalent group in which at least one arylene group and at least one divalent heterocyclic group are bonded directly to each other, and these groups each optionally have a substituent. When a plurality of Ar^{X2} and Ar^{X4} are present, they may be the same or different at each occurrence.
R^{X1}, R^{X2} and R^{X3} each independently represent a hydrogen atom, an alkyl group, a cycloalkyl group, an aryl group or a monovalent heterocyclic group, and these groups each optionally have a substituent. When a plurality of R^{X2} and R^{X3} are present, they may be the same or different at each occurrence.]

a^{X1} is preferably 2 or less, more preferably 1, because the light emitting device which is more excellent in the luminance life can be produced.

a^{X2} is preferably 2 or less, more preferably 0, because the light emitting device which is more excellent in the luminance life can be produced.

R^{X1}, R^{X2} and R^{X3} are preferably an alkyl group, a cycloalkyl group, an aryl group or a monovalent heterocyclic group, more preferably an aryl group, and these groups each optionally have a substituent.

The arylene group represented by Ar^{X1} and Ar^{X3} is preferably a group represented by the formula (A-1) or the formula (A-9), more preferably a group represented by the formula (A-1).

The divalent heterocyclic group represented by Ar^{X1} and Ar^{X3} is preferably a group represented by the formula (AA-1), the formula (AA-2) or the formulae (AA-7) to (AA-26).

Ar^{X1} and Ar^{X3} are preferably an arylene group optionally having a substituent.

The arylene group represented by Ar^{X2} and Ar^{X4} is preferably a group represented by the formula (A-1), the formula (A-6), the formula (A-7), the formulae (A-9) to (A-11) or the formula (A-19).

The preferable example of the divalent heterocyclic group represented by Ar^{X2} and Ar^{X4} is preferably a group represented by the formula (AA-1), the formula (AA-2), the formula (AA-4) or the formulae (AA-7) to (AA-26).

The number of carbon atoms of a divalent group in which at least one arylene group and at least one divalent heterocyclic group are bonded directly to each other represented by Ar^{X2} and Ar^{X4} is, not including the number of carbon atoms of a substituent, usually 10 to 80, preferably 10 to 60, and more preferably 12 to 28. The preferable examples of an arylene group and a divalent heterocyclic group in the divalent group are the same as the preferable examples of the arylene group and the divalent heterocyclic group represented by Ar^{X1} and Ar^{X3}, respectively.

The divalent group in which at least one arylene group and at least one divalent heterocyclic group are bonded directly to each other includes, for example, groups represented by the following formulae, and they optionally have a substituent. 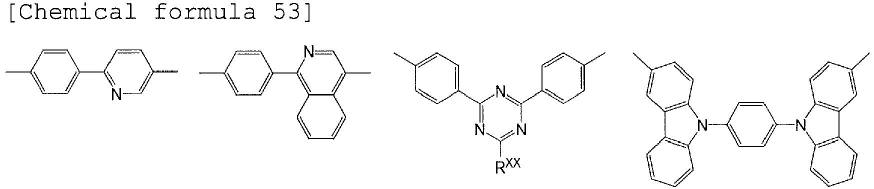 [wherein, R^{XX} represents a hydrogen atom, an alkyl group, a cycloalkyl group, an aryl group or a monovalent heterocyclic group, and these groups each optionally have a substituent.]

R^{XX} is preferably an alkyl group, a cycloalkyl group or an aryl group, and these groups each optionally have a substituent.

Ar^{X2} and Ar^{X4} are preferably an arylene group optionally having a substituent.

The preferable substituent which the group represented by Ar^{X1} to Ar^{X4} and R^{X1} to R^{X3} optionally has is preferably an alkyl group, a cycloalkyl group or an aryl group, and these groups optionally further have a substituent.

The constitutional unit represented by the formula (X) is preferably a constitutional unit represented by the formulae (X-1) to (X-7), more preferably a constitutional unit represented by the formulae (X-3) to (X-7), further preferably a constitutional unit represented by the formulae (X-3) to (X-6). 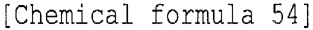 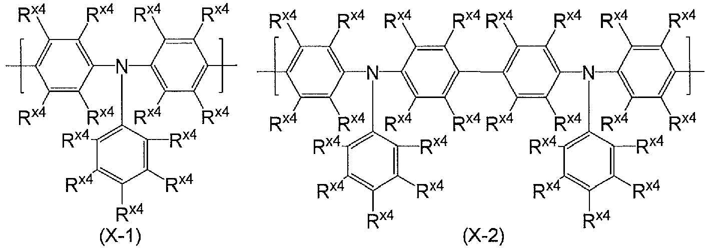 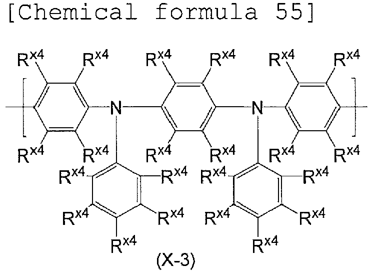 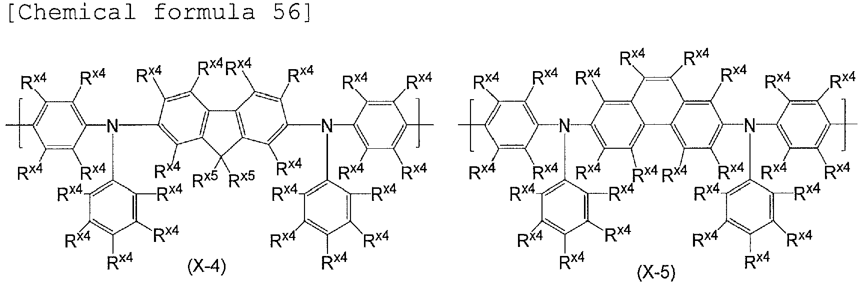 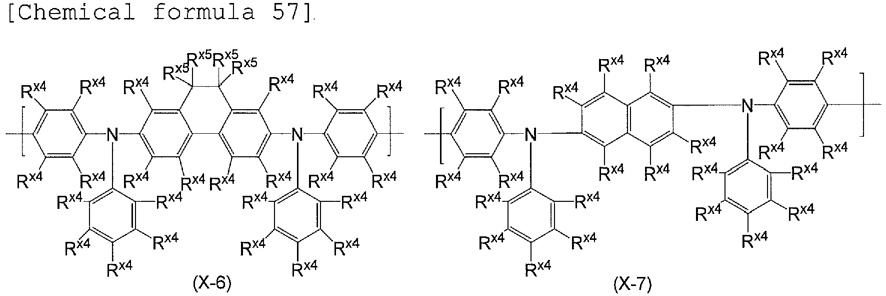 [wherein, R^{X4} and R^{X5} each independently represent a hydrogen atom, an alkyl group, a cycloalkyl group, an alkoxy group, a cycloalkoxy group, an aryl group, an aryloxy group, a halogen atom, a monovalent heterocyclic group or a cyano group, and these groups each optionally have a substituent. The plurality of R^{X4} may be the same or different. The plurality of R^{X5} may be the same or different, and adjacent groups R^{X5} may be combined together to form a ring together with the carbon atoms to which they are attached.]

The amount of the constitutional unit represented by the formula (X) is preferably 0.1 to 65 mol%, more preferably 1 to 50 mol%, further preferably 5 to 50 mol% with respect to the total amount of constitutional units contained in the polymer compound, because hole transportability is excellent.

The constitutional unit represented by the formula (X) includes, for example, constitutional units represented by the formulae (X1-1) to (X1-19), preferably constitutional units represented by the formulae (X1-6) to (X1-14).

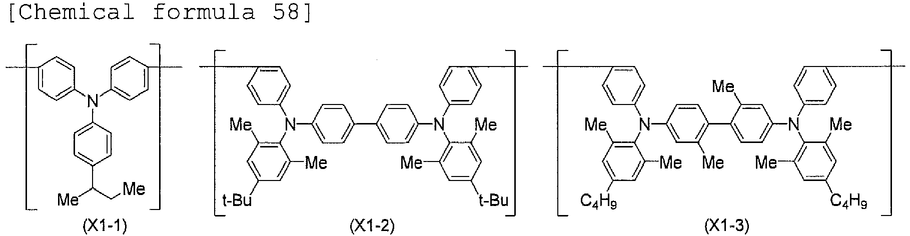

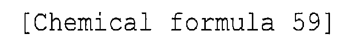

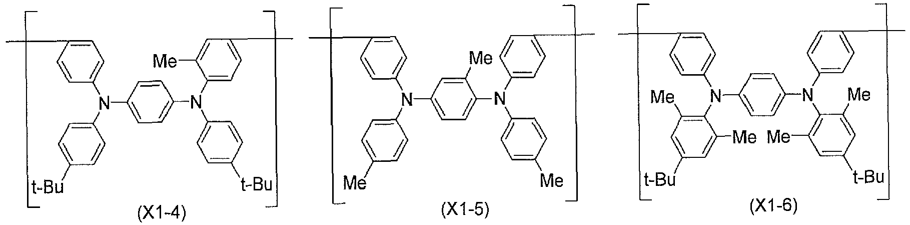

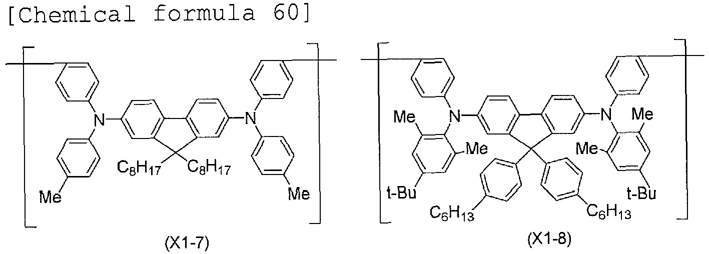

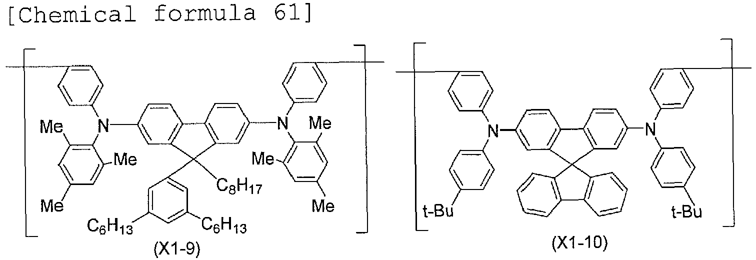

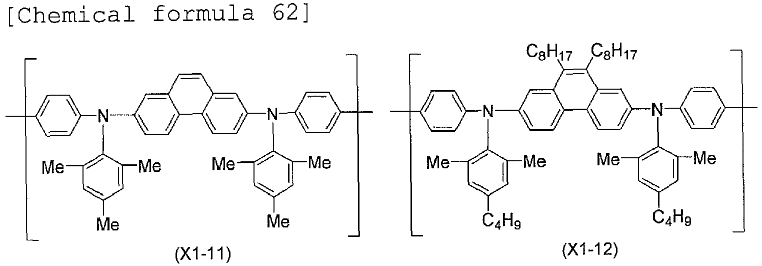

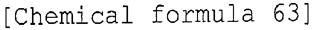

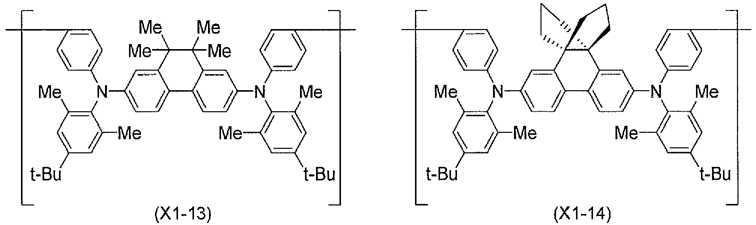

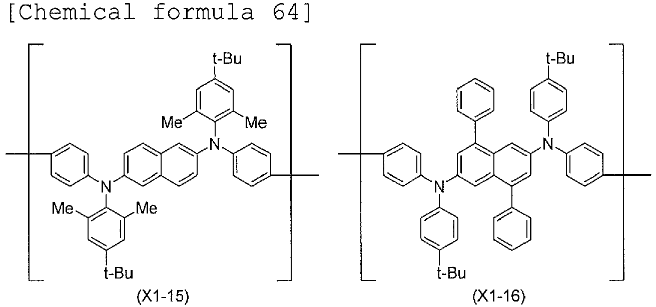

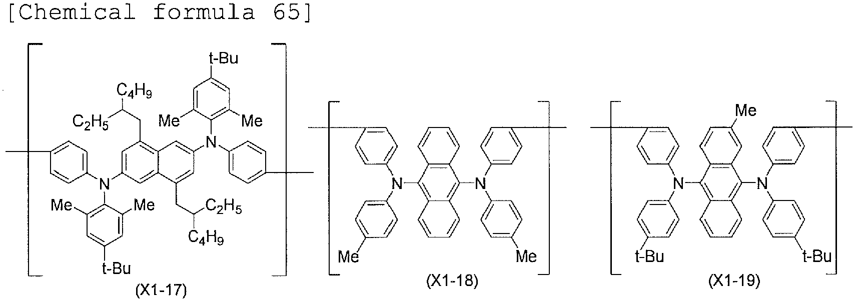

The constitutional unit represented by the formula (X) may be contained only singly or two or more units thereof may be contained in the polymer compound of the present invention. 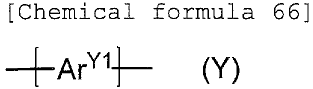 [wherein, Ar^{Y1} represents an arylene group, a divalent heterocyclic group or a divalent group in which at least one arylene group and at least one divalent heterocyclic group are bonded directly to each other, and these groups each optionally have a substituent.]

The arylene group represented by Ar^{Y1} is preferably a group represented by the formula (A-1), the formula (A-6), the formula (A-7), the formulae (A-9) to (A-11), the formula (A-13) or the formula (A-19), more preferably a group represented by the formula (A-1), the formula (A-7), the formula (A-9) or the formula (A-19).

The divalent heterocyclic group represented by Ar^{Y1} is preferably a group represented by the formula (AA-4), the formula (AA-10), the formula (AA-13), the formula (AA-15), the formula (AA-18) or the formula (AA-20), more preferably a group represented by formula (AA-4), the formula (AA-10), the formula (AA-18) or the formula (AA-20).

The preferable examples of the arylene group and the divalent heterocyclic group in the divalent group in which at least one arylene group and at least one divalent heterocyclic group are bonded directly to each other represented by Ar^{Y1} are the same as the preferable examples of the arylene group and the divalent heterocyclic group represented by Ar^{Y1} described above, respectively.

The number of carbon atoms and preferable examples of the divalent group in which at least one arylene group and at least one divalent heterocyclic group are bonded directly to each other represented by Ar^{Y1} are the same as the divalent group in which at least one arylene group and at least one divalent heterocyclic group are bonded directly to each other represented by Ar^{X2} and Ar^{X4} in the formula (X), respectively.

The substituent which the group represented by Ar^{Y1} optionally has is preferably an alkyl group, a cycloalkyl group, an alkoxy group, a cycloalkoxy group, an aryl group or a monovalent heterocyclic group, more preferably an alkyl group, a cycloalkyl group or an aryl group, and these groups optionally further have a substituent.

The constitutional unit represented by the formula (Y) includes, for example, constitutional units represented by the formulae (Y-1) to (Y-10), and from the standpoint of the luminance life of the light emitting device produced by using the polymer compound preferable is constitutional unit represented by the formula (Y-1) or the formula (Y-2), from the standpoint of electron transportability preferable is constitutional unit represented by the formula (Y-3) or the formula (Y-4), and from the standpoint of hole transportability preferable are constitutional units represented by the formulae (Y-5) to (Y-7). 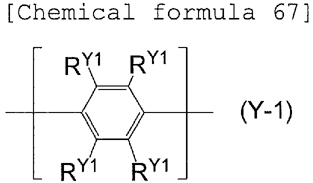 [wherein, R^{Y1} represents a hydrogen atom, an alkyl group, a cycloalkyl group, an alkoxy group, a cycloalkoxy group, an aryl group or a monovalent heterocyclic group, and these groups optionally have a substituent. The plurality of R^{Y1} may be the same or different, and adjacent groups R^{Y1} may be combined together to form a ring together with the carbon atoms to which they are attached.]

R^{Y1} is preferably a hydrogen atom, an alkyl group, a cycloalkyl group or an aryl group, and these groups optionally have a substituent.

The constitutional unit represented by the formula (Y-1) is preferably a constitutional unit represented by the formula (Y-1'). 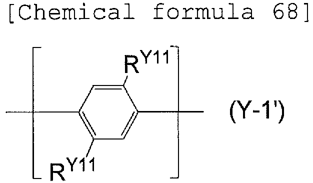 [wherein, R^{Y11} represents an alkyl group, a cycloalkyl group, an alkoxy group, a cycloalkoxy group, an aryl group or a monovalent heterocyclic group, and these groups optionally have a substituent. The plurality of R^{Y11} may be the same or different.]

R^{Y11} is preferably an alkyl group, a cycloalkyl group or an aryl group, more preferably an alkyl group or a cycloalkyl group, and these groups optionally have a substituent. 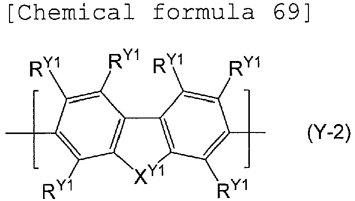 [wherein, R^{Y1} represents the same meaning as described above. X^{Y1} represents a group represented by -C(R^{Y2})₂-,-C(R^{Y2})=C(R^{Y2}) - or -C(R^{Y2})₂-C(R^{Y2})₂-R^{Y2} represents a hydrogen atom, an alkyl group, a cycloalkyl group, an alkoxy group, a cycloalkoxy group, an aryl group or a monovalent heterocyclic group, and these groups optionally have a substituent. The plurality of R^{Y2} may be the same or different, and groups R^{Y2} may be combined together to form a ring together with the carbon atoms to which they are attached.]

R^{Y2} is preferably an alkyl group, a cycloalkyl group, an aryl group or a monovalent heterocyclic group, more preferably an alkyl group a cycloalkyl group or an aryl group, and these groups each optionally have a substituent.

Regarding the combination of two R^{Y2} S in the group represented by -C(R^{Y2})₂- in X^{Y1}, it is preferable that the both are an alkyl group or a cycloalkyl group, the both are an aryl group, the both are a monovalent heterocyclic group, or one is an alkyl group or a cycloalkyl group and the other is an aryl group or a monovalent heterocyclic group, it is more preferable that one is an alkyl group or cycloalkyl group and the other is an aryl group, and these groups each optionally have a substituent. The two groups R^{Y2} may be combined together to form a ring together with the atoms to which they are attached, and when the groups R^{Y2} form a ring, the group represented by -C(R^{Y2})₂- is preferably a group represented by the formulae (Y-A1) to (Y-A5), more preferably a group represented by the formula (Y-A4), and these groups each optionally have a substituent.

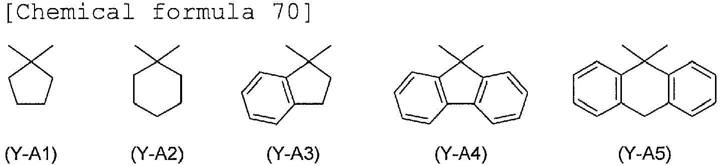

Regarding the combination of two R^{Y2}s in the group represented by -C(R^{Y2})=C(R^{Y2})- in X^{Y1}, it is preferable that the both are an alkyl group or cycloalkyl group, or one is an alkyl group or a cycloalkyl group and the other is an aryl group, and these groups each optionally have a substituent.

Four R^{Y2}_{S} in the group represented by -C(R^{Y2})₂-C(R^{Y2})₂- in X^{Y1} are preferably an alkyl group or a cycloalkyl group, and these groups optionally have a substituent. The plurality of R^{Y2} may be combined together to form a ring together with the atoms to which they are attached, and when the groups R^{Y2} form a ring, the group represented by -C(R^{Y2})₂-C(R^{Y2})₂- is preferably a group represented by the formulae (Y-B1) to (Y-B5), more preferably a group represented by the formula (Y-B3), and these groups each optionally have a substituent. 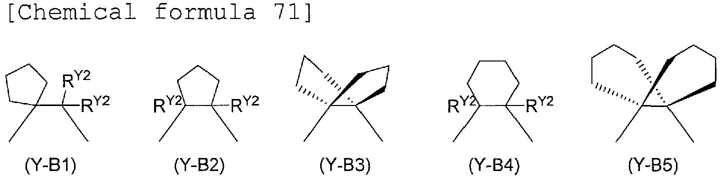 [wherein, R^{Y2} represents the same meaning as described above.]

It is preferable that the constitutional unit represented by the formula (Y-2) is a constitutional unit represented by the formula (Y-2'). 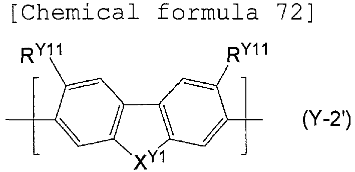 [wherein, R^{Y11} and X^{Y1} represents the same meaning as described above.] 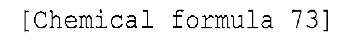 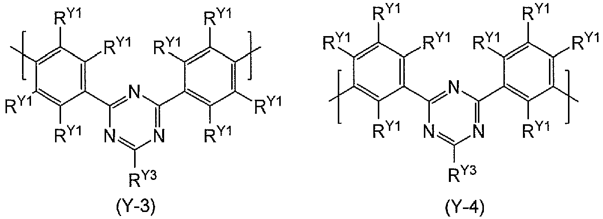 [wherein, R^{Y1} represents the same meaning as described above. R^{Y3} represents a hydrogen atom, an alkyl group, a cycloalkyl group, an alkoxy group, a cycloalkoxy group, an aryl group or a monovalent heterocyclic group, and these groups each optionally have a substituent.]

R^{Y3} is preferably an alkyl group, a cycloalkyl group, an alkoxy group, a cycloalkoxy group, an aryl group or a monovalent heterocyclic group, more preferably an aryl group, and these groups optionally have a substituent. 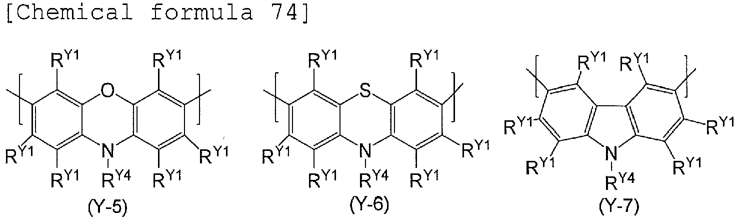 [wherein, R^{Y1} represents the same meaning as described above. R^{Y4} represents a hydrogen atom, an alkyl group, a cycloalkyl group, an alkoxy group, a cycloalkoxy group, an aryl group or a monovalent heterocyclic group, and these groups optionally have a substituent.]

R^{Y4} is preferably an alkyl group, a cycloalkyl group, an alkoxy group, a cycloalkoxy group, an aryl group or a monovalent heterocyclic group, more preferably an aryl group, and these groups optionally have a substituent.

The constitutional unit represented by the formula (Y) includes, for example, a constitutional unit represented by the formulae (Y-11) to (Y-55).

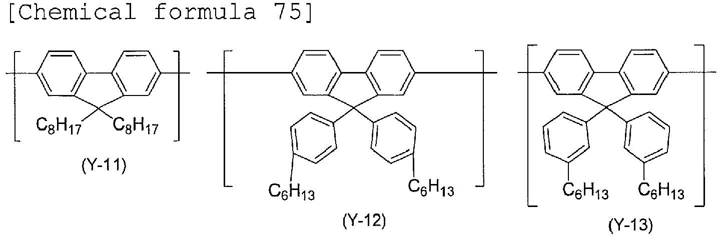

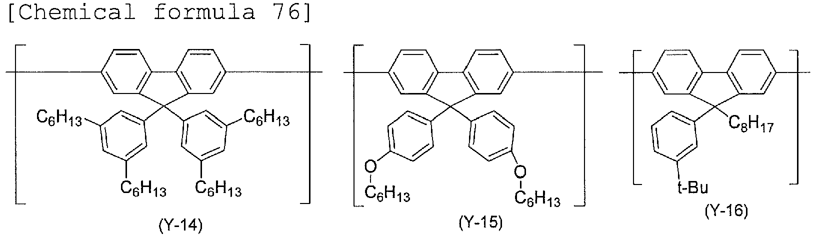

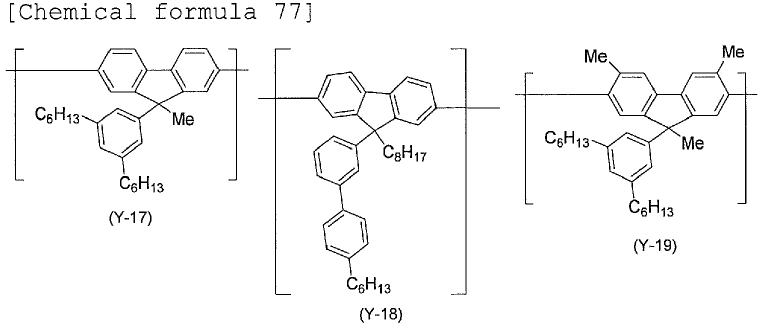

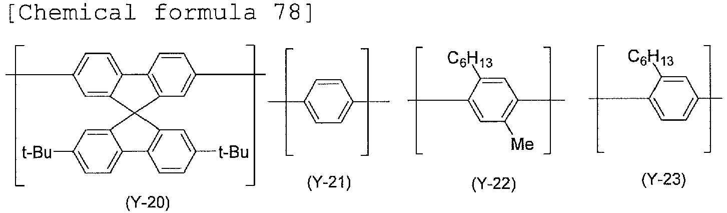

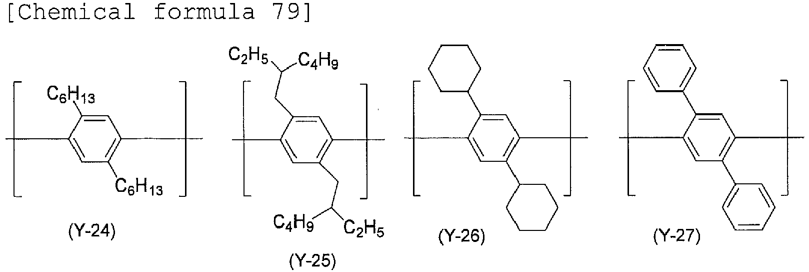

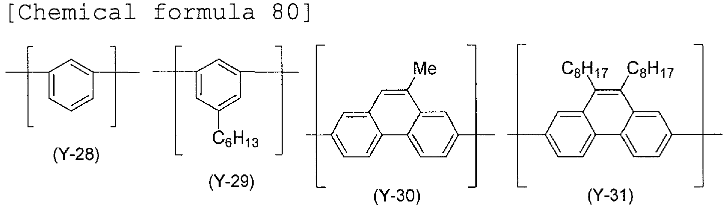

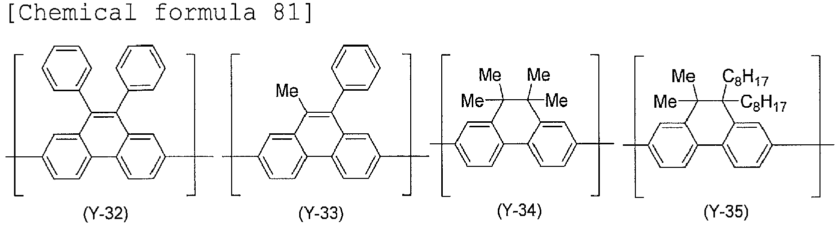

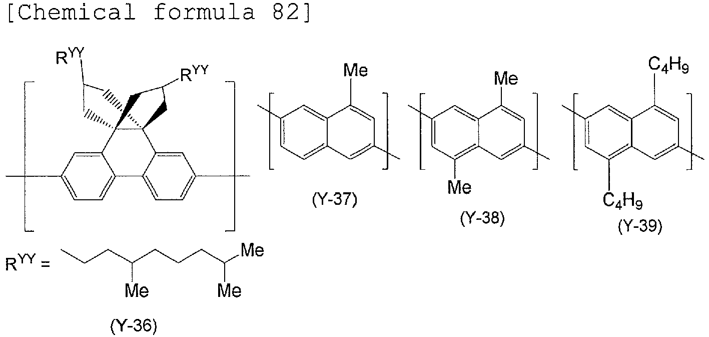

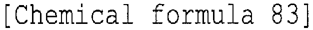

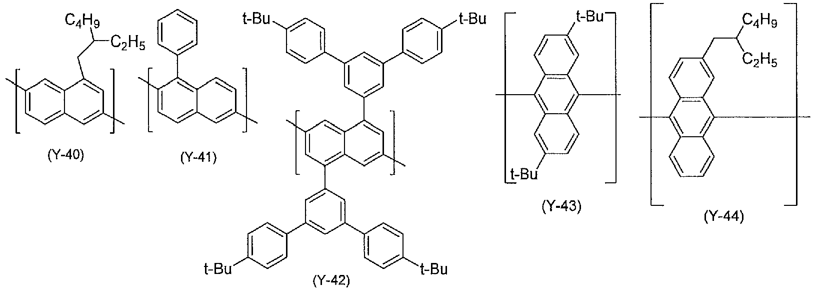

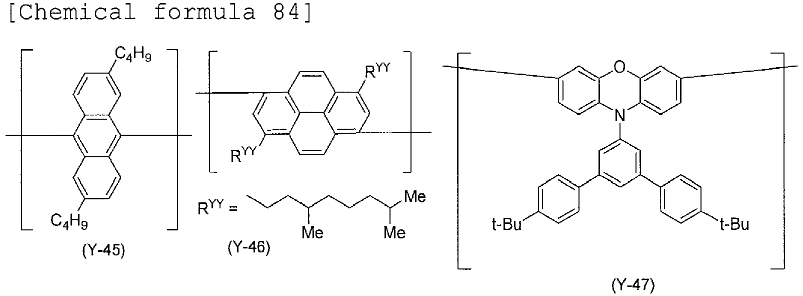

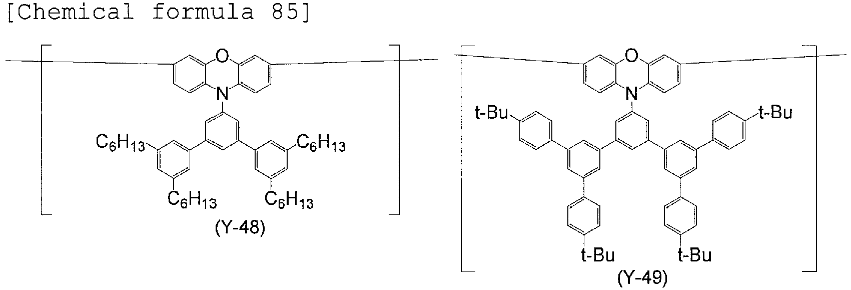

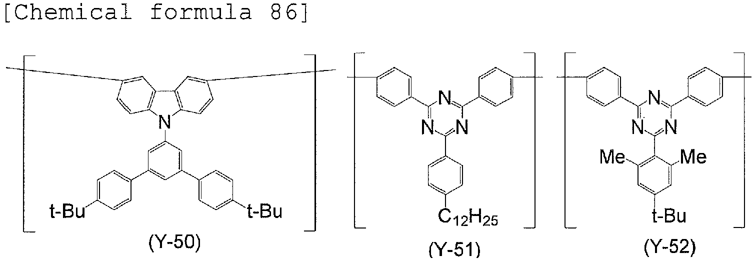

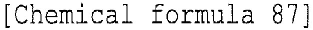

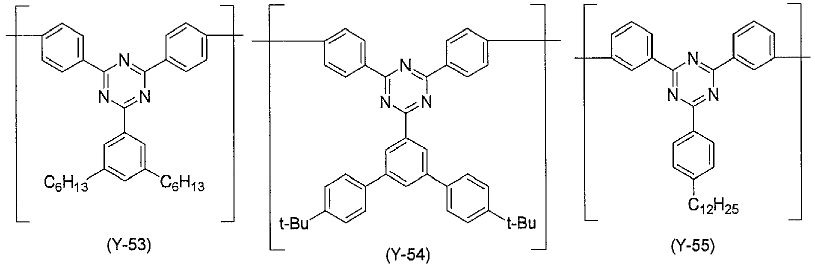

The amount of the constitutional unit represented by the formula (Y) (wherein, Ar^{Y1} is an arylene group) is preferably 0.5 to 70 mol%, more preferably 5 to 60 mol% with respect to the total amount of constitutional units contained in the polymer compound, because the light emitting device which is more excellent in the luminance life can be produced.

The amount of the constitutional unit represented by the formula (Y) (wherein, Ar^{Y1} is a divalent heterocyclic group or a divalent group in which at least one arylene group and at least one divalent heterocyclic group are bonded directly to each other) is preferably 0.5 to 30 mol%, more preferably 3 to 40 mol% with respect to the total amount of constitutional units contained in the polymer compound, because the light emitting device which is excellent in the charge transportability can be produced.

The constitutional unit represented by the formula (Y) may be contained only singly or two or more units thereof may be contained in the polymer compound.

For the polymer compound comprising a constitutional unit having a cross-linkable group and a phosphorescent constitutional unit in the present invention, a polymer compound comprising a constitutional unit having a group represented by the formula (13) and a phosphorescent constitutional unit is preferable, because a light emitting device which is excellent in the luminous efficiency can be produced. 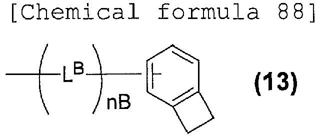 [wherein,
nB represents an integer of 1 to 5.
L^{B} represents an alkylene group, a cycloalkylene group, an arylene group, a divalent heterocyclic group, a group represented by -NR'-, an oxygen atom or a sulfur atom, and these groups optionally have a substituent. R' represents a hydrogen atom, an alkyl group, a cycloalkyl group, an aryl group or a monovalent heterocyclic group, and these groups optionally have a substituent. When a plurality of L^{B} are present, they may be the same or different.
The benzocyclobutene ring optionally has a substituent. When a plurality of the substituents are present, they may be the same or different, and they may be combined together to form a ring together with the carbon atoms to which they are attached.]

L^{B} is preferably an alkylene group or an arylene group.

The constitutional unit having the group represented by the above-described formula (13) includes, for example, the formula (11-2), the formula (11-5), the formula (11-6), the formula (11-9), the formula (11-11), the formula (11-12), the formula (11-23), the formula (11-30) and the formula (12-2).

The above-described phosphorescent constitutional unit are the same as described above.

The polymer compound comprising the constitutional unit having a group represented by the above-described formula (13) and a phosphorescent constitutional unit may further comprise at least one constitutional unit selected from the group consisting of a constitutional unit represented by the formula (X) and a constitutional unit represented by the formula (Y). These constitutional units represented by the formula (X) and the formula (Y) are the same as described above.

The amount of the constitutional unit represented by the formula (13) is preferably 0.5 to 40 mol%, more preferably 5 to 40 mol%, and further preferably 10 to 40 mol% with respect to the total amount of 100 mol% of the constitutional units contained in the polymer compound comprising the constitutional unit having a group represented by the above-described formula (13) and a phosphorescent constitutional unit, because a polymer compound which is excellent in crosslinkability can be obtained.

The polymer compound comprising the constitutional unit having a cross-linkable group and a phosphorescent constitutional unit may be particularly as long as it is a polymer compound that emits phosphorescence in a visible range (400 nm to 700 nm), because the luminous efficiency of the light emitting device is excellent. The polymer compound has an emission peak wavelength of preferably 490 nm to 700 nm, more preferably 550 nm to 700 nm, and further preferably 570 nm to 700 nm.

The polymer compound comprising a constitutional unit having a cross-linkable group and a phosphorescent constitutional unit may comprise the phosphorescent constitutional unit singly or two or more phosphorescent constitutional units. The phosphorescent constitutional unit is preferably contained singly, because the luminous efficiency of the light emitting device is excellent. That is, a polymer compound comprising a constitutional unit represented by the formula (13) and a phosphorescent constitutional unit preferably comprises the phosphorescent constitutional unit singly.

The polymer compound comprising a cross-linkable constitutional unit and a phosphorescent constitutional unit includes, for example, the polymer compounds P-1 to P-8 in Table 1.

**(Table 1)**

| Polymer compound | Constitutional unit and its mole ratio | | | | | | |
|---|---|---|---|---|---|---|---|
| | Constitutional unit having cross-linkable group | Phosphorescent constitutional unit | Formula (X) | Formula (Y) | | | Other |
| | Formulae | Formulae | Formulae | Formulae | Formulae | Formulae | |
| | (11)-(12) | (1G)-(4G) | (X-1) | (Y-1) | (Y-3) | (Y-5) | |
| | | | -(X-7) | -(Y-2) | -(Y-4) | -(Y-7) | |
| | p | q | r | s | t | u | v |
| P-1 | 0.1 to 40 | 0.1 to 30 | 0 | 0.1 to 99.8 | 0 | 0 | 0 to 30 |
| P-2 | 0.1 to 40 | 0.1 to 30 | 0.1 to 99.7 | 0.1 to 99.7 | 0 | 0 | 0 to 30 |
| P-3 | 0.1 to 40 | 0.1 to 30 | 0 | 0.1 to 99.7 | 0.1 to 99.7 | 0 | 0 to 30 |
| P-4 | 0.1 to 40 | 0.1 to 30 | 0 | 0.1 to 99.7 | 0 | 0.1 to 99.7 | 0 to 30 |
| P-5 | 0.1 to 40 | 0.1 to 30 | 0.1 to 99.6 | 0.1 to 99.6 | 0.1 to 99.6 | 0 | 0 to 30 |
| P-6 | 0.1 to 40 | 0.1 to 30 | 0.1 to 99.6 | 0.1 to 99.6 | 0 | 0.1 to 99.6 | 0 to 30 |
| P-7 | 0.1 to 40 | 0.1 to 30 | 0 | 0.1 to 99.6 | 0.1 to 99.6 | 0.1 to 99.6 | 0 to 30 |
| P-8 | 0.1 to 40 | 0.1 to 30 | 0.1 to 99.5 | 0.1 to 99.5 | 0.1 to 99.5 | 0.1 to 99.5 | 0 to 30 |

[In the Table, p, q, r, s, t, u and v denote molar ratio of the respective constitutional units. p+q+r+s+t+u+v=100, and 100≥p+q+r+s+t+u≥70. The other constitutional unit denotes any constitutional unit other than the constitutional unit having a cross-linkable group, the phosphorescent constitutional unit, the constitutional unit represented by the formula (X) and the constitutional unit represented by the formula (Y).]

The polymer compound comprising a constitutional unit having a cross-linkable group and a phosphorescent constitutional unit preferably exhibits a desired luminescent color in the light emitting device. The emission peak wavelength of the polymer compound comprising a constitutional unit having a cross-linkable group and a phosphorescent constitutional unit can be confirmed, for example, by measuring the emission peak wavelength at the time of voltage application for an evaluation light emitting device having a film of the polymer compound formed between a cathode and an anode. The materials of the cathode and the anode used for the evaluation device, film thickness of the polymer compound and the voltage to be applied may be determined suitably in accordance with the light emitting device intended as a product. In the evaluation element, the polymer compound may be crosslinked or not, but it is preferably crosslinked under a crosslinking reaction condition to be applied during production of the light emitting device intended as a product.

Described next is a method for producing the polymer compound comprising a constitutional unit having a cross-linkable group and a phosphorescent constitutional unit.

It can be produced by, for example, condensation polymerization of at least one compound selected from the group consisting of compounds represented by the formula (M-1) and the formula (M-2), at least one compound selected from the group consisting of compounds represented by the formula (M-3) to the formula (M-6), and further, if necessary, at least one compound selected from the group consisting of compounds represented by the formula (M-7) and the formula (M-8). In the present specification, the compounds used in production of a polymer compound may be collectively referred to as a "raw material monomer." The compounds represented by the formula (M-1) and the formula (M-2) are raw material monomers to provide constitutional units having a cross-linkable group, more specifically, the constitutional units each represented by the formula (11) and the formula (12). The compounds represented by the formula (M-3) to the formula (M-6) are raw material monomers to provide a phosphorescent constitutional unit, more specifically, constitutional units each represented by the formula (1G) to the formula (4G). The compounds represented by the formula (M-7) and the formula (M-8) are raw material monomers to provide constitutional units each represented by the formula (X) and the formula (Y). The compound represented by the formula (M-3) preferably may be used as a terminal encapsulating material as having been explained for the constitutional unit represented by the formula (1G). 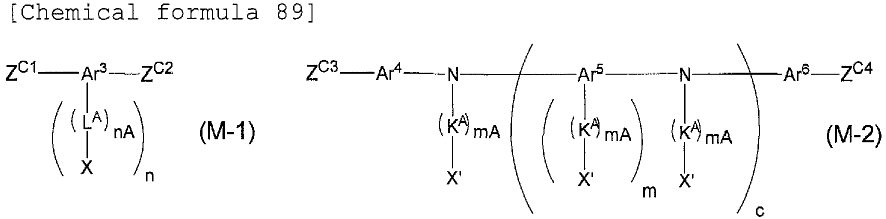 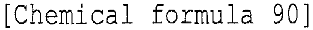 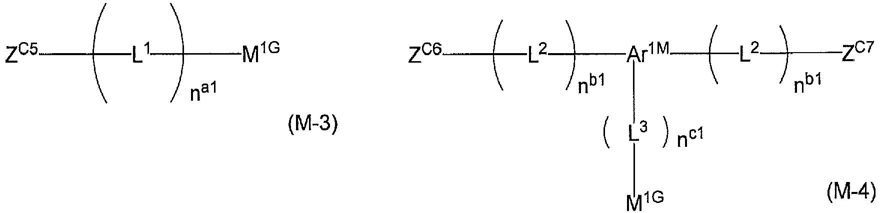 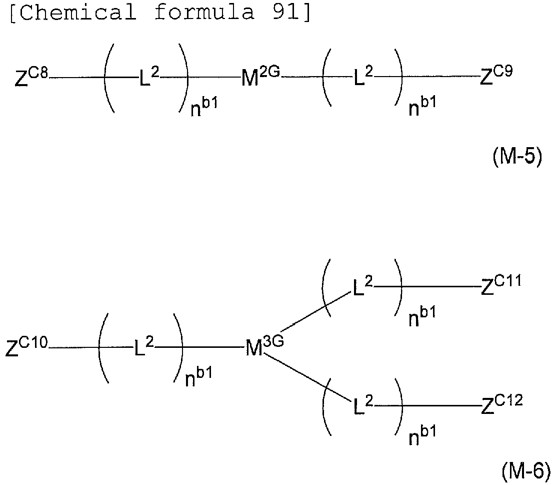 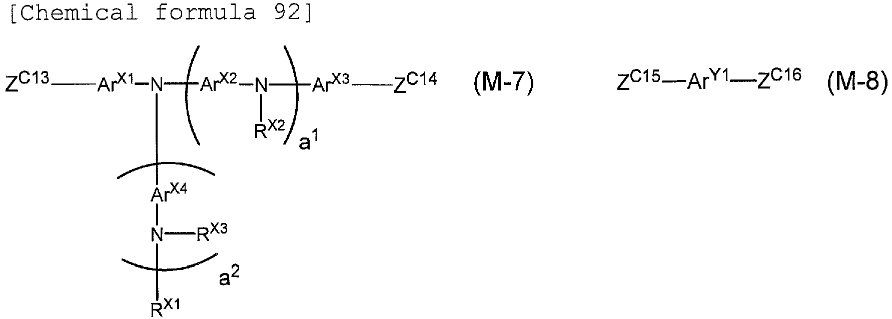 [wherein,
nA, n, Ar³, L^{A}, X, mA, m, c, Ar⁴ to Ar⁶, K^{A}, X', L¹, L², L³ n^{al}, n^{b1}, n^{c1}, M^{1G}, M^{2G}, M^{3G}, Ar^{Y1}, a¹, a², Ar^{X1} to Ar^{X4} and R^{X1} to R^{X3} represent the same meaning as described above.
Z^{C1} to Z^{C16} each independently represent a group selected from the group consisting of the Group A of substituent and the Group B of substituent.]

For example, when Z^{C1}, Z^{C2}, Z^{C3} and Z^{C4} are groups selected from the Group A of substituent, Z^{C5}, Z^{C6}, Z^{C7}, Z^{C8}, Z^{C9}, Z^{C10}, Z^{C11}, Z^{C12}, Z^{C13}, Z^{C14}, Z^{C15} and Z^{C16} are selected from the Group B of substituent.

For example, when Z^{C1}, Z^{C2}, Z^{C3} and Z^{C4} are groups selected from the Group B of substituent, Z^{C5}, Z^{C6}, Z^{C7}, Z^{C8}, Z^{C9}, Z^{C10}, Z^{C11}, Z^{C12}, Z^{C13} , Z^{C14}, Z^{C15} and Z^{C16} are selected from the Group A of substituent.

### < Group A of substituent >

A chlorine atom, a bromine atom, an iodine atom, and a group represented by -O-S(=O)₂R^{C1} (wherein, R^{C1} represents an alkyl group, a cycloalkyl group or an aryl group, and these groups optionally have a substituent).

### < Group B of substituent >

A group represented by -B(OR^{C2})2 (wherein, R^{C2} is a hydrogen atom, an alkyl group, a cycloalkyl group or an aryl group, and these groups optionally have a substituent. The plurality of R^{C2} may be the same or different, and they may be combined together to form a ring structure together with the oxygen atoms to which they are attached);
a group represented by -BF₃Q' (wherein, Q' represents Li, Na, K, Rb or Cs);
a group represented by -MgY' (wherein, Y' represents a chlorine atom, a bromine atom or an iodine atom);
a group represented by -ZnY'' (wherein, Y'' represents a chlorine atom, a bromine atom or an iodine atom); and
a group represented by -Sn(R^{C3})₃ (wherein, R^{C3} is a hydrogen atom, an alkyl group, a cycloalkyl group or an aryl group, and these groups optionally have substituent. The plurality of R^{C3} may be the same or different, and they may be combined together to form a ring structure together with the tin atoms to which they are attached).

The group represented by -B(OR^{C2})₂ includes, for example, groups represented by the following formulae.

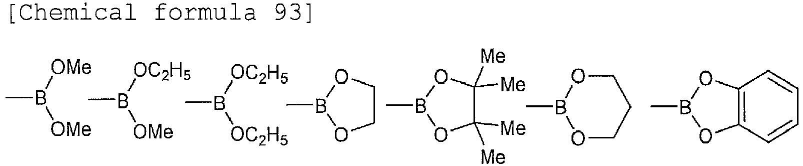

A compound having a group selected from the Group A of substituent and a compound having a group selected from the Group B of substituent are condensation-polymerized by a known coupling reaction, and a carbon atom bonded to the group selected from the group A of substituent and a carbon atom bonded to the group selected from the Group B of substituent are bonded to each other. For this reason, a compound having two groups selected from the Group A of substituent and a compound having two groups selected from the Group B of substituent are subjected to a known coupling reaction, thereby obtaining a condensation polymer of these compounds by a condensation polymerization.

Condensation-polymerization is usually performed under presence of a catalyst, a base and a solvent. A phase-transfer catalyst may coexist if necessary.

Examples of the catalyst include a transition metal complex such as a palladium complex like dichlorobis(triphenylphosphine)palladium, dichlorobis(tris-o-methoxyphenylphosphine)palladium, palladium[tetrakis(triphenylphosphine)], [tris(dibenzylideneacetone)]dipalladium and palladium acetate, and a nickel complex like nickel[tetrakis(triphenylphosphine)], [1,3-bis(diphenylphosphino)propane]dichloronickel and [bis(1,4-cyclooctadiene)]nickel; a complex of any of these transition metal complexes having further ligands such as triphenylphosphine, tri-o-tolylphosphine, tri-tert-butylphosphine, tricyclohexylphosphine, diphenylphosphinopropane and bipyridyl. The catalyst may be used singly, or two or more catalysts may be used in combination.

The usage amount of the catalyst is usually 0.00001 to 3 molar equivalent with respect to the total by the number of moles of the raw material monomer.

Examples of the base and the phase transition catalyst include inorganic bases such as sodium carbonate, potassium carbonate, cesium carbonate, potassium fluoride, cesium fluoride and tripotassium phosphate; organic bases such as tetrabutylammonium fluoride and tetrabutylammonium hydroxide; and phase transition catalysts such as tetrabutylammonium chloride and tetrabutylammonium bromide. The base and the phase transition catalyst each may be used singly, or two or more bases or phase transition catalysts may be used in combination.

Usage amount of each of the base and the phase transition catalyst is usually 0.001 to 100 molar equivalent with respect to the total number of moles of the raw material monomer.

Examples of the solvent include an organic solvent such as toluene, xylene, mesitylene, tetrahydrofuran, 1,4-dioxane, dimethoxyethane, N,N-dimethylacetamide, N,N-dimethylformamide and water. The solvent may be used singly, or two or more solvents may be used in combination.

The usage amount of the solvent is 10 to 100000 parts by weight with respect to the total of 100 parts by weight of the raw material monomer.

The reaction temperature of the condensation polymerization is usually from -100°C to 200°C. The reaction time for the condensation polymerization is usually 1 hour or more.

A post-treatment for the polymerization reaction is performed by any known method. Examples of the method may include a method of removing water-soluble impurities by separation and a method of adding a reaction liquid after the polymerization reaction to a lower alcohol such as methanol, filtrating a deposited precipitate and then drying the precipitate. These methods may be performed singly or in combination. When the purity of the polymer compound is low, it may be purified by common methods such as recrystallization, reprecipitation, continuous extraction by a Soxhlet extraction apparatus and column chromatography.

### <Second light-emitting layer>

A second light-emitting layer is a layer obtained by using a composition comprising a non-phosphorescent low molecular weight compound having a heterocyclic structure and at least two phosphorescent compounds.

The non-phosphorescent low molecular weight compound having a heterocyclic structure has at least one function selected from the group consisting of hole injection property, hole transportability, electron injection property and electron transportability. The low molecular weight compound may be contained singly, or two or more thereof may be contained in combination.

The amount of the at least two phosphorescent compounds in the second light-emitting layer is usually 0.05 to 80 parts by weight, preferably 0.1 to 50 parts by weight, and more preferably 0.5 to 40 parts by weight with respect to the total of 100 parts by weight of the material contained in the second light-emitting layer.

The lowest excited triplet state (T₁) which the non-phosphorescent low molecular weight compound having a heterocyclic structure has is preferably at an energy level equivalent to or higher than that of a phosphorescent compound having the highest T₁ energy among at least two phosphorescent compounds, because a light emitting device which is excellent in external quantum efficiency can be produced.

The non-phosphorescent low molecular weight compound having a heterocyclic structure is preferably dissolved in a solvent that is capable of dissolving the at least two phosphorescent compounds described above, because a light emitting device of the present invention can be fabricated in a solution application process.

The non-phosphorescent low molecular weight compound having a heterocyclic structure is preferably a compound having at least one heterocyclic structure selected from the group consisting of a carbazole ring, a phenanthroline ring, a triazine ring, an azole ring, a thiophene ring, a furan ring, a pyridine ring and a diazabenzene ring.

The non-phosphorescent low molecular weight compound having a heterocyclic structure is preferably a compound represented by the formula (H-1). 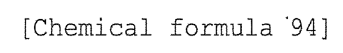 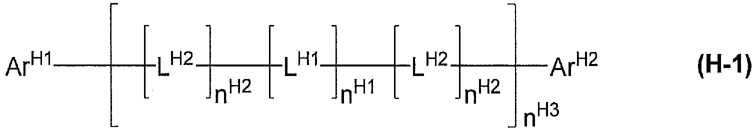 [wherein,
Ar^{H1} and Ar^{H2} represent an aryl group or a monovalent heterocyclic group, and these groups optionally have a substituent.
n^{H1} and n^{H2} each independently represent 0 or 1. When a plurality of n^{H1} are present, they may be the same or different. The plurality of n^{H2} may be the same or different.
n^{H3} represent an integer of 1 or more.
L^{H1} represents an arylene group or divalent heterocyclic group, and these groups optionally have a substituent. When a plurality of L^{H1} are present, they may be the same or different.
L^{H2} represents a group represented by -N(-L^{H3}-R^{HA})- or a group represented by - [C(R^{HB})2]n^{H4}-. R^{HA} represents an alkyl group, a cycloalkyl group, an aryl group or a monovalent heterocyclic group, and these groups optionally have a substituent. L^{H3} represents a single bond, an arylene group or divalent heterocyclic group, and these groups optionally have a substituent. R^{HB} represents a hydrogen atom, an alkyl group, a cycloalkyl group, an alkoxy group, a cycloalkoxy group, an aryl group or a monovalent heterocyclic group, and these groups optionally have a substituent. The plurality of R^{HB} may be the same or different, and they may be combined together to form a ring together with the carbon atoms to which they are attached. n^{H4} represents an integer of 1 to 10. When a plurality of L^{H2} are present, they may be the same or different.
At least one of Ar^{H1}, Ar^{H2}, L^{H1} and L^{H2} is a monovalent or divalent heterocyclic group or a group having a monovalent or divalent heterocyclic group.]

Ar^{H1} and Ar^{H2} is preferably a phenyl group, a fluorenyl group, a spirobifluorenyl group, a pyridyl group, a pyrimidinyl group, a triazinyl group, a quinolinyl group, an isoquinolinyl group, a thienyl group, a benzothienyl group, a dibenzothienyl group, a furyl group, a benzofuryl group, a dibenzofuryl group, a pyrrolyl group, an indolyl group, an azaindolyl group, a carbazolyl group, an azacarbazolyl group, a diazacarbazolyl group, a phenoxazinyl group or a phenothiazinyl group, more preferably a phenyl group, a spirobifluorenyl group, a pyridyl group, a pyrimidinyl group, a triazinyl group, a dibenzothienyl group, a dibenzofuryl group, a carbazolyl group or an azacarbazolyl group, further preferably a phenyl group, a pyridyl group, a carbazolyl group or an azacarbazolyl group, particularly preferably a group represented by the formula (TDA-1) or a group represented by the formula (TDA-3), especially preferably a group represented by the formula (TDA-3), and these groups optionally have a substituent.

A substituent which Ar^{H1} and Ar^{H2} optionally have is preferably a halogen atom, an alkyl group, a cycloalkyl group, an alkoxy group, a cycloalkoxy group, an aryl group or a monovalent heterocyclic group, more preferably an alkyl group, a cycloalkoxy group, an alkoxy group or a cycloalkoxy group, further preferably an alkyl group or a cycloalkoxy group, and these groups optionally further have a substituent.

L^{H1} is preferably a group represented by the formula (A-1) to the formula (A-3), the formula (A-8) to the formula (A-10), the formula (AA-1) to the formula (AA-6), the formula (AA-10) to the formula (AA-21), the formula (AA-24) to the formula (AA-34), more preferably a group represented by the formula (A-1), the formula (A-2), the formula (A-8), the formula (A-9), the formula (AA-1) to the formula (AA-4), the formula (AA-10) to the formula (AA-15), the formula (AA-29) to the formula (AA-34), further preferably a group represented by the formula (A-1), the formula (A-2), the formula (A-8), the formula (A-9), the formula (AA-2), the formula (AA-4), the formula (AA-10) to the formula (AA-15), particularly preferably a group represented by the formula (A-1), the formula (A-2), the formula (A-8), the formula (AA-2), the formula (AA-4), the formula (AA-10), the formula (AA-12) or the formula (AA-14), and especially preferably a group represented by the formula (A-1), the formula (A-2), the formula (AA-2), the formula (AA-4) or the formula (AA-14).

A substituent which L^{H1} optionally has is preferably a halogen atom, an alkyl group, a cycloalkyl group, an alkoxy group, a cycloalkoxy group, an aryl group or a monovalent heterocyclic group, more preferably an alkyl group, an alkoxy group, an aryl group or a monovalent heterocyclic group, further preferably an alkyl group, an aryl group or a monovalent heterocyclic group, and these groups optionally further a substituent.

Examples of the aryl group and the monovalent heterocyclic group in substituents which L^{H1} optionally has are the same as the examples of the aryl group and the monovalent heterocyclic group represented by Ar^{H1} and Ar^{H2}.

R^{HA} is preferably an aryl group or a monovalent heterocyclic group, and these groups optionally have a substituent.

Examples of the aryl group and the monovalent heterocyclic group represented by R^{HA} are the same as the examples of the aryl group and the monovalent heterocyclic group represented by Ar^{H1} and Ar^{H2}.

Definition and examples of a substituent which R^{HA} optionally has are the same as the definition and the examples of the substituent which Ar^{H1} and Ar^{H2} optionally have.

Examples of an arylene group and a divalent heterocyclic group represented by L^{H3} are the same as the examples of the arylene group and the divalent heterocyclic group represented by L^{H1}_{.}

Examples of the substituent which L^{H3} optionally has are the same as the examples of the substituent which L^{H1} optionally has.

R^{HB} is preferably a hydrogen atom, an alkyl group, a cycloalkyl group, an aryl group or a monovalent heterocyclic group, more preferably a hydrogen atom, an alkyl group or a cycloalkyl group, further preferably a hydrogen atom or an alkyl group, and these groups optionally have a substituent.

n^{H4} is preferably 1 to 5, more preferably 1 to 3, and further preferably 1.

n^{H1} is preferably 1.

n^{H2} is preferably 0.

n^{H3} is usually 1 to 10, preferably 1 to 5, further preferably 1 to 3, and particularly preferably 1.

In the formula (H-1), at least one of Ar^{H1}, Ar^{H2}, L^{H1} and L^{H2} is a monovalent or divalent heterocyclic group or a group having a monovalent or divalent heterocyclic group. Specifically, regarding Ar^{H1}, Ar^{H2}, L^{H1} (when a plurality thereof are present, at least one L^{H1}) and L^{H2} (when a plurality thereof are present, at least one L^{H2}), at least one of the following i) to v) is satisfied: i) Ar^{H1} is a monovalent heterocyclic group; ii) Ar^{H2} is a monovalent heterocyclic group; iii) L^{H1} is a divalent heterocyclic group; iv) L^{H2} is a group represented by -N(-L^{H3}-R^{HA}) -, and L^{H3} is a divalent heterocyclic group or R^{HA} is a monovalent heterocyclic group; v) L^{H2} is a group represented by -[C(R^{HB}) 2]n^{H4}-, and R^{HB} is a monovalent heterocyclic group.

The compound represented by the formula (H-1) is preferably a compound represented by the formula (H-2). 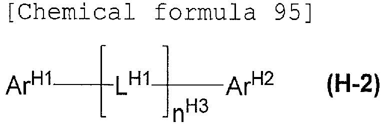 [wherein, L^{H1}, n^{H3}, Ar^{H1} and Ar^{H2} represent the same meaning as described above. At least one of Ar^{H1}, Ar^{H2} and L^{H1} is a monovalent heterocyclic group or a divalent heterocyclic group.]

Examples of the compound represented by the formula (H-1) include the compounds represented by the following formulae.

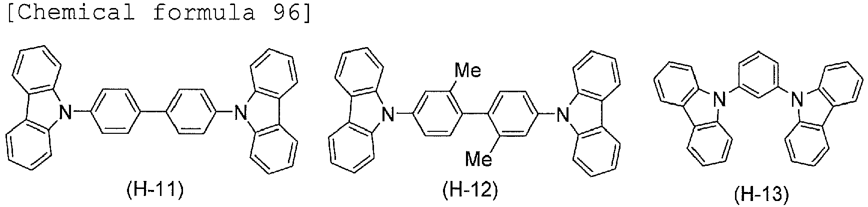

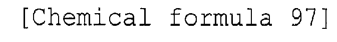

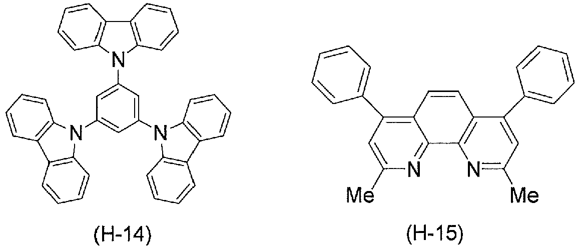

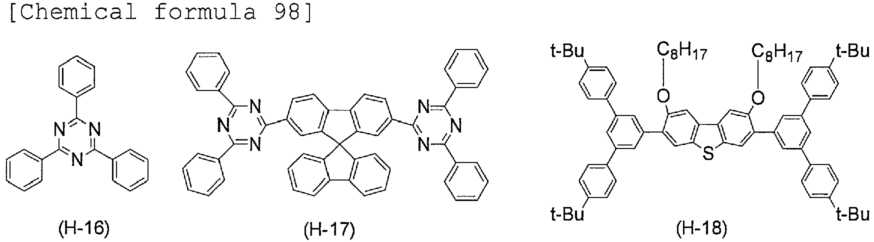

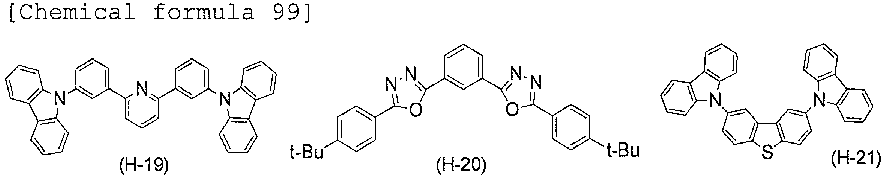

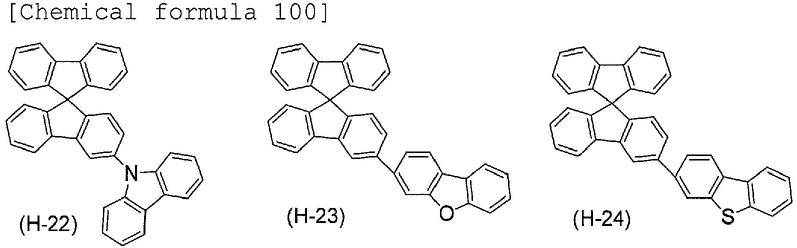

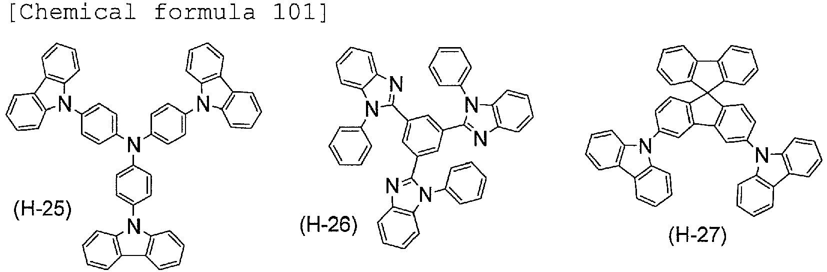

For the at least two phosphorescent compounds, any phosphorescent compound having a high luminescent quantum yield at room temperature can be used preferably.

From the standpoint of efficiency, the at least two phosphorescent compounds preferably comprise at least one phosphorescent compound having an emission spectrum the maximum peak wavelength of which is between 400 nm or more and less than 480 nm (B), and at least one phosphorescent compound having an emission spectrum the maximum peak wavelength of which is between 480 nm or more and less than 680 nm (G).

The emission spectrum maximum peak wavelength of the phosphorescent compound can be evaluated by dissolving the phosphorescent compound in an organic solvent such as toluene, xylene, chloroform, tetrahydrofuran and the like to prepare a dilute solution (about 1×10⁻⁶ to 1×10⁻³ wt%) and measuring the PL spectrum of the dilute solution at room temperature. The organic solvent for dissolving the phosphorescent compound is preferably xylene.

When the second light-emitting layer is a layer obtained by using a composition comprising a non-phosphorescent low molecular weight compound having a heterocyclic structure, the phosphorescent compound (B) and the phosphorescent compound (G), the amount of the phosphorescent compound (B) in the second light-emitting layer is usually 75.0 to 99.9 parts by weight, preferably 90.0 to 99.9 parts by weight, and more preferably 98 to 99.5 parts by weight with respect to the total of 100 parts by weight of the phosphorescent compound (B) and the phosphorescent compound (G).

The second light-emitting layer of the present invention may contain three or more phosphorescent compounds.

The above-described phosphorescent compound (B) includes, for example, a phosphorescent compound represented by the formula (1). 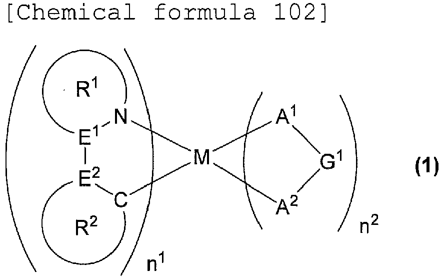 [wherein,
M represents a ruthenium atom, a rhodium atom, a palladium atom, an iridium atom or a platinum atom.
n¹ represents an integer of 1 or more, n² represents an integer of 0 or more, and n¹+n² is 2 or 3. When M is a ruthenium atom, a rhodium atom or an iridium atom, n¹+n² is 3. When M is a palladium atom or a platinum atom, n¹+n² is 2.
E¹ and E² each independently represent a carbon atom or a nitrogen atom, and at least one of E¹ and E² is a carbon atom.

The ring R¹ represents a five-membered or a six-membered aromatic heterocyclic ring, and these rings optionally have a substituent. When a plurality of the substituents are present, they may be the same or different, and they may be combined together to form a ring together with the atoms to which they are attached. When a plurality of R¹ are present, they may be the same or different. E¹ is a carbon atom when the ring R¹ is a six-membered aromatic heterocyclic ring.

The ring R² is a five-membered or six-membered aromatic carbon ring, or a five-membered or six-membered aromatic heterocyclic ring, and these rings optionally have a substituent. When a plurality of the substituents are present, they may be the same or different, and they may be combined together to form a ring together with the atoms to which they are attached. When a plurality of ring R² are present, they may be the same or different. E² is a carbon atom when the ring R² is a six-membered aromatic heterocyclic ring.

The ring R² has an electron-withdrawing group when the ring R¹ is a six-membered aromatic heterocyclic ring.

A¹-G¹-A² represents an anionic bidentate ligand. A¹ and A² each independently represent a carbon atom, an oxygen atom or a nitrogen atom, and these atoms may be atoms to constitute a ring. G¹ represents a single bond or an atomic group constituting the bidentate ligand together with A¹ and A². When a plurality of A¹-G¹-A² are present, they may be the same or different.]

When M is a ruthenium atom, a rhodium atom or an iridium atom, n¹ is preferably 2 or 3, and more preferably 3.

When M is a palladium atom or a platinum atom, n¹ is preferably 1 or 2, and more preferably 2.

The ring R¹ is preferably a five-membered aromatic heterocyclic ring having 1 to 3 nitrogen atoms as constitutional atoms, or a six-membered aromatic heterocyclic ring having 1 to 4 nitrogen atoms as constitutional atoms, more preferably a five-membered aromatic heterocyclic ring having 1 to 3 nitrogen atoms as constitutional atoms, and further preferably an imidazole ring or a triazole ring.

The ring R² is preferably a six-membered aromatic carbon ring, or a five-membered or six-membered aromatic heterocyclic ring, more preferably a benzene ring, a naphthalene ring, a fluorene ring, a phenanthrene ring, a pyridine ring, a diazabenzene ring, a pyrrole ring, a furan ring or a thiophene ring, further preferably a benzene ring, a naphthalene ring, a fluorene ring, a pyridine ring or a diazabenzene ring, particularly preferably a benzene ring, a pyridine ring or a pyrimidine ring, and these rings optionally have a substituent.

A substituent which the ring R¹ and the ring R² optionally have is preferably an alkyl group, a cycloalkyl group, an alkoxy group, a cycloalkoxy group, an aryl group, an aryloxy group, a monovalent heterocyclic group, a halogen atom or a dendron, more preferably an alkyl group, a cycloalkyl group, an aryl group, a monovalent heterocyclic group, a halogen atom or a dendron, further preferably an alkyl group, a cycloalkyl group, an aryl group, a halogen atom or a dendron, and these groups optionally further have a substituent. When the ring R¹ is a six-membered aromatic heterocyclic ring, the electron-withdrawing group which the ring R² has is preferably a halogen atom, an alkyl group or a cycloalkyl group having a halogen atom as a substituent, or an aryl having a halogen atom as a substituent, and more preferably a fluorine atom, an alkyl group or a cycloalkyl group having a fluorine atom as a substituent, or an aryl group having a fluorine atom as a substituent, and further preferably a fluorine atom, a trifluoromethyl group or a pentafluorophenyl group.

The anionic bidentate ligand represented by A¹-G¹-A² is the same as described above.

The phosphorescent compound represented by the the formula (1) includes, for example, a phosphorescent compound represented by the formula (1-A). 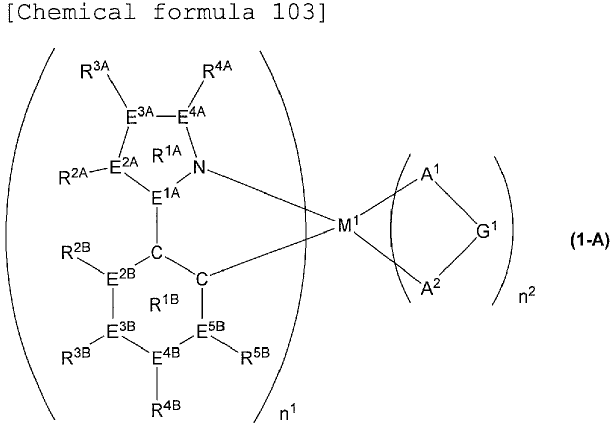 [wherein,
n¹,n² and A¹-G¹-A² represent the same meaning as described above.
M¹ represents an iridium atom or a platinum atom.
E^{1A}, E^{2A}, E^{3A}, E^{4A}, E^{2B}, E^{3B}, E^{4B} and E^{5B} each independently represent a nitrogen atom or a carbon atom. When a plurality of E^{1A}, E^{2A}, E^{3A}, E^{4A}, E^{2B}, E^{3B}, E^{4B}, and E^{5B} are present, they may be the same or different at each occurrence. When E^{2A}, E^{3A}, and E^{4A}, are nitrogen atoms, R^{2A}, R^{3A} and R^{4A} may be either present or not present. When E^{2B}, E^{3B}, E^{4B} and E^{5B} are nitrogen atoms, R^{2B}, R^{3B}, R^{4B} and R^{5B} are not present.
R^{2A}, R^{3A}, R^{4A}, R^{2B}, R^{3B}, R^{4B} and R^{5B} each independently represent a hydrogen atom, an alkyl group, a cycloalkyl group, an alkoxy group, a cycloalkoxy group, an aryl group, an aryloxy group, a monovalent heterocyclic group, a halogen atom or a dendron, and these groups optionally have a substituent. When a plurality of R^{2A}, R^{3A}, R^{4A}, R^{2B}, R^{3B}, R^{4B} and R^{5B} are present, they may be the same or different at each occurrence. R^{2A} and R^{3A}, R^{3A} and R^{4A}, R^{2A} and R^{2B}, R^{2B} and R^{3B}, R^{3B} and R^{4B}, and R^{4B} and R^{5B} each may be combined to form a ring together with the atoms to which they are attached.
The ring R^{1A} represents a triazole ring or an imizole ring constituted of a nitrogen atom, E^{1A}, E^{2A}, E^{3A}, and E^{4A}_{.}
The ring R^{1B} represents a benzene ring, a pyridine ring or a pyrimidine ring each constituted of two carbon atoms, E^{2B}, E^{3B}, E^{4B} and E^{5B}_{.}]

The bond between respective atoms constituting the ring R^{1A} is not necessarily a single bond but it may be a double bond. The bond between respective atoms constituting the ring R^{1B} is not necessarily a single bond but it may be a double bond. The phrase "when E^{2A}, E^{3A}, and E^{4A}, are nitrogen atoms, R^{2A}, R^{3A} and R^{4A} may be either present or not present." is synonymous with "when E^{2A} is a nitrogen atom, R^{2A} may be either present or not present; when E^{3A}, is a nitrogen atom, R^{3A} may be either present or not present; and when E^{4A} is a nitrogen atom, R^{4A} may be either present or not present."

R^{2A}, R^{3A}, R^{4A}, R^{2B}, R^{3B}, R^{4B} and R^{5B} are preferably a hydrogen atom, an alkyl group, a cycloalkyl group, an aryl group, a monovalent heterocyclic group, a halogen atom or a dendron, more preferably a hydrogen atom, an alkyl group, an aryl group, a halogen atom or a dendron, and these groups optionally have a substituent.

The phosphorescent compound represented by the formula (1-A) may be phosphorescent compounds represented by the formula (1-A1), the formula (1-A2), the formula (1-A3) or the formula (1-A4). 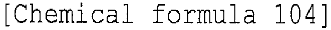 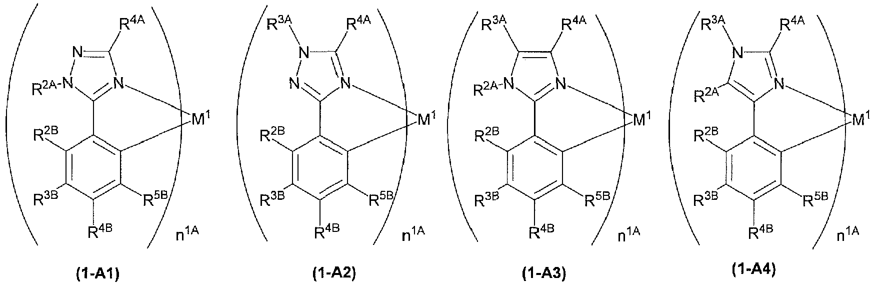 [wherein,
M¹, R^{2A}, R^{3A}, R^{4a}, R^{2B}, R^{3b}, R^{4B} and R^{5B} represent the same meaning as described above.
n^{1A} represents an integer of 2 or 3. When M¹ is iridium, n^{1A} is 3. When M¹ is platinum, n^{1A} is 2.]

The phosphorescent compound represented by the formula (1-A1) include, for example, phosphorescent compounds represented by the formula (1-A1-1) to the formula (1-A1-11). 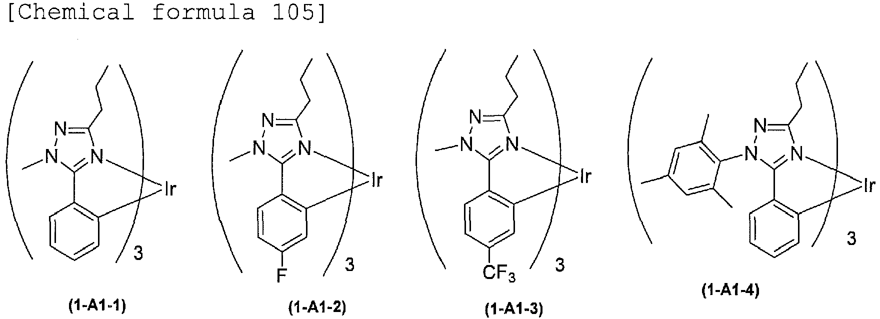 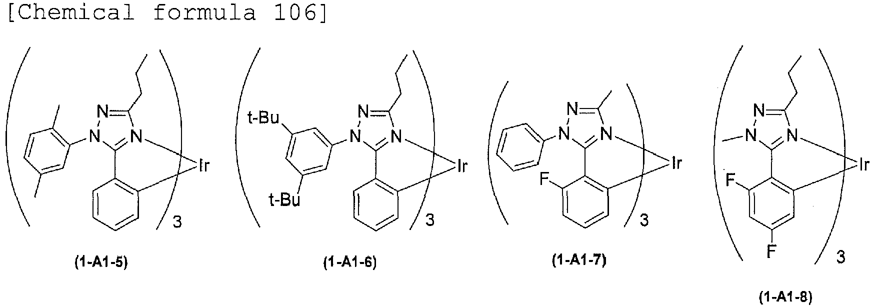 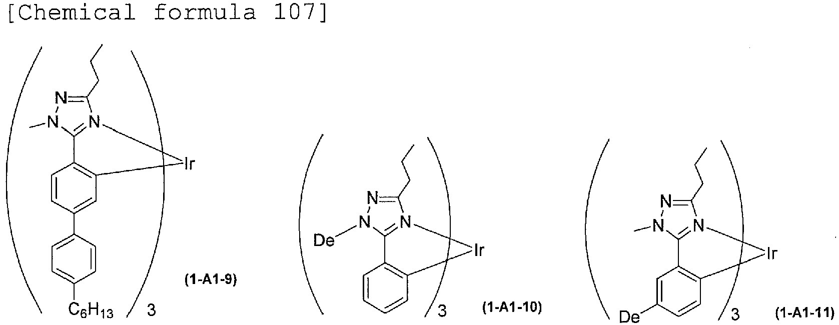 [wherein, De represents the same meaning as described above.]

In the formula (1-A1-11), an example in which De is a group represented by the formula (D-A) or the formula (D-B) is the following formula (1-A1-12).

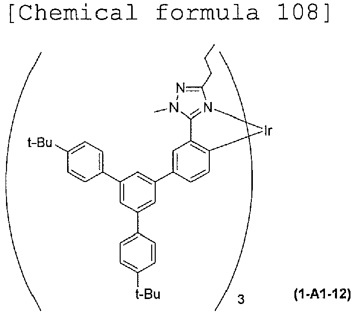

The phosphorescent compound represented by the above-described formula (1-A2) includes, for example, phosphorescent compounds represented by the formula (1-A2-1) to the formula (1-A2-11).

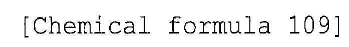

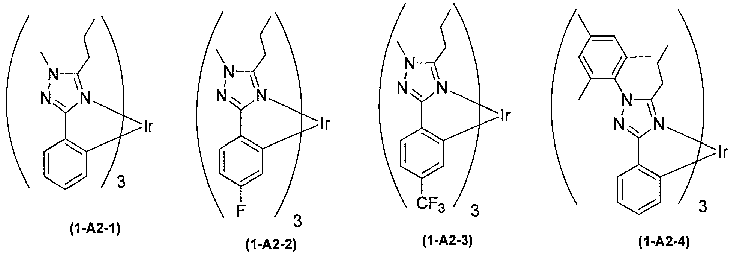

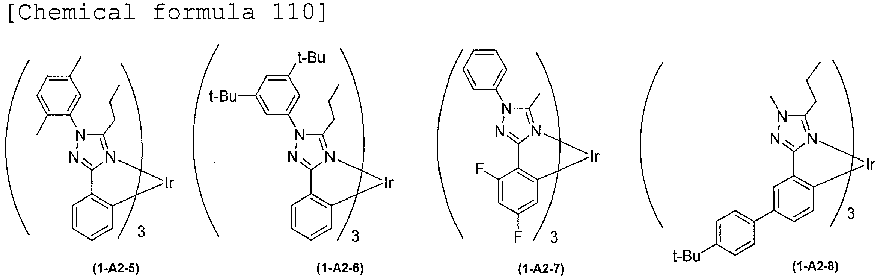

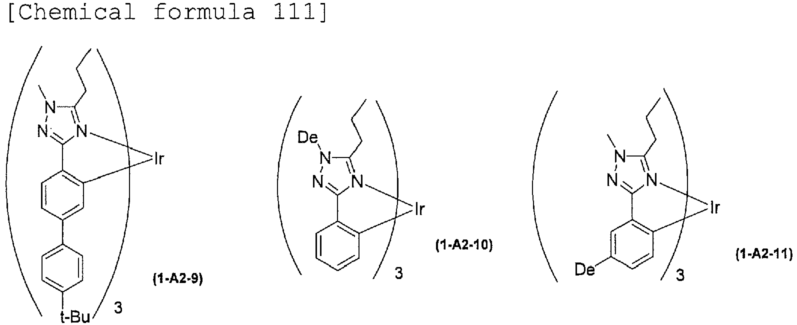

The phosphorescent compound represented by the formula (1-A3) includes, for example, phosphorescent compounds represented by the formula (1-A3-1) to the formula (1-A3-11).

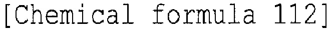

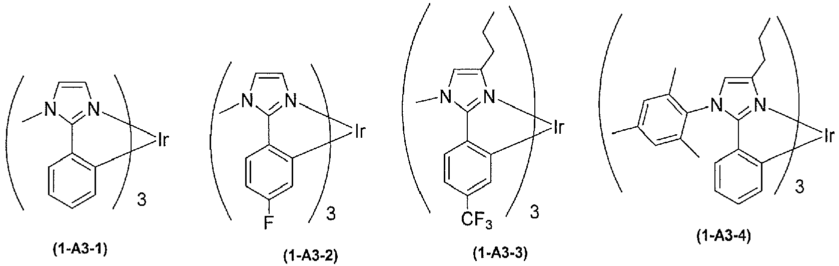

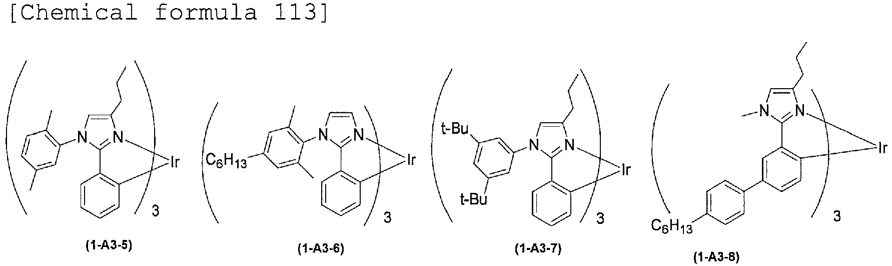

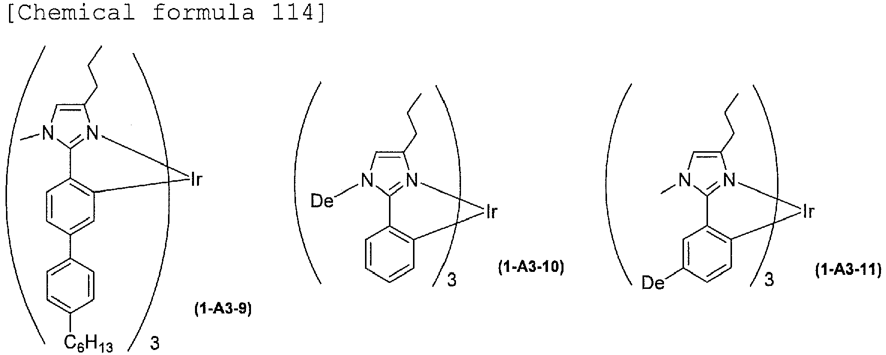

The phosphorescent compound represented by the formula (1-A4) includes, for example, phosphorescent compounds represented by the formula (1-A4-1) to the formula (1-A4-11).

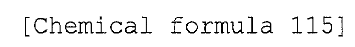

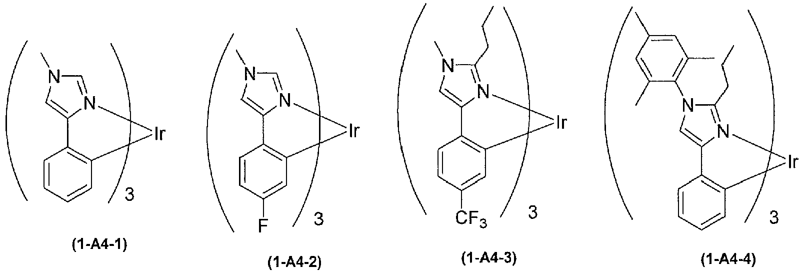

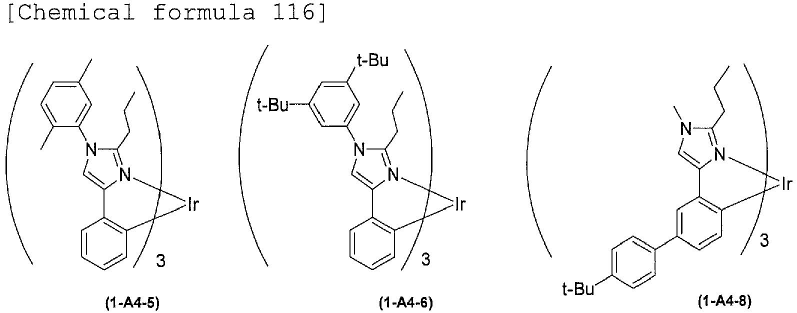

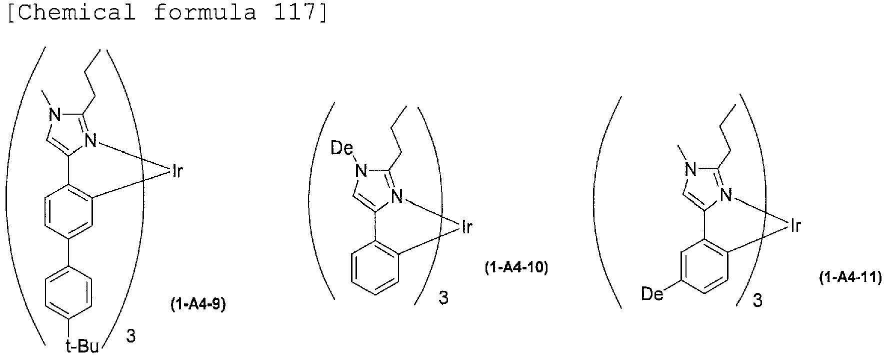

The phosphorescent compound (G) includes, for example, a phosphorescent compound represented by the formula (2). 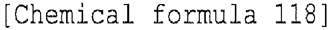 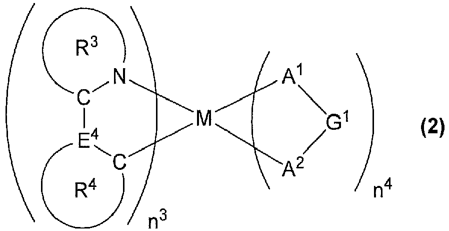 [wherein,
M represents a ruthenium atom, a rhodium atom, a palladium atom, an iridium atom or a platinum atom.
n³ represents an integer of 1 or more, n⁴ represents an integer of 0 or more, n³+n⁴ is 2 or 3. When M is a ruthenium atom, a rhodium atom or an iridium atom, n³+n⁴ is 3. When M is a palladium atom or a platinum atom, n³+n⁴ is 2.
E⁴ represents a carbon atom or a nitrogen atom.
The ring R³ represents a six-membered aromatic heterocyclic ring, and this ring optionally has a substituent. When a plurality of the substituents are present, they may be the same or different, and they may be combined together to form a ring together with the atoms to which they are attached. When a plurality of ring R³ are present, they may be the same or different.
The ring R⁴ represents a five-membered or six-membered aromatic carbon ring, or a five-membered or six-membered aromatic heterocyclic ring, and these rings optionally have a substituent. When a plurality of the substituents are present, they may be the same or different, and they may be combined together to form a ring together with the atoms to which they are attached. When a plurality of ring R⁴ are present, they may be the same of different. E⁴ is a carbon atom when the ring R⁴ is a six-membered aromatic heterocyclic ring.
A¹-G¹-A² represents an anionic bidentate ligand. A¹ and A² each independently represent a carbon atom, an oxygen atom or a nitrogen atom, and these atoms may be atoms constituting a ring. G¹ represents a single bond or an atomic group constituting the bidentate ligand together with A¹ and A². When a plurality of A¹-G¹-A² are present, they may be the same or different.]

When M is a ruthenium atom, a rhodium atom or an iridium atom, n³ is preferably 2 or 3, and more preferably 3.

When M is a palladium atom or a platinum atom, n¹ is preferably 1 or 2, and more preferably 2.

Examples of the ring R³ and the ring R⁴ each are the same as the examples of the ring R^{1G} and the ring R^{2G}. Moreover, examples of substituent which the ring R³ and ring R⁴ optionally have are the same as the examples of substituents which the ring R^{1G} and the ring R^{2G} optionally have.

The anionic bidentate ligand represented by A¹-G¹-A² is the same as described above.

For the phosphorescent compound (G), iridium complexes like metal complexes represented by the formula Ir-1 to the formula Ir-3 are preferable. 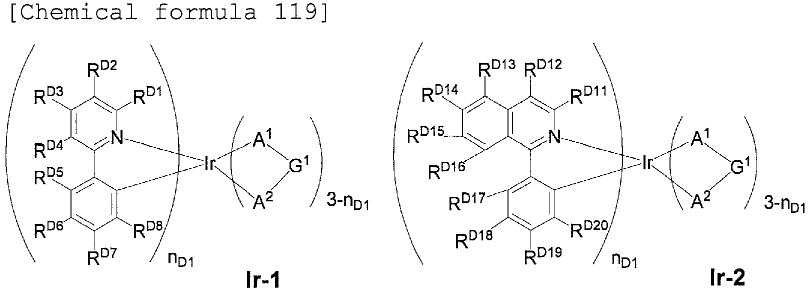 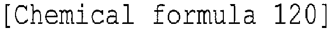 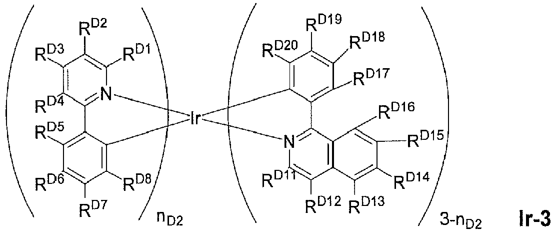 [wherein,
R^{D1} to R^{D8} and R^{D11} to R^{D20} each independently represent a hydrogen atom, an alkyl group, a cycloalkyl group, an alkoxy group, a cycloalkoxy group, an aryl group, an aryloxy group, a monovalent heterocyclic group, a halogen atom or a dendron, and these groups optionally have a substituent. When a plurality of R^{D1} to R^{D8} and R^{D11} to R^{D20} are present, they may be the same or different at each occurrence.
A¹-G¹-A² represents the same meaning as described above.
n_{D1} represents 1, 2 or 3. n_{D2} represents 1 or 2.]

In the structures represented by the formula Ir-1 to the formula Ir-3, at least one of the substituents is preferably a dendron, more preferably a group represented by the formula (D-A) or the formula (D-B), and further preferably a group represented by the formula (D-A).

The meal complex represented by the formula Ir-1 is preferably metal complexes represented by the formula Ir-11 to the formula Ir-13. The metal complex represented by the formula Ir-2 is preferably a metal complex represented by the formula Ir-21. The metal complex represented by the formula Ir-3 is preferably metal complexes represented by the formula Ir-31 to the formula Ir-33. 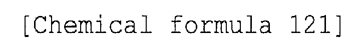 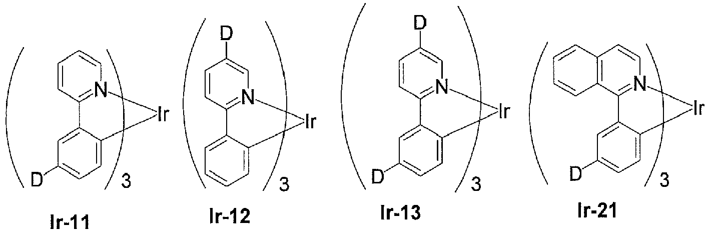 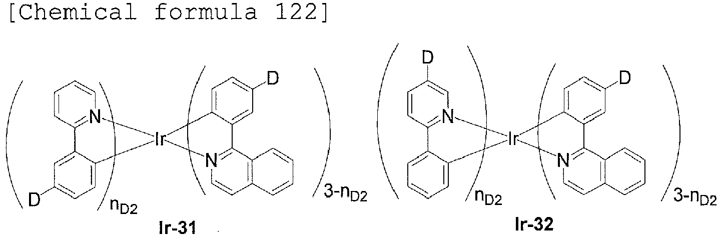 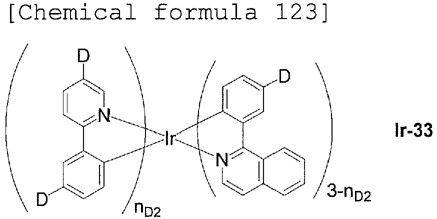 [wherein, D represents a group represented by the formula (D-A). n_{D2} represents the same meaning as described above.]

The above-described phosphorescent compound (G) includes, for example, the following metal complexes.

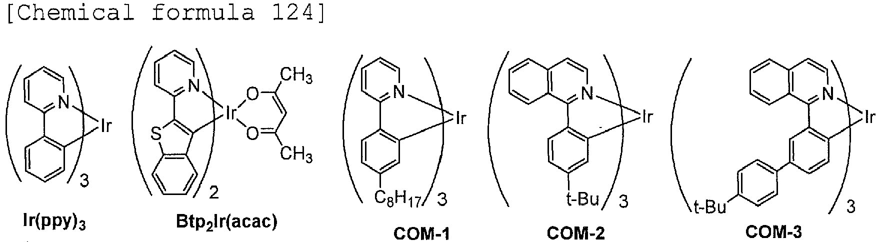

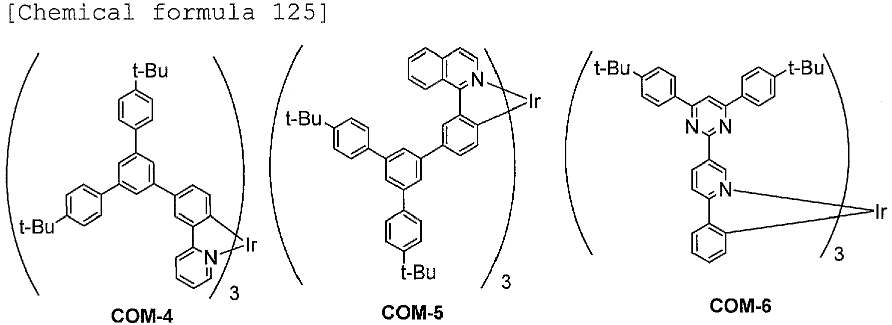

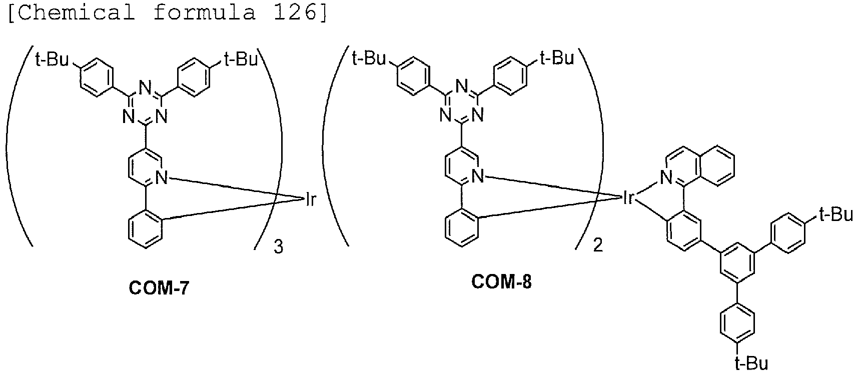

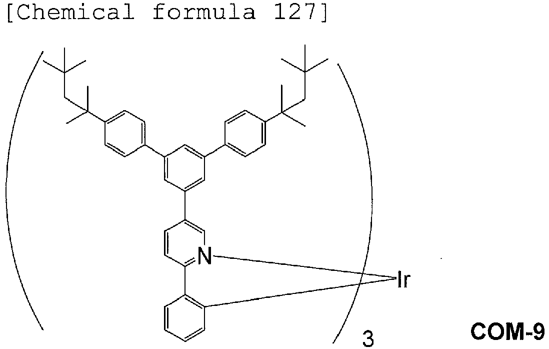

### [Layer constitution of light emitting device]

The light emitting device of the present invention comprises an anode, a cathode, and the first and second light-emitting layers provided between the anode and the cathode. The light emitting device of the present invention preferably further comprises at least one layer selected from the group consisting of a hole transport layer, a hole injection layer, an electron transport layer and an electron injection layer.

The light emitting device may include a layer strucure as described below, for example. In the following layer structure, the symbol "/" means that the layers written before and after the symbol are stacked adjacent to each other.
(D1) Anode / first light-emitting layer / second light-emitting layer / cathode
(D2) Anode / first light-emitting layer / second light-emitting layer / electron transport layer / cathode
(D3) Anode / first light-emitting layer / second light-emitting layer / electron injection layer / cathode
(D4) Anode / first light-emitting layer / second light-emitting layer / electron transport layer / electron injection layer / cathode
(D5) Anode / hole injection layer / first light-emitting layer / second light-emitting layer / cathode
(D6) Anode / hole injection layer / first light-emitting layer / second light-emitting layer / electron transport layer / cathode
(D7) Anode / hole injection layer / first light-emitting layer / second light-emitting layer / electron injection layer / cathode
(D8) Anode / hole injection layer / first light-emitting layer / second light-emitting layer / electron transport layer / electron injection layer / cathode
(D9) Anode / hole transport layer / first light-emitting layer / second light-emitting layer / cathode
(D10) Anode / hole transport layer / first light-emitting layer / second light-emitting layer / electron transport layer / cathode
(D11) Anode / hole transport layer / first light-emitting layer / second light-emitting layer / electron injection layer / cathode
(D12) Anode / hole transport layer / first light-emitting layer / second light-emitting layer / electron transport layer / electron injection layer / cathode
(D13) Anode / hole injection layer / hole transport layer / first light-emitting layer / second light-emitting layer / cathode
(D14) Anode / hole injection layer / hole transport layer / first light-emitting layer / second light-emitting layer / electron transport layer / cathode
(D15) Anode / hole injection layer / hole transport layer / first light-emitting layer / second light-emitting layer / electron injection layer / cathode
(D16) Anode / hole injection layer / hole transport layer / first light-emitting layer / second light-emitting layer / electron transport layer / electron injection layer / cathode
(D17) Anode / second light-emitting layer / first light-emitting layer / cathode
(D18) Anode / second light-emitting layer / first light-emitting layer / electron transport layer / cathode
(D19) Anode / second light-emitting layer / first light-emitting layer / electron injection layer / cathode
(D20) Anode / second light-emitting layer / first light-emitting layer / electron transport layer / electron injection layer / cathode
(D21) Anode / hole injection layer / second light-emitting layer / first light-emitting layer / cathode
(D22) Anode / hole injection layer / second light-emitting layer / first light-emitting layer / electron transport layer / cathode
(D23) Anode / hole injection layer / second light-emitting layer / first light-emitting layer / electron injection layer / cathode
(D24) Anode / hole injection layer / second light-emitting layer / first light-emitting layer / electron transport layer / electron injection layer / cathode
(D25) Anode / hole transport layer / second light-emitting layer / first light-emitting layer / cathode
(D26) Anode / hole transport layer / second light-emitting layer / first light-emitting layer / electron transport layer / cathode
(D27) Anode / hole transport layer / second light-emitting layer / first light-emitting layer / electron injection layer / cathode
(D28) Anode / hole transport layer / second light-emitting layer / first light-emitting layer / electron transport layer / electron injection layer / cathode
(D29) Anode / hole injection layer / hole transport layer / second light-emitting layer / first light-emitting layer / cathode
(D30) Anode / hole injection layer / hole transport layer / second light-emitting layer / first light-emitting layer / electron transport layer / cathode
(D31) Anode / hole injection layer / hole transport layer / second light-emitting layer / first light-emitting layer / electron injection layer / cathode
(D32) Anode / hole injection layer / hole transport layer / second light-emitting layer / first light-emitting layer / electron transport layer / electron injection layer / cathode

From the standpoint of external quantum efficiency, the first light-emitting layer is preferably provided between the anode and the second light-emitting layer. The light emitting device of the present invention may further comprise a functional layer having a fuction of emitting light, a function of inhibiting carrier transportation or a function of inhibiting diffusion of exiton, and the functional layer is provided between the first light-emitting layer and the second light-emitting layer. The first light-emitting layer and the second light-emitting layer are preferably adjacent to each other.

Preferably the light emitting device of the present invention further comprises at least one layer selected from the group consisting of a hole transport layer and a hole injection layer between the first light-emitting layer and the anode. Preferably the light emitting device of the present invention further comprises at least one layer selected from the group consisting of an electron transport layer and an electron injection layer between the second light-emitting layer and the cathode. These layers have usually a thickness of 1 nm to 10 µm.

In the light emitting device of the present invention, two or more hole injection layers, hole transport layers, electron transport layers and electron injection layers may be respectively provided if necessary.

### <Electron transport layer>

An electron transporting material used for the electron transport layer includes, for example, a compound comprising a structural unit represented by the formula (ET-1) or the formula (ET-2). The electron transporting material may be used singly, or two or more electron transporting materials may be used in combination. 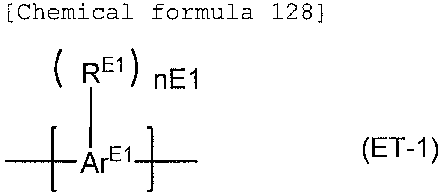 [wherein,
nE1 represent an integer of 1 or more.
Ar^{E1} represents a (nE1+2)-valent aromatic hydrocarbon group or a (nE1+2)-valent heterocyclic group, and these groups optionally have a substituent other than R^{E1}.
R^{E1} represents a group represented by the following formula (ES-1). When a plurality of R^{E1} are present, they may be the same or different.]

-(R^{E3})_{cE1}-(Q^{E1})_{nE4}-Y^{E1}(M^{E2})_{aE1}(Z^{E1})_{bE1} (ES-1)

[wherein,
cE1 represents 0 or 1, nE4 represents an integer of 0 or more, aE1 represents an integer of 1 or more, and bE1 represents an integer of 0 or more.
R^{E3} represents an arylene group or divalent heterocyclic group, and these groups optionally have a substituent.
Q^{E1} represents an alkylene group, an arylene group, an oxygen atom or a sulfur atom, and these groups optionally have a substituent. When a plurality of Q^{E1} are present, they may be the same or different.
Y^{E1} represents -CO₂⁻, -SO₃⁻, -SO₂⁻ or PO₃²⁻.
M^{E2} represents a metal cation or an ammonium cation, and the ammonium cation optionally has a substituent. When a plurality of M^{E2} are present, they may be the same or different.
Z^{E1} represents F⁻, Cl⁻, Br⁻, I⁻, OH⁻, R^{E4}SO₃⁻, R^{E4}COO⁻, ClO⁻, ClO₂⁻, ClO₃⁻, ClO₄⁻, SCN⁻, CN⁻, NO₃⁻, SO₄²⁻, HSO₄⁻, PO₄³⁻, HPO₄²⁻, H₂PO₄⁻, BF₄⁻ or PF₆⁻. R^{E4} represents an alkyl group, a cycloalkyl group or an aryl group, and these groups optionally have a substituent. When a plurality of Z^{E1} are present, they may be the same or different.
aE1 and bE1 are selected in such a manner that electric charge of the group represented by the formula (ES-1) becomes 0.]

The (nE1+2)-valent group represented by Ar^{E1} is preferably an atomic group remaining after removing from a divalent aromatic hydrocarbon group or heterocyclic group the nE1-number of hydrogen atoms linked directly to atoms constituting the ring, where the divalent aromatic hydrocarbon group or heterocyclic group is selected from the group consisting of a 1,4-phenylene group, a 1,3-phenylene group, a 1,2-phenylene group, a 2,6-naphthalenediyl group, a 1,4-naphthalenediyl group, a 2,7-fluorenediyl group, a 3,6-fluorenediyl group, a 2,7-phenanthrenediyl group and a 2,7-carbazolediyl group, and they optionally have a substituent other than R^{E1}.

The substituent which is other than R^{E1} and which the Ar^{E1} optionally has may be a halogen atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, cycloalkenyl group, an alkynyl group, a cycloalkynyl group, a carboxy group, an acyl group or a group represented by the formula (ES-3).

-O(C_{n'}H_{2n'}O)ₙₓC_{m'}H_{2m'+1} (ES-3)

[wherein,
n' and m' are integers of 1 or more,
nx is an integer of 1 or more representing the number of repetitions of an alkylene oxide part.]

nE1 is preferably an integer of 1 to 4, and more preferably 1 or 2. Q^{E1} is preferably an alkylene group, an arylene group or an oxygen atom. Y^{E1} is preferably -CO₂⁻, or -SO₃⁻. M^{E2} is preferably Li⁺, Na⁺, K⁺, Cs⁺, N(CH₃)₄⁺, NH(CH₃)₃⁺, NH₂(CH₃)₂⁺ or N(C₂H₅)₄⁺. Z^{E1} is preferably F⁻, Cl⁻, Br⁻, I⁻, OH⁻, R^{E4}SO₃⁻ or R^{E4}COO⁻.

The substituent which R^{E3} optionally has may be an alkyl group, a cycloalkyl group, an aryl group, a monovalent heterocyclic group or a group represented by the formula (ES-3). R^{E3} preferably has a group represented by the formula (ES-3) as a substituent, because the external quantum efficiency of the light emitting device of the present invention is excellent.

Examples of the group represented by the above-described formula (ES-1) include a group represented by the following formulae. 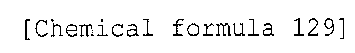 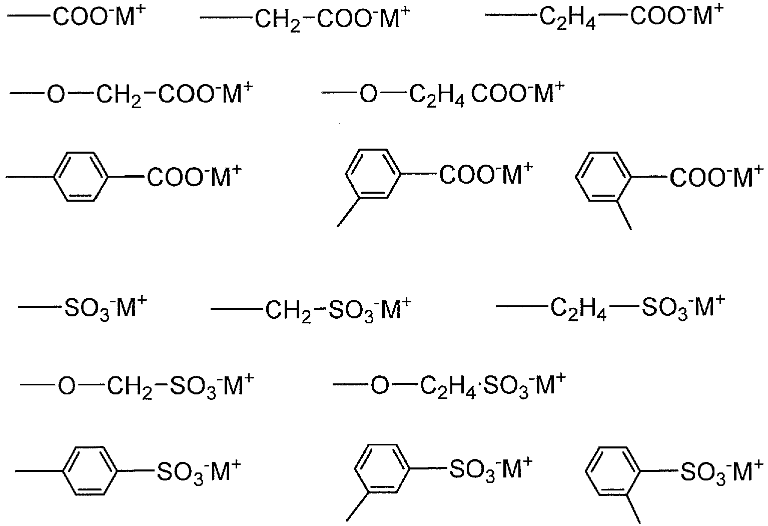 [wherein, M⁺ represents Li⁺, Na⁺, K⁺, Cs⁺, N(CH₃)₄⁺, NH(CH₃)₃⁺, NH₂(CH₃)₂⁺ and N(C₂H₅)₄⁺.] 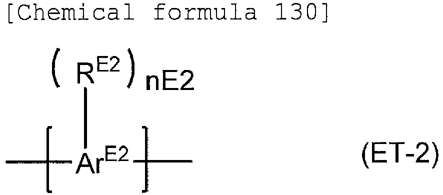 [wherein,
nE2 represents an integer of 1 or more.
Ar^{E2} represents a (nE2+2)-valent aromatic hydrocarbon group or a (nE2+2)-valent heterocyclic group, and these groups optionally have a substituent other than R^{E2}.
R^{E2} represents a group represented by the following formula (ES-2). When a plurality of R^{E2} are present, they may be the same or different.]

-(R^{E6})_{cE2}-(Q^{E2})_{nE6}-Y^{E2}(M^{E3})_{bE2}(Z^{E2})_{aE2} (ES-2)

[wherein,
cE2 represents 0 or 1, nE6 represents an integer of 0 or more, bE2 represents an integer of 1 or more, and aE2 represents an integer of 0 or more.
R^{E6} represents an arylene group or divalent heterocyclic group, and these groups optionally have a substituent.
Q^{E2} represents an alkylene group, an arylene group, an oxygen atom or a sulfur atom, and these groups optionally have a substituent. When a plurality of Q^{E2} are present, they may be the same or different.
Y^{E2} represents a carbocation, an ammonium cation, a phosphonyl cation or a sulfonyl cation.
M^{E3} represents F⁻, Cl⁻, Br⁻, I⁻, OH⁻, R^{E7}SO₃⁻, R^{E7}COO⁻, ClO⁻, ClO₂⁻, ClO₃⁻, ClO₄⁻, SCN⁻, CN⁻, NO₃⁻, SO₄²⁻, HSO₄⁻, PO₄³⁻, HPO₄²⁻, H₂PO₄⁻, tetraphenylborate, BF₄⁻ or PF₆⁻. R^{E7} represents an alkyl group, a perfluoroalkyl group or an aryl group, and these groups optionally have a substituent. When a plurality of M^{E3} are present, they may be the same or different.
Z^{E2} represents a metal ion or an ammonium ion, and this ammonium ion optionally has a substituent. When a plurality of Z^{E2} are present, they may be the same or different.
aE2 and bE2 are selected in such a manner that the electric charge of the group represented by the formula (ES-2) becomes 0.]

The (nE2+2)-valent group represented by Ar^{E2} is preferably an atomic group remaining after removing from a divalent aromatic hydrocarbon group or heterocyclic group the nE2-number of hydrogen atoms linked directly to atoms constituting the ring, where the divalent aromatic hydrocarbon group or heterocyclic group is selected from the group consisting of a 1,4-phenylene group, a 1,3-phenylene group, a 1,2-phenylene group, a 2,6-naphthalenediyl group, a 1,4-naphthalenediyl group, a 2,7-fluorenediyl group, a 3,6-fluorenediyl group, a 2,7-Phenanthrenediyl group and a 2,7-carbazolediyl group, and they optionally have a substituent other than R^{E2}.

The substituent which is other than R^{E2} and which Ar^{E2} optionally has is the same as the substituent which is other than R^{E1} and which Ar^{E1} optionally has.

nE2 is preferably an integer of 1 to 4, and more preferably 1 or 2. Q^{E2} is preferably an alkylene group, an arylene group or an oxygen atom. Y^{E2} is preferably a carbocation or an ammonium cation. M^{E3} is preferably F⁻, Cl⁻, Br⁻, I⁻, tetraphenylborate, CF₃SO₃⁻ or CH₃COO⁻.

The substituent which R^{E6} optionally has may be an alkyl group, a cycloalkyl group, an aryl group, a monovalent heterocyclic group or a group represented by the formula (ES-3). R^{E6} preferably has a group represented by the formula (ES-3) as a substituent, because the external quantum efficiency of the light emitting device of the present invention is excellent.

The group represented by the formula (ES-2) includes, for example, groups represented by the following formulae. 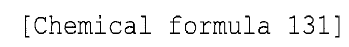 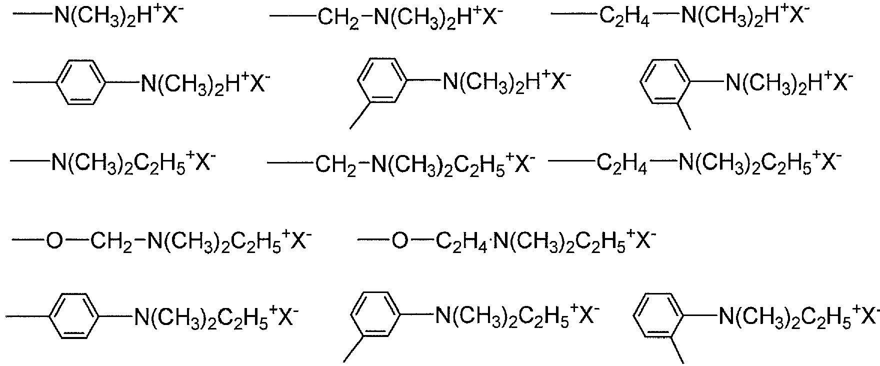 [wherein, X⁻ represents F⁻, Cl⁻, Br⁻, I⁻, tetraphenylborate, CF₃SO₃⁻ or CH₃COO⁻.]

The structural units represented by the formula (ET-1) and the formula (ET-2) include, for example, structural units represented by the following formula (ET-31) to formula (ET-34).

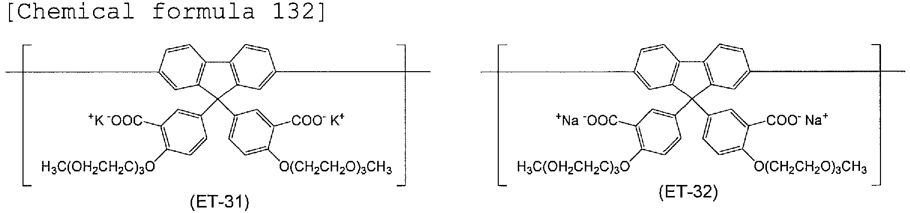

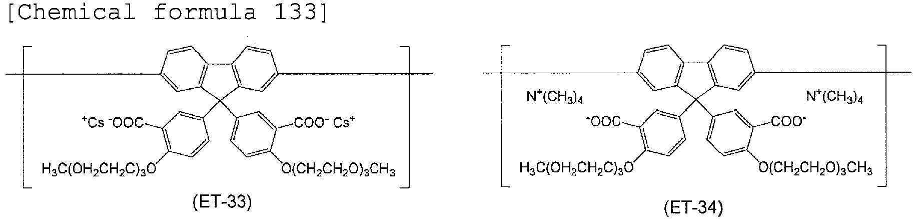

The compound comprising a structural unit represented by the above-described formula (ET-1) or formula (ET-2) may be a polymer compound, may be a low molecular weight compound comprising one structural unit of the formula (ET-1) or the formula (ET-2), and may be an oligomer comprising 2 to 5 structural units thereof.

### <Hole transporting layer>

The hole transporting material used in the hole transporting layer is classified into low molecular weight compounds and polymer compounds, and polymer compounds are preferable. The hole transporting material optionally has a crosslikable group.

The polymer compound includes, for example, polyvinylcarbazole and derivatives thereof; polyarylene having an aromatic amine structure in the side chain or main chain and derivatives thereof. The polymer compound may also be a compound in which an electron accepting portion is linked. The electron accepting portion includes, for example, fullerene, tetrafluorotetracyanoquinodimethane, tetracyanoethylene, trinitrofluorenone and the like, preferably fullerene.

The hole transporting material may be used singly, or two or more hole transporting materials may be used in combination.

### <Electron injection layer and hole injection layer>

The electron injection material and the hole injection material used in the electron injection layer and the hole injection layer are each classified into low molecular weight compounds and polymer compounds. The electron injection material and the hole injection material optionally has a cross-linkable group.

The low molecular weight compound includes, for example, metal phthalocyanines such as copper phthalocyanine; carbon; oxides of metals such as molybdenum and tungsten; metal fluorides such as lithium fluoride, sodium fluoride, cesium fluoride and potassium fluoride.

The polymer compound includes, for example, polyaniline, polythiophene, polypyrrole, polyphenylenevinylene, polythienylenevinylene, polyquinoline and polyquinoxaline, and derivatives thereof; electrically conductive polymers such as a polymer compound comprising an aromatic amine structure in the main chain or side chain.

The electron injection material and the hole injection material may each be used singly, or two or more of them may be used in combination.

### [Method for fabricating a light emitting device]

In fabricating a light emitting device of the present invention, a method of forming the first light-emitting layer, a second light-emitting layer, a hole transport layer, an electron transport layer, a hole injection layer, an electron injection layer and the like may be determined suitably depending on the materials to be used. When a low molecular weight compound is used, examples of the method include vacuum evaporation using a powdery material and a method of film formation using a material in a solution or fused state, and when a polymer compound is used, an example thereof is a method of film formation using a material in a solution or fused state.

The order and the number of the layers to be stacked and the thickness of the layers need to be adjusted considering external quantum efficiency and device life.

In fabrication of the light emitting device, the respective materials for the hole transport layer, the electron transport layer, the first light-emitting layer and the second light-emitting layer are preferably prevented from being dissolved in a solvent used for forming layers each adjacent to the hole transport layer, the electron transport layer, the first light-emitting layer and the second light-emitting layer at the time of dissolving the materials in the solvent. The method of preventing dissolution of the materials is preferably i) a method of using a material having a cross-linkable group or ii) a method of differentiating solubility of adjacent layers. In the method i), a layer can be insolubilized by forming the layer with a material having a cross-linkable group, and then crosslinking the cross-linkable group. In the method ii), layers adjacent to each other preferably have polarities different from each other.

The solvent to be used for the solution include, for example, chlorinated solvents such as 1,2-dichloroethane, 1,1,2-trichloroethane, chlorobenzene and o-dichlorobenzene; ether-based solvents such as tetrahydrofuran, dioxane, anisole and 4-methylanisole; aromatic hydrocarbon-based solvents such as toluene, xylene, mesitylene, ethylbenzene, n-hexylbenzene and cyclohexylbenzene; aliphatic hydrocarbon-based solvents such as cyclohexane, methylcyclohexane, n-pentane, n-hexane, n-heptane, n-octane, n-nonane, n-decane, n-dodecane and bicyclohexyl; and ketone-based solvents such as acetone, methyl ethyl ketone, cyclohexanone, benzophenone and acetophenone. The solvent may be used singly, or two or more solvents may be used in combination.

### [Substrate/electrode]

The substrate in the light emitting device may advantageously be a substrate on which an electrode can be formed and which does not chemically change in forming a functional layer, and is a substrate made of a material such as, for example, glass, plastic and silicon. In the case of an opaque substrate, it is preferable that an electrode most remote from the substrate is transparent or semi-transparent.

The material of the anode includes, for example, electrically conductive metal oxides and semi-transparent metals, preferably, indium oxide, zinc oxide and tin oxide; electrically conductive compounds such as indium·tin·oxide (ITO) and indium·zinc·oxide; a composite of silver, palladium and copper (APC); NESA, gold, platinum, silver and copper.

The material of the cathode includes, for example, metals such as lithium, sodium, potassium, rubidium, cesium, beryllium, magnesium, calcium, strontium, barium, aluminum, zinc and indium; alloys composed of two or more of them; alloys composed of one or more of them and at least one of silver, copper, manganese, titanium, cobalt, nickel, tungsten and tin; and graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy and a calcium-aluminum alloy.

The anode and the cathode may each take a lamination structure composed of two or more layers.

A polymer compound used for a first light-emitting layer comprises a constitutional unit having a cross-linkable group and a phosphorescent constitutional unit. Since the polymer compound has a cross-linkable group, it can be crosslinked by external stimuli such as heat and light irradiation so as to provide the first light-emitting layer. The crosslinked first light-emitting layer is substantially insoluble in a solvent, and thus it is preferably used for fabricating the light emitting device by formation of a film from a solution (which is called also "solution coating method").

The heating temperature to crosslink the first light-emitting layer is usually 25 to 300°C. It is preferably 50 to 250°C, and more preferably 150 to 200°C, because external quantum efficiency is improved.

The light used for light irradiation to crosslink the first light-emitting layer is, for example, ultraviolet light, near-ultraviolet light and visible light.

The first light-emitting layer can be formed from a solution (it is also called "ink") containing a polymer compound comprising a constitutional unit having a cross-linkable group and a phosphorescent constitutional unit by, for example, a spin coating method, a casting method, a microgravure coating method, a gravure coating method, a bar coating method, a roll coating method, a wire-bar coating method, a dip coating method, a spray coating method, a screen printing method, a flexo printing method, an offset printing method, an inkjet printing method, a capillary coating method or a nozzle coating method.

The thickness of the first light-emitting layer is usually from 1 nm to 10 µm.

The second light-emitting layer is obtained by using a composition comprising a non-phosphorescent low molecular weight compound comprising a heterocyclic structure and at least two phosphorescent compounds. In a case of formation from a solution or a fused state, the layer can be formed through the substantially same process as that for the first light-emitting layer.

The thickness of the second light-emitting layer is usually from 1 nm to 10 µm.

When an electron transport layer or an electron injection layer is stacked further on the second light-emitting layer by utilizing the difference in solubility, the electron transport layer or the electron injection layer can be stacked by using a solution having a low solubility with respect to the second light-emitting layer.

The solvent to be used for the solution is preferably water, alcohols, ethers, esters, nitrile compounds, nitro compounds, fluorinated alcohol, thiols, sulfides, sulfoxides, thioketones, amides, carboxylic acids or the like. Examples of the solvent include methanol, ethanol, 2-propanol, 1-butanol, tert-butyl alcohol, acetonitrile, 1,2-ethanediol, N,N-dimethylformamide, dimethyl sulfoxide, acetic acid, nitromethane, propylene carbonate, pyridine, carbon disulfide, and a mixture of these solvents. When a mixed solvent is used, it may be a mixed solvent of at least one solvent selected from the group consisting of water, alcohols, ethers, esters, nitrile compounds, nitro compounds, fluorinated alcohol, thiols, sulfides, sulfoxides, thioketones, amides and carboxylic acids and at least one solvent selected from the group consisting of a chlorinated solvent, an aromatic hydrocarbon-based solvent, an aliphatic hydrocarbon-based solvent and a ketone-based solvent.

### [Use of light emitting device]

For producing planar light emission by using a light emitting device, a planar anode and a planar cathode are disposed so as to overlap with each other. Patterned light emission can be produced by a method of placing a mask with a patterned window on the surface of a planer light emitting device, a method of forming extremely thick a layer intended to be a non-light emitting, thereby having the layer essentially no-light emitting or a method of forming an anode, a cathode or both electrodes in a patterned shape. By forming a pattern with any of these methods and disposing certain electrodes so as to switch ON/OFF independently, a segment type display capable of displaying numbers and letters and the like is provided. For producing a dot matrix display, both an anode and a cathode are formed in a stripe shape and disposed so as to cross with each other. Partial color display and multicolor display are made possible by a method of printing separately certain polymer compounds showing different emission or a method of using a color filter or a fluorescence conversion filter. The dot matrix display can be passively driven, or actively driven combined with TFT and the like. These displays can be used in computers, television sets, portable terminals and the like. The planar light emitting device can be suitably used as a planer light source for backlight of a liquid crystal display or as a planar light source for illumination. If a flexible substrate is used, it can be used also as a curved light source and a curved display.

### EXAMPLES

The present invention will be illustrated further in detail by examples below, but the present invention is not limited to these examples.

In the present examples, the polystyrene-equivalent number average molecular weight (Mn) and the polystyrene-equivalent weight average molecular weight (Mw) of polymer compounds were measured by a size exclusion chromatography (SEC) (manufactured by Shimadzu Corp., trade name: LC-10Avp). Measurement conditions of SEC were as described below.

### [Measurement condition]

A polymer compound to be measured was dissolved in THF at a concentration of about 0.05 wt%, and 10 µL of the solution was injected into SEC. As the mobile phase of SEC, THF was used, and was flowed at a flow rate of 2.0 mL/min. PLgel MIXED-B (manufactured by Polymer Laboratories) was used as a column. An UV-VIS detector (manufactured by Shimadzu Corp., trade name: SPD-10Avp) was used as a detector.

Liquid chromatograph mass spectrometry (LC-MS) was carried out according to the following method.

A measurement sample was dissolved in chloroform or THF at a concentration of about 2 mg/mL, and about 1 µL of the solution was injected into LC-MS (manufactured by Agilent Technologies, trade name: 1100LCMSD). As a mobile phase of LC-MS, acetonitrile and THF were used while changing the ratio thereof, and was flowed at a rate of 0.2 mL/min. L-column 2 ODS (3 µm) (manufactured by Chemicals Evaluation and Research Institute, internal diameter: 2.1 mm, length: 100 mm, particle size: 3 µm) was used as a column.

Measurement of NMR was carried out according to the following method.

5 to 10 mg of a measurement sample was dissolved in about 0.5 mL of deuterated chloroform (CDCl₃), deuterated tetrahydrofuran (THF-d₈) or deuterated methylene chloride (CD₂Cl₂), and measurement was performed using a NMR apparatus (manufactured by Varian, Inc., trade name:
MERCURY 300).

As the index of the purity of a compound, a value of the high performance liquid chromatography (HPLC) area percentage was used. This value was a value in high performance liquid chromatography (HPLC, manufactured by Shimadzu Corp., trade name: LC-20A) at 254 nm, unless otherwise state. In this operation, a compound to be measured was dissolved in THF or chloroform at a concentration of 0.01 to 0.2 wt%, and depending on the concentration, 1 to 10 µL of the solution was injected into the HPLC. As a mobile phase of HPLC, acetonitrile and THF were used, and were flowed at a flow rate of 1 mL/min as gradient analysis of acetonitrile/THF = 100/0 to 0/100 (volume ratio). Kaseisorb LC ODS 2000 (manufactured by Tokyo Chemical Industry Co., Ltd.) or an ODS column having an equivalent performance was used as a column. A photo diode array detector (manufactured by Shimadzu Corp., trade name: SPD-M20A) was used as a detector.

### <Raw material>

Compound 1 was synthesized according to a method described in JP-A No. 2010-189630.
Compounds 2, 3 and 4 were synthesized according to a method described in International Publication WO 2013/146806.
Compound 5 was synthesized according to a method described in JP-A No. 2008-106241.
Compound 6 was synthesized according to a method described in International Publication WO 2009/157424.
Compound 7 was synthesized according to a method described in JP-A No. 2011-174062.
Compound 8 was synthesized according to a method described in International Publication WO 2005/049546.
For Compound 9, a commercially available compound was used.
Compound 10 was synthesized according to a method described in JP-A No. 2011-105701.
Compounds 11a and 11b were synthesized according to a method described in JP-A No. 2012-33845.
Compound 12 was synthesized according to a method described in International Publication WO 2013/191088.
Compound 13 was synthesized according to a method described in JP-A No. 2010-215886.
Compound 14 was synthesized according to a method described in International Publication WO 2013/021180.
For Compound (H-21), a product purchased from Luminescence Technology Corp. was used.
Compound (1-A3-6) was synthesized according to a method described in International Publication WO 2008/090795.
Compound (1-A1-12) was synthesized according to a method described in JP-A No. 2013-147551.
Compound COM-1 was synthesized according to a method described in JP-A No. 2013-237789.
Compound COM-2 was synthesized according to a method described in International Publication WO 2002/44189.
Compound COM-4 was synthesized according to a method described in International Publication WO 2009/131255.
Compound COM-8 was synthesized according to a method described in JP-A No. 2011-105701.
Compound COM-9 was prepared in accordance with the following Preparation Example 1.

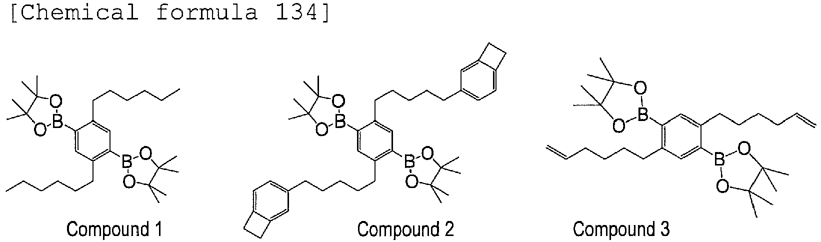

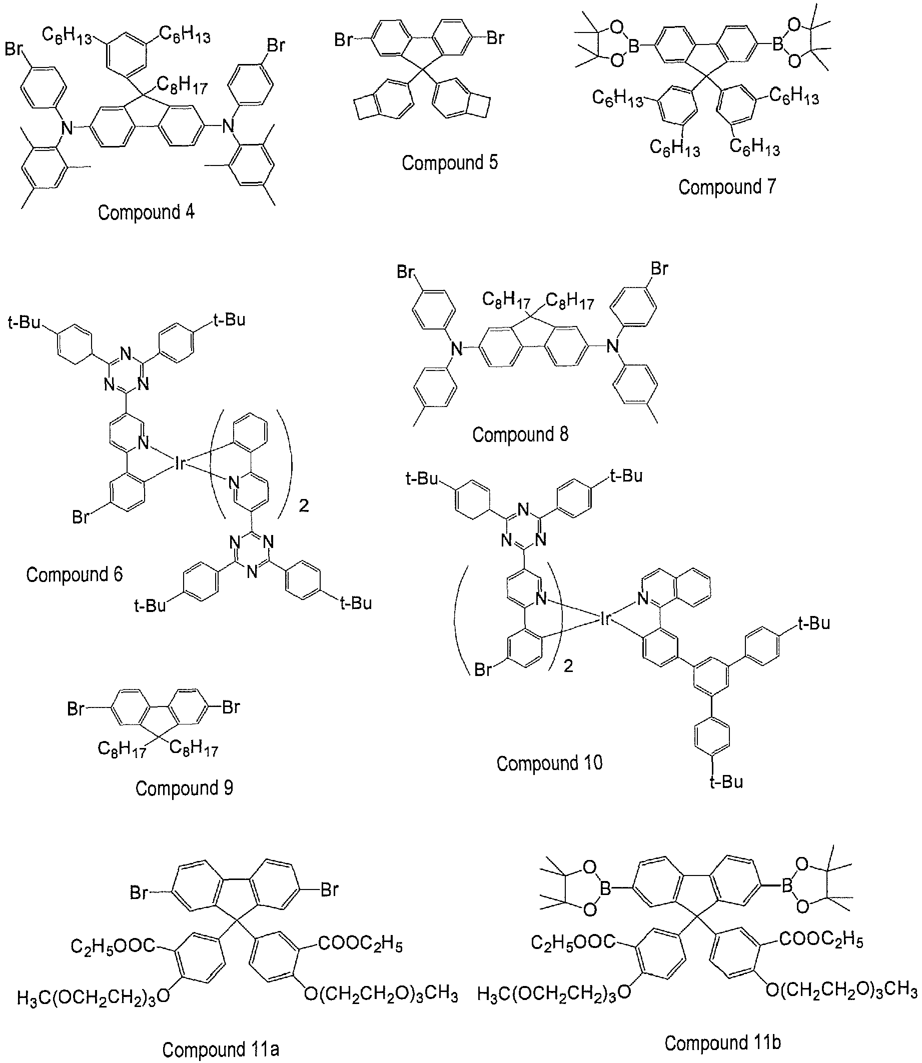

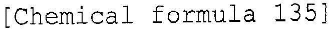

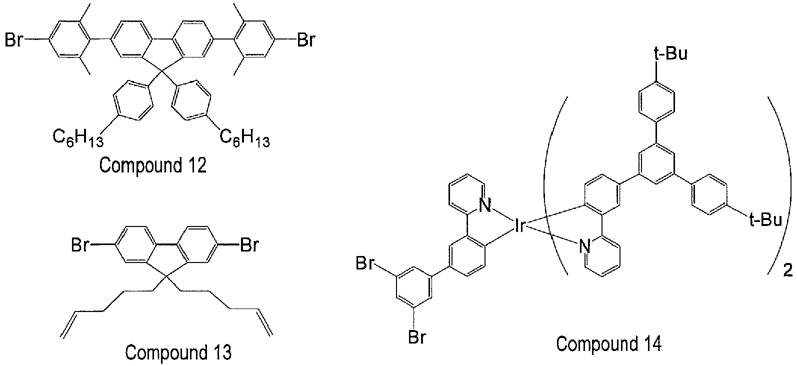

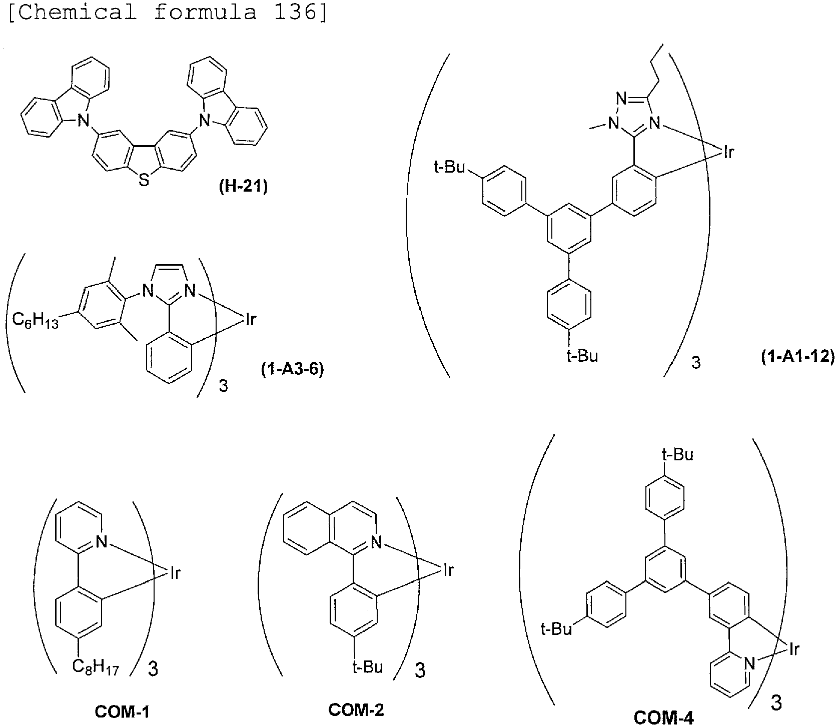

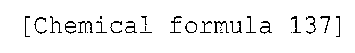

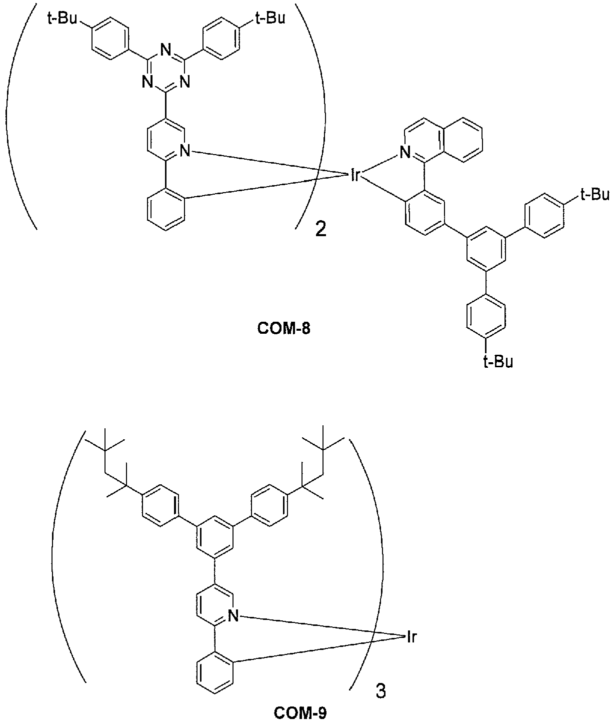

### <Preparation Example 1> Preparation of Compound COM-9

### [Compound S1]

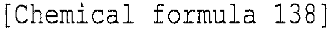

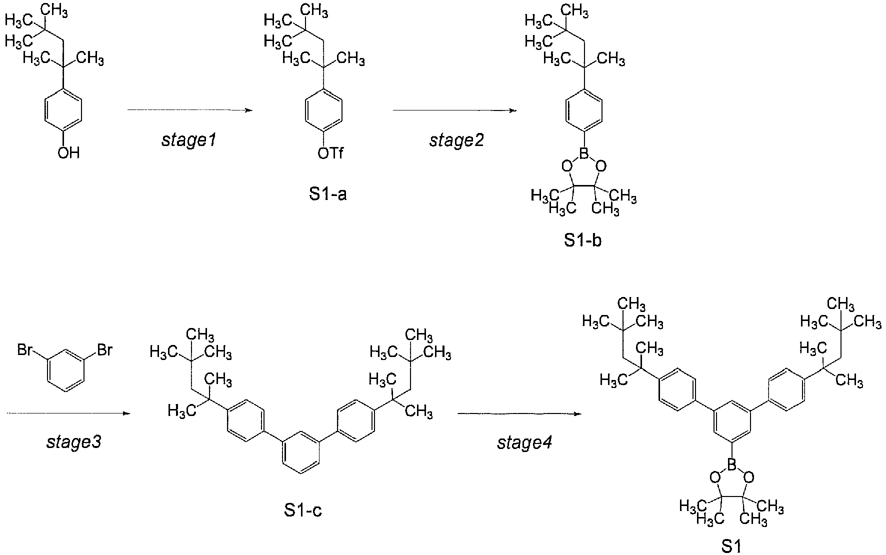

### <Stage 1>

The atmosphere in a reaction vessel was turned into a nitrogen gas stream, then, 4-tert-octylphenol (250.00 g, 1.21 mol, manufactured by Aldrich), N,N-dimethyl-4-aminopyridine (177.64 g, 1.45 mol) and dichloromethane (3100 mL) were added, and this mixture was cooled down to 5°C with ice. Thereafter, trifluoromethanesulfonic anhydride (376.06 g, 1.33 mol) was dropped into this over a period of 45 minutes. After completion of dropping, the mixture was stirred for 30 minutes under ice cool, then, returned to room temperature and further stirred for 15 hours. To the resultant reaction mixture was added hexane (3100 mL), and this reaction mixture was filtrated using 410 g of silica gel, and further, the silica gel was washed with a mixed solvent (2.5 L) of hexane/dichloromethane (1/1 (by volume)). The resultant filtrate and the wash solution were concentrated, to obtain a compound S1-a (410.94 g, LC purity: 99.7%) as a colorless oil.

### <Stage 2>

The atmosphere in a reaction vessel was turned into a nitrogen gas stream, then, the compound S1-a (410.94 g, 1.21 mol), bis(pinacolato)diboron (338.47 g, 1.33 mol), potassium acetate (237.83 g, 2.42 mol), 1,4-dioxane (2600 mL), palladium acetate (4.08 g, 0.018 mol) and tricyclohexylphosphine (10.19 g, 0.036 mol) were added, and the mixture was refluxed for 2 hours. After cooling down to room temperature, the resultant reaction mixture was filtrated and the filtrate was collected, and further, the filtrated substance was washed with 1,4-dioxane (2.5 L), and the resultant filtrate and the wash solution were concentrated. The resultant residue was dissolved into a mixed solvent of hexane/dichloromethane (1/1 (by volume)), and the solution was filtrated using 770 g silica gel, and further, the silica gel was washed with a mixed solvent (2.5 L) of hexane/dichloromethane (1/1 (by volume)). The resultant filtrate and the wash solution were concentrated, and to the resultant residue was added methanol (1500 mL), and the mixture was ultrasonically cleaned for 30 minutes. Thereafter, this was filtrated to obtain a compound S1-b (274.27 g). The filtrate and the wash solution were concentrated, and methanol was added, and the mixture was ultrasonically cleaned and filtrated, and such an operation was repeated, to obtain a compound S1-b (14.29 g). The total yielded amount of the resultant compound S1-b was 288.56 g.

### <Stage 3>

The atmosphere in a reaction vessel was turned into a nitrogen gas stream, then, 1,3-dibromobenzene (102.48 g, 0.434 mol), the compound S1-b (288.56 g, 0.912 mol), toluene (2100 mL), a 20 wt% tetraethyl ammonium hydroxide aqueous solution (962.38 g, 1.31 mol) and bis(triphenylphosphine)palladium(II) dichloride (3.04 g, 0.004 mol) were added, and the mixture was refluxed for 7 hours. After cooling down to room temperature, the aqueous layer and the organic layer were separated, and the organic layer was collected. To this aqueous layer was added toluene (1 L), and the organic layer was further extracted. The resultant organic layers were combined, and this mixture was washed with a mixed aqueous solution of distilled water/saturated saline (1.5 L/1.5 L). The resultant organic layer was filtrated through 400 g silica gel, and further, the silica gel was washed with toluene (2 L). The resultant solution was concentrated, and the resultant residue was dissolved in hexane. This solution was purified by silica gel column chromatography. Impurities were eluted with a developing solvent hexane, then, developed with a mixed solvent of hexane/dichloromethane (10/1 (by volume)). The each resultant fraction was concentrated under reduced pressure to remove the solvent, obtaining a colorless crystalline compound S1-c (154.08 g, LC purity: 99.1%) and a coarse compound S1-c (38.64 g, LC purity: 83%). This coarse compound S1-c was column-purified again under the same developing conditions, and the solvent was distilled off under reduced pressure, to obtain a compound S1-c (28.4 g, LC purity: 99.6%). The total yielded amount the resultant compound S1-c was 182.48 g (0.40 mol).

### <Stage 4>

The atmosphere in a reaction vessel was turned into a nitrogen gas stream, then, the compound S1-c (182, 48 g, 0.401 mol), bis(pinacolato)diboron (112.09 g, 0.441 mol), 4,4'-di-tert-butyl-2,2'-dipyridyl (3.23 g, 0.012 mol), cyclohexane (2000 mL) and bis(1,5-cyclooctadiene)di-µ-methoxydiiridium(I) (3.99 g, 0.006 mol) were added, and the mixture was refluxed for 2 hours. After cooling with air down to room temperature, silica gel (220 g) was added over a period of 20 minutes while stirring the resultant reaction mixture. The resultant suspension was filtrated through 440 g of silica gel, and further, the silica gel was washed with dichloromethane (2 L), and the solution was concentrated. To the resultant residue were added methanol (1100 mL) and dichloromethane (110 mL), and the mixture was refluxed for 1 hour. After cooling down to room temperature, this was filtrated. The resultant filtrated substance was washed with methanol (500 mL), and the resultant solid was dried, to obtain a compound S1 (220.85 g, 0.380 mol).
¹H-NMR (CDCl₃, 300 MHz): δ (ppm) = 8.00 (d, J=1.8Hz, 2H), 7.92 (t,J=1.9Hz, 1H), 7.60 (d, J = 8.5Hz, 4H), 7.44 (t, J = 8.5Hz, 4H), 1.78 (s, 4H), 1.41 (s, 12H), 1.37 (s, 12H), 0.75 (s, 18H).

### [Compound COM-9]

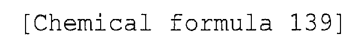

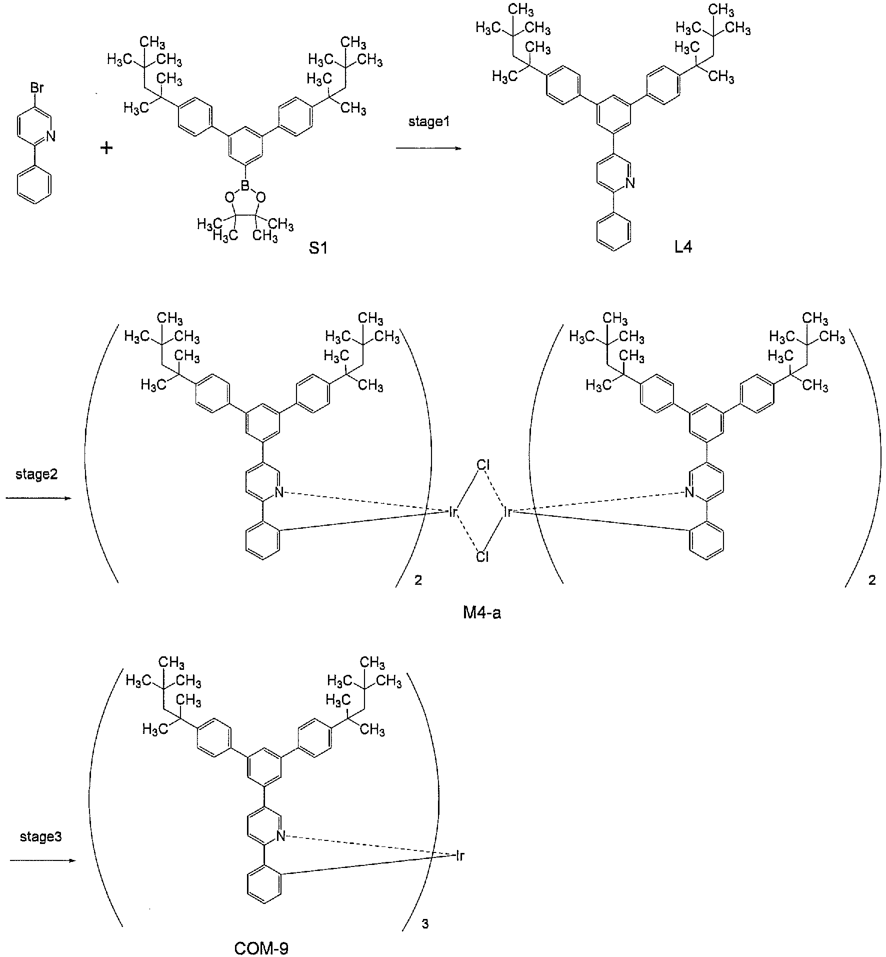

### <Stage 1>

5-bromo-2-phenylpyridine was synthesized according to a method described in JP-A No. 2008-179617.

The atmosphere in a reaction vessel was turned into an argon gas atmosphere, then, 5-bromo-2-phenylpyridine (36.17 g, 155 mmol), the compound S1 (94.20 g, 162 mmol), toluene (1545 mL), a 20 wt% tetraethyl ammonium hydroxide aqueous solution (341.28 g, 463.5 mmol) and tetrakis(triphenylphosphine)palladium(0) (8.927 g, 7.725 mmol) were added, and the mixture was stirred for 4 hours at 80°C. After cooling down to room temperature, to the resultant reaction solution was added water (1545 mL), and the organic layer was extracted. The resultant organic layer was washed with water (1545 mL) twice, and with saline (1545 mL) once. The resultant organic layer was filtrated using 188 g of silica gel. The resultant filtrate was concentrated under reduced pressure. To the resultant residue were added toluene (235 g) and methanol (1174 g), and the mixture was heated at 60°C for 30 minutes. Thereafter, this was cooled down to 5°C by an ice bath, to cause deposition of a solid. The resultant solid was filtrated, and washed with cold methanol. The resultant solid was dried under reduced pressure, to obtain a compound L4 (82.0 g, 135 mmol) represented by the above-described formula.

### <Stage 2>

The atmosphere in a reaction vessel was turned into a nitrogen gas atmosphere, then, iridium chloride trihydrate (11.51 g, 32.3 mmol) and ion-exchange water (114 mL) were added, and these were dissolved by heating at 50°C. Into another reaction vessel having a nitrogen gas atmosphere were added the compound L4 (43.80 g, 72.1 mmol), 2-ethoxyethanol (792 mL) and ion-exchange water (57 mL), and the mixture was stirred for 1 hour with heating at 100°C. Thereafter, into this solution, an iridium chloride aqueous solution (total amount) prepared previously was dropped slowly. After completion of dropping, the mixture was stirred for 15 hours at 120°C. After cooling down to room temperature, to the resultant reaction mixture was added methanol (207 g), and the mixture was filtrated. The resultant solid was washed with methanol (207 g) four times, and with hexane (115 g) once. The resultant solid was dried under reduced pressure, to obtain a metal complex M4-a (42.88 g).

### <Stage 3>

The atmosphere in a reaction vessel was turned into a nitrogen gas atmosphere, then, the metal complex M4-a (7.61 g, 2.64 mmol), the compound L4 (16.05 g, 26.40 mmol), silver trifluoromethanesulfonate (1.63 g, 6.34 mmol) and diethylene glycol dimethyl ether (79 mL) were added, and the mixture was stirred for 16 hours at 160°C. After cooling down to room temperature, to the resultant reaction mixture was added methanol (304 mL), and the generated precipitate was filtrated. The resultant precipitate was purified by silica gel column chromatography (a mixed solvent of hexane/toluene = 4/6.5 (by volume)), and the solvent was removed under reduced pressure. The resultant residue (8.05 g) was dissolved in dichloromethane (80 mL), and to this solution was added methanol (80 mL). The generated precipitate was collected by filtration, and this was dried under reduced pressure, to obtain a compound COM-9 which is a metal complex (6.25 g, 3.1 mmol).
¹H-NMR (CD₂Cl₂, 300 MHz): δ (ppm) = 8.09 (t, J=1.4Hz, 3H), 8.01 (d, J=1.2Hz, 6H), 7.84 (t, J=1.4Hz, 6H), 7.72 (dd, J=7.4Hz and 1.4 Hz, 3H), 7.57 (t, J=1.4Hz, 3H), 7.42 (d, J=8.5Hz, 12H), 7.19 (d, J=8.5Hz, 12H), 7.03 (dd, J=7.2Hz and 1.5Hz, 3H), 6.96-6.86 (mult, 6H), 1.65 (s, 12H), 1.24 (s, 36H), 0.63 (s, 54H).

### <Example 1> Synthesis of polymer compound 1

### (Step 1)

The atmosphere in a reaction vessel was turned into an inert gas atmosphere, then, Compound 1 (0.673 g), Compound 2 (0.304 g), Compound 3 (0.222 g), Compound 4 (1.95 g), Compound 5 (0.238 g), Compound 6 (0.0953 g), dichlorobis(tris-o-methoxyphenylphosphine)palladium (2.0 mg), 20 wt% tetraethyl ammonium hydroxide aqueous solution (7.5 mL) and toluene (50 mL) were added thereto, and the mixture was stirred for 4 hours under reflux.

### (Step 2)

After the reaction, phenyl boronic acid (27 mg), dichlorobis(tris-o-methoxyphenylphosphine)palladium (2.0 mg) and 20 wt% tetraethyl ammonium hydroxide aqueous solution (7.5 mL) were added thereto and the mixture was further stirred for 17 hours under reflex.

### (Step 3)

Thereafter, an aqueous solution prepared by dissolving N,N-sodium diethyldithiocarbamate (1.25 g) in ion-exchange water (25 ml) was added thereto, and the mixture was stirred for 2 hours at 85°C. The reaction liquid was cooled to room temperature, an aqueous layer was removed, and then the resultant organic layer was washed with ion-exchange water. When the resultant organic layer was dropped into methanol, a precipitate was generated. The resultant precipitate was filtrated and dried to obtain a solid. This solid was dissolved in toluene, and purified by passing through a Celite Column. The resultant solution was dropped into methanol, the mixture was stirred, and the resultant precipitate was filtrated and dried to obtain a Polymer compound 1 (2.3 g). Mn of the Polymer compound 1 was 4.0×10⁴, and Mw was 2.0×10⁵.

With reference to a theoretical value calculated from the amount of charged raw materials, Polymer compound 1 is a copolymer comprising a constitutional unit derived from Compound 1, a constitutional unit derived from Compound 2, a constitutional unit derived from Compound 3, a constitutional unit derived from Compound 4, a constitutional unit derived from Compound 5, and a constitutional unit derived from Compound 6 with a molar ratio of 30:10:10:39.4:10:1.2.

### <Synthesis Example 1> Synthesis of Polymer compound 2

### (Step 1)

The atmosphere in a reaction vessel was turned into an inert gas atmosphere, then, Compound 7 (2.5625 mmol), Compound 8 (1.5000 mmol), Compound 9 (0.4750 mmol), Compound 5 (0.3750 mmol), Compound 10 (0.1500 mmol), dichlorobis(tris-o-methoxyphenylphosphine)palladium (4.5 mg) and toluene (83 mL) were added thereto and heated to 100°C.

### (Step 2)

After the reaction, 20 wt% tetraethyl ammonium hydroxide aqueous solution (8.5 mL) was dropped thereinto and refluxed for 9.5 hours.

### (Step 3)

After the reaction, phenylboronic acid (61 mg) and dichlorobis(tris-o-methoxyphenylphosphine)palladium (2.2 mg) were added thereto and refluxed for 19 hours.

### (Step 4)

Thereafter, a sodium diethyldithiocarbamate aqueous solution (10 mL, concentration: 0.05 g/mL) was added thereto and the mixture was stirred for 2 hours at 85°C. After cooling, the reaction liquid was washed with a 3.6 wt% hydrochloric acid, a 2.5 wt% ammonia water and water, the resultant solution was dropped into methanol, and thus a precipitate was generated. The precipitate was dissolved in toluene, and then purified by passing through an alumina column and a silica gel column in this order. The resultant solution was dropped into methanol, and the mixture was stirred and then the resultant precipitate was filtrated and dried to obtain 3.152 g of Polymer compound 2. Mn of the Polymer compound 2 was 4.4×10⁴, and Mw was 1.5×10⁵.

With reference to a theoretical value calculated from the amount of charged raw materials, Polymer compound 2 is a copolymer comprising a constitutional unit derived from Compound 7, a constitutional unit derived from Compound 8, a constitutional unit derived from Compound 9, a constitutional unit derived from Compound 5, and a constitutional unit derived from Compound 10 with a molar ratio of 50:30:9.5:7.5:3.

### <Synthesis Example 2> Synthesis of Polymer compound 3

The Polymer compound 3 was synthesized by using Compound 7, Compound 8, Compound 9 and Compound 5 according to a method described in JP-A 2012-144722.

Mn of the Polymer compound 3 was 8.0×10⁴, and Mw was 2.6×10⁵.

With reference to a theoretical value calculated from the amount of charged raw materials, Polymer compound 3 is a copolymer comprising a constitutional unit derived from Compound 7, a constitutional unit derived from Compound 8, a constitutional unit derived from Compound 9, and a constitutional unit derived from Compound 5, with a molar ratio of 50:30:12.5:7.5.

### <Synthesis Example 3> Synthesis of Polymer compound 4

### (Step 1)

The atmosphere in a reaction vessel was turned into an inert gas atmosphere, then, Compound 11a (0.55 g), Compound 11b (0.61 g), triphenylphosphine palladium (0.01 g), methyltrioctylammonium chloride (manufactured by Aldrich, product name: Aliquat336 (registered trademark))(0.20 g), and toluene (10 mL) were mixed and heated to 105°C.

### (Step 2)

Into the reaction liquid, a 2M sodium carbonate aqueous solution (6 mL) was dropped, and the mixture was refluxed for 8 hours.

### (Step 3)

Into the reaction liquid, 4-tert-butylbenzeneboronic acid (0.01 g) was dropped, and the mixture was refluxed for 6 hours.

### (Step 4)

Next, a sodium diethyldithiocarbamate aqueous solution (10 mL, concentration: 0.05 g/mL) was added, and the mixture was stirred for 2 hours. The mixed solution was dropped into 300 mL of methanol and the mixture was stirred for 1 hour, then the resultant deposited precipitate was filtrated and dried under a reduced pressure for 2 hours and dissolved in 20 ml of tetrahydrofuran. The resultant solution was dropped into a mixed solvent of 120 ml of methanol and 50 ml of 3 wt% acetic acid aqueous solution, and the mixture was stirred for 1 hour. After that, the deposited precipitate was filtrated, and dissolved in 20 ml of tetrahydrofuran.

### (Step 5)

The resultant solution was dropped into 200 ml of methanol and the mixture was stirred for 30 minutes, and then a deposited precipitate was filtrated to obtain a solid. The resultant solid was dissolved in tetrahydrofuran and purified by passing through an alumina column and silica gel column in this order. The resultant solution was dropped into methanol, the mixture was stirred and then the resultant precipitate was filtrated and dried to obtain 520 mg of Polymer compound 4. Mn of the Polymer compound 4 was 5.2×10⁴.

### <Synthesis Example 4> Synthesis of Polymer compound 5

A 100 mL flask was charged with Polymer compound 4 (200 mg), and then the atmosphere within the flask was replaced with a nitrogen gas. Tetrahydrofuran (20 mL) and ethanol (20 mL) were added into the flask, and the mixture was heated to 55°C. Thereto was added an aqueous solution prepared by dissolving cesium hydroxide (200 mg) in water (2 mL), and the mixture was stirred at 55°C for 6 hours. The mixture was cooled to room temperature, and thereafter the reaction solvent was distilled off under reduced pressure. The resultant solid was washed with water and dried under reduced pressure so as to obtain Polymer compound 5 (150 mg) as a pale yellow solid. It was confirmed from the NMR spectrum that the signal derived from an ethyl group at the ethyl ester site of in Polymer compound 4 disappeared completely.

The structure of Polymer compound 5 is shown in the following formula. Wherein, n^{p5} denotes a polymerization degree.

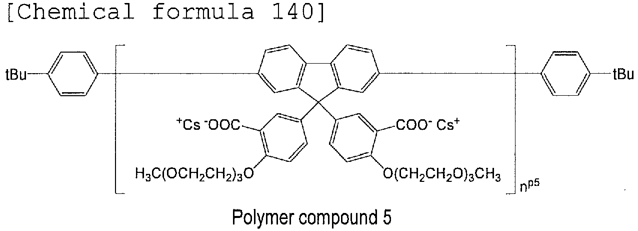

### <Synthesis Example 5> Synthesis of Polymer compound 6

### (Step 1)

The atmosphere in a reaction vessel was turned into an inert gas atmosphere, then, Compound 1 (0.85675 g), Compound 12 (0.86638 g), Compound 5 (0.09367 g), Compound 13 (0.08090 g), Compound 14 (0.56072 g), dichlorobis(tris-o-methoxyphenylphosphine)palladium (1.54 mg) and toluene (36 mL) were added thereto, and the mixture was heated to 105°C.

### (Step 2)

Into the reaction liquid, 20 wt% tetraethyl ammonium hydroxide aqueous solution (5.9 mL) was dropped, and the mixture was refluxed for 6 hours.

### (Step 3)

After the reaction, phenylboronic acid (85.4 mg) and dichlorobis(tris-o-methoxyphenylphosphine)palladium (1.54 mg) were added thereto, and the mixture was refluxed for 14.5 hours.

### (Step 4)

Thereafter, a sodium diethyldithiocarbamate aqueous solution (10 mL, concentration: 0.05 g/mL) was added thereto and the mixture was stirred for 2 hours at 80°C. After cooling, the resultant reaction liquid was washed twice with water, twice with 3 wt% acetic acid aqueous solution, and twice with water. When the resultant solution was dropped into methanol, a precipitate was generated. The resultant precipitate was dissolved in toluene, and the solution was purified by passing through an alumina column and a silica gel column in this order. The resultant solution was dropped into methanol and stirred, and thereafter, the resultant precipitate was filtrated and dried to obtain 1.33 g of Polymer compound 6. The polystyrene-equivalent number average molecular weight of Polymer compound 6 was 7.0×10⁴, and the polystyrene-equivalent weight average molecular weight was 1.8×10⁵.

With reference to a theoretical value calculated from the amount of charged raw materials, Polymer compound 6 is a copolymer comprising a constitutional unit derived from Compound 1, a constitutional unit derived from Compound 12, a constitutional unit derived from Compound 5, a constitutional unit derived from Compound 13, and a constitutional unit derived from Compound 14 with a molar ratio of 50:30:5:5:10.

### <Evaluation of phosphorescent spectra of Polymer compounds 1, 2 and 6>

### <Evaluation Example E1> Fabrication and evaluation of light emitting device E1

An anode was formed by providing an ITO film having a thickness of 45 nm by sputtering method on a glass substrate. On the anode, AQ-1200 (manufactured by Plectronics Inc.) as a hole injection agent based on polythiophene·sulfonic acid was applied by spin-coating method to form a film having a thickness of 35 nm, and then it was heated on a hot plate for 15 minutes at 170°C in an ambient atmosphere, thereby forming a hole injection layer.

Polymer compound 1 was dissolved in xylene at a concentration of 1.2 wt%. The resultant xylene solution was used to form a film having a thickness of 70 nm by spin-coating method on the hole injection layer, and then it was heated on a hot plate for 60 minutes at 180°C in a nitrogen gas atmosphere, thereby forming a light-emitting layer.

On the light-emitting layer, sodium fluoride as a cathode was evaporated to have a thickness of about 7 nm and then aluminum was evaporated to have a thickness of about 120 nm, whereby a light emitting device E1 was fabricated. Here, evaporation of the metal started after the degree of vacuum reached 1×10⁻⁴ Pa or less.

When a voltage of 15 V was applied to the light emitting device E1, EL emission (emission peak wavelength: 600 nm) derived from the constitutional unit obtained from Compound 6 was obtained.

### <Evaluation example E2> Fabrication and evaluation of light emitting device E2

A light emitting device E2 was fabricated in the same manner as Evaluation Example E1 except that Polymer compound 2 was used in place of Polymer compound 1.

When a voltage of 15 V was applied to the light emitting device E2, EL emission (emission peak wavelength: 615 nm) derived from the constitutional unit obtained from Compound 10 was obtained.

### <Evaluation Example E3> Fabrication and evaluation of light emitting device E3

An anode was formed by providing an ITO film having a thickness of 45 nm by sputtering method on a glass substrate. On the anode, AQ-1200 (manufactured by Plectronics Inc.) as a hole injection agent based on polythiophene·sulfonic acid was applied by spin-coating method to form a film having a thickness of 35 nm, and then it was heated on a hot plate for 15 minutes at 170°C in an ambient atmosphere, thereby forming a hole injection layer.

Polymer compound 6 was dissolved in xylene at a concentration of 1.7 wt%. The resultant xylene solution was used to form a film having a thickness of 65 nm by spin-coating method on the hole injection layer, and then it was heated on a hot plate for 60 minutes at 180°C in a nitrogen gas atmosphere, thereby forming a light-emitting layer.

On the light-emitting layer, sodium fluoride as a cathode was evaporated to have a thickness of about 7 nm and then aluminum was evaporated to have a thickness of about 120 nm, whereby a light emitting device E3 was fabricated. Here, evaporation of the metal started after the degree of vacuum reached 1×10⁻⁴ Pa or less.

When a voltage of 15 V was applied to the light emitting device E3, EL emission (emission peak wavelength: 515 nm) derived from the constitutional unit obtained from Compound 14 was obtained.

### <Evaluation of emission spectrum maximum peak wavelength of phosphorescent compound>

The emission spectrum maximum peak wavelengths of the phosphorescent Compounds, i.e., Compound (1-A3-6), Compound (1-A1-12), Compound COM-1, Compound COM-4, Compound COM-9, Compound COM-2 and Compound COM-8 were measured at room temperature with a spectrophotometer (FP6500 manufactured by JASCO Corporation). The sample in use was a xylene solution prepared by dissolving a phosphorescent compound in xylene at a concentration of about 0.8×10⁻⁴ wt%. For the excitation light, UV light having a wavelength of 325 nm was used. The evaluation results are shown in Table 2.

**(Table 2)**

| Phosphorescent compound | Emission peak wavelength (nm) |
|---|---|
| Compound (1-A3-6) | 470 |
| Compound (1-A1-12) | 475 |
| Compound COM-1 | 508 |
| Compound COM-4 | 513 |
| Compound COM-9 | 545 |
| Compound COM-2 | 620 |
| Compound COM-8 | 611 |

### <Example 2> Fabrication and evaluation of light emitting device D1

An anode was formed by providing an ITO film having a thickness of 45 nm by sputtering method on a glass substrate. On the anode, AQ-1200 (manufactured by Plectronics Inc.) as a hole injection agent based on polythiophene·sulfonic acid was applied by spin-coating method to form a film having a thickness of 35 nm, and then it was heated on a hot plate for 15 minutes at 170°C in an ambient atmosphere, thereby forming a hole injection layer.

Polymer compound 1 was dissolved in xylene at a concentration of 0.6 wt%. The resultant xylene solution was used to form a film having a thickness of 20 nm by spin-coating on the hole injection layer, and then it was heated on a hot plate for 60 minutes at 180°C in a nitrogen gas atmosphere, thereby forming a first light-emitting layer.

An ink D1 was prepared by dissolving Compound (H-21), Compound (1-A3-6), Compound COM-1 (Compound (H-21)/Compound (1-A3-6)/Compound COM-1 (weight ratio)=69.77/30/0.23) in toluene at a concentration of 2.0 wt%. The ink D1 was applied on the first light-emitting layer by spin-coating method to form a film having a thickness of 75 nm, and then it was heated on a hot plate for 10 minutes at 130°C in a nitrogen gas atmosphere, thereby forming a second light-emitting layer.

An ink P1 was prepared by dissolving Polymer compound 5 in 2,2,3,3,4,4,5,5-octafluoro-1-pentanol at a concentration of 0.25 wt%. The ink P1 was applied on the second light-emitting layer by spin-coating method to form a film having a thickness of 10 nm, and then it was heated on a hot plate for 10 minutes at 130°C in a nitrogen gas atmosphere, thereby forming an electron transport layer. On the electron transport layer, sodium fluoride as a cathode was evaporated to have a thickness of about 7 nm and then aluminum was evaporated to have a thickness of about 120 nm, whereby a light emitting device D1 was fabricated. Here, evaporation of the metal started after the degree of vacuum reached 1×10⁻⁴ Pa or less.

When a voltage was applied to the light emitting device D1, EL emission was observed. Evaluation results at the emission luminance of 1000 cd/m² are shown in Table 3.

### <Example 3 and Comparative Example 1> Fabrication and evaluation of light emitting device D2 and light emitting device CD1

A light emitting device D2 and a light emitting device CD1 were fabricated in the same manner as Example 2 except that 1) a first light-emitting layer (hole transport layer in Comparative Example 1) was formed by using, not the Polymer compound 1 but the polymer compounds shown in Table 3, and 2) a second light-emitting layer (light-emitting layer in Comparative Example 1) was formed by using, not the ink D1 but an ink whose constitutional materials and constitutional ratio were modified as shown in Table 3. The evaluation results for the light emitting device D2 and the light emitting device CD1 at the emission luminance of 1000 cd/m² also are shown in Table 3.

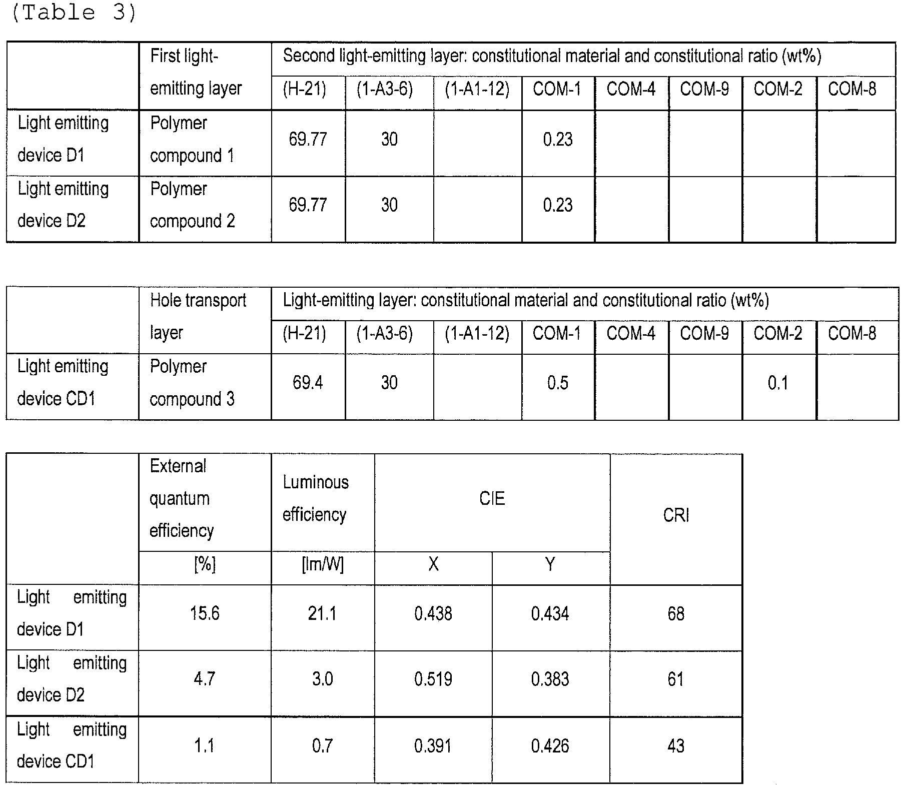

### <Examples 4 and 5, and, Comparative Example 2> Fabrication and evaluation of light emitting devices D3 and D4, and, light emitting device CD2

Light emitting devices D3 and D4 and a light emitting device CD2 were fabricated in the same manner as Example 2 except that 1) first light-emitting layers (hole transport layer in Comparative Example 2) were formed by using the polymer compounds shown in Table 4, and 2) second light-emitting layers (light-emitting layer in Comparative Example 2) were formed by using, not the ink D1 but inks whose constitutional materials and constitutional ratios were modified as shown in Table 4. The evaluation results for the light emitting devices D3 and D4 and the light emitting device CD2 at the emission luminance of 1000 cd/m² also are shown in Table 4.

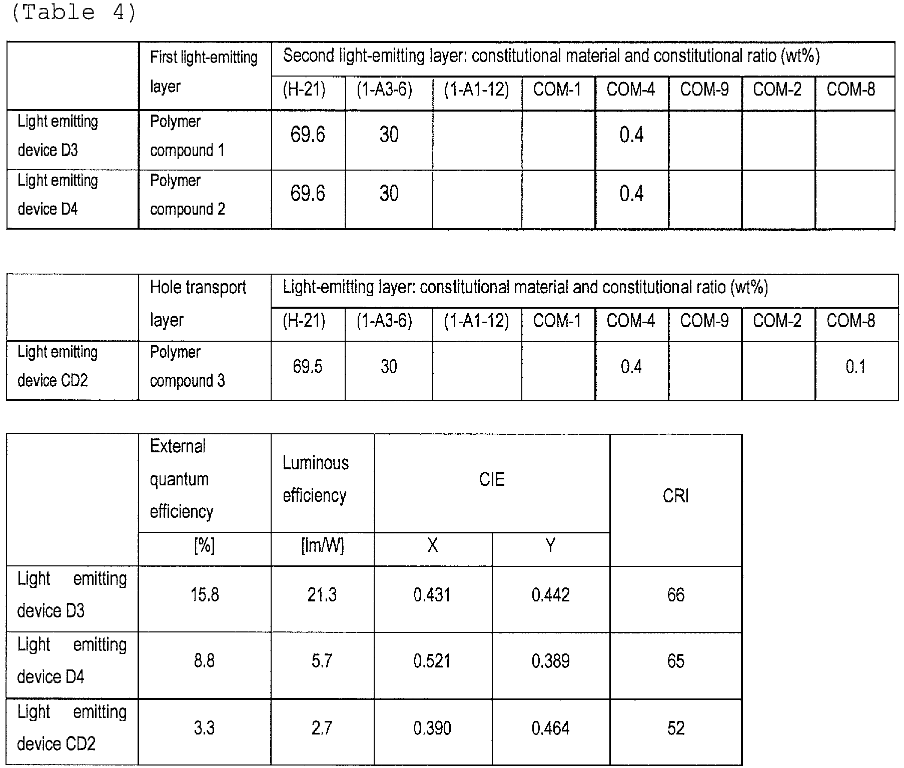

### <Example 6> Fabrication and evaluation of light emitting device D5

A light emitting device D5 was fabricated in the same manner as Example 2 except that the second light-emitting layer was formed by using, not the ink D1 but an ink whose constitutional material and constitutional ratio were modified as shown in Table 5. The evaluation results for the light emitting device D5 at the emission luminance of 1000 cd/m² also are shown in Table 5.

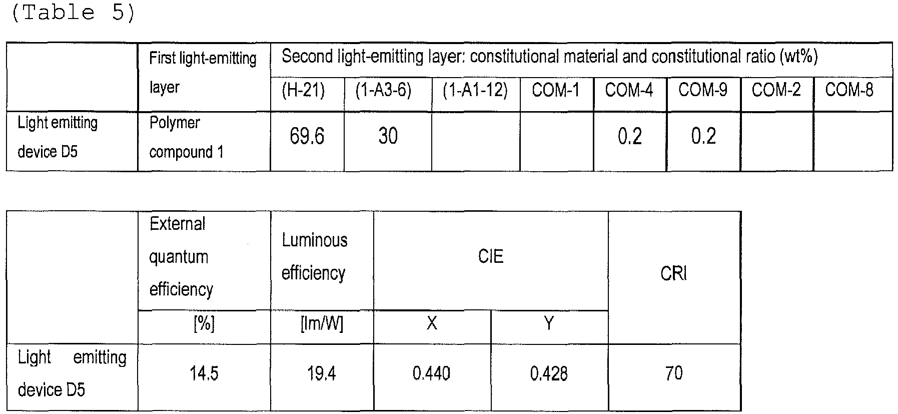

### <Examples 7 and 8, and, Comparative Example 3> Fabrication and evaluation of light emitting devices D6 and D7, and, light emitting device CD3

Light emitting devices D6 and D7 and a light emitting device CD3 were fabricated in the same manner as Example 2 except that 1) first light-emitting layers (hole transport layer in Comparative Example 3) were formed by using the polymer compounds shown in Table 6, and 2) second light-emitting layers (light-emitting layer in Comparative Example 3) were formed by using, not the ink D1 but inks whose constitutional materials and constitutional ratios were modified as shown in Table 6. The evaluation results for the light emitting devices D6 and D7 and the light emitting device CD3 at the emission luminance of 1000 cd/m² also are shown in Table 6.

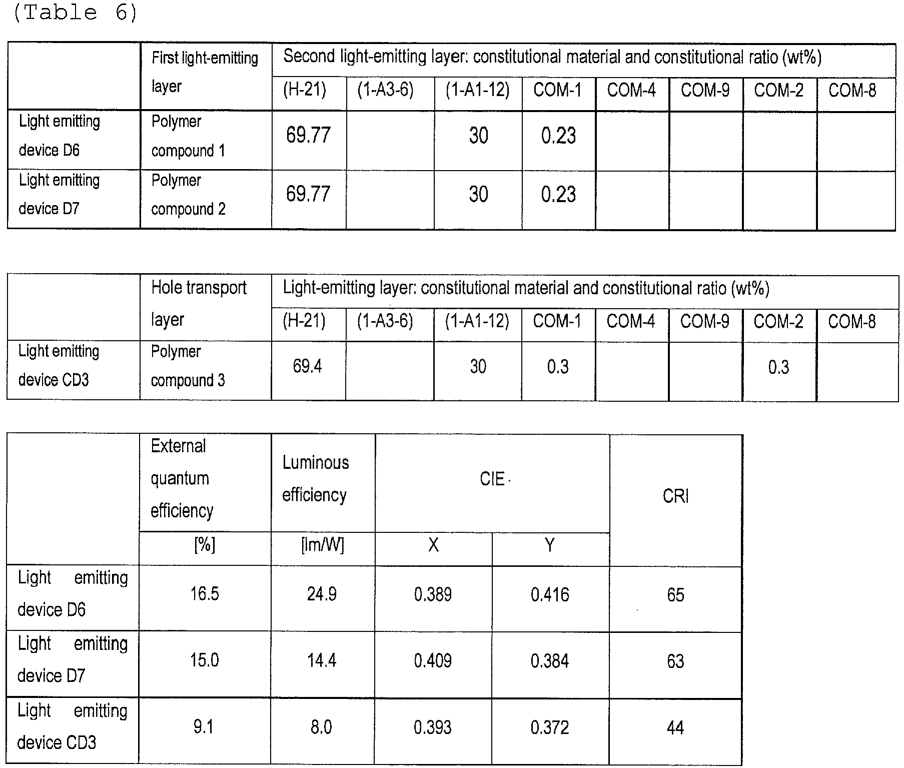

### <Examples 9 and 10, and, Comparative Example 4> Fabrication and evaluation of light emitting devices D8 and D9, and, light emitting device CD4

Light emitting devices D8 and D9 and a light emitting device CD4 were fabricated in the same manner as Example 2 except that 1) first light-emitting layers (hole transport layer in Comparative Example 4) were formed by using the polymer compounds shown in Table 7, and 2) second light-emitting layers (light-emitting layer in Comparative Example 4) were formed by using, not the ink D1 but inks whose constitutional materials and constitutional ratios were modified as shown in Table 7. The evaluation results for the light emitting devices D8 and D9 and the light emitting device CD4 at the emission luminance of 1000 cd/m² also are shown in Table 7.

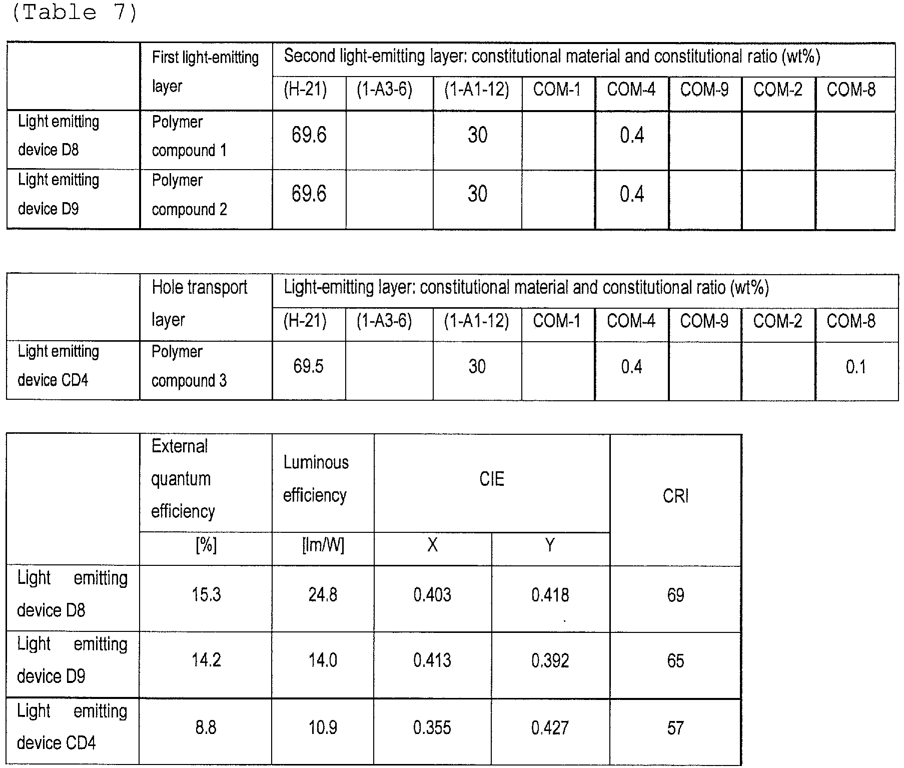

### <Example 11> Fabrication and evaluation of light emitting device D10

An anode was formed by providing an ITO film having a thickness of 45 nm by sputtering method on a glass substrate. On the anode, AQ-1200 (manufactured by Plectronics Inc.) as a hole injection agent based on polythiophene·sulfonic acid was applied by spin-coating method to form a film having a thickness of 35 nm, and then it was heated on a hot plate for 15 minutes at 170°C in an ambient atmosphere, thereby forming a hole injection layer.

Polymer compound 3 was dissolved in xylene at a concentration of 0.6 wt%. The resultant xylene solution was used to form a film having a thickness of 20 nm by spin-coating method on the hole injection layer, and then it was heated on a hot plate for 60 minutes at 180°C in a nitrogen gas atmosphere, thereby forming a hole transport layer.

Polymer compound 6 was dissolved in xylene at a concentration of 0.6 wt%. The resultant xylene solution was used to form a film having a thickness of 20 nm by spin-coating metod on the hole transport layer, and then it was heated on a hot plate for 60 minutes at 180°C in a nitrogen gas atmosphere, thereby forming a first light-emitting layer.

An ink D10 was prepared by dissolving Compound (H-21), Compound (1-A1-12) and Compound COM-8 (Compound (H-21)/Compound (1-A1-12)/Compound COM-8 (weight ratio)=69.9/30/0.1) in toluene at a concentration of 2.0 wt%. The ink D10 was applied on the first light-emitting layer by spin-coating metod to form a film having a thickness of 60 nm, and then it was heated on a hot plate for 10 minutes at 130°C in a nitrogen gas atmosphere, thereby forming a second light-emitting layer.

An ink P1 was prepared by dissolving Polymer compound 5 in 2,2,3,3,4,4,5,5-octafluoro-1-pentanol at a concentration of 0.25 wt%. The ink P1 was applied on the second light-emitting layer by spin-coating method to form a film having a thickness of 10 nm, and then it was heated on a hot plate for 10 minutes at 130°C in a nitrogen gas atmosphere, thereby forming an electron transport layer. On the electron transport layer, sodium fluoride as a cathode was evaporated to have a thickness of about 7 nm and then aluminum was evaporated to have a thickness of about 120 nm, whereby a light emitting device D10 was fabricated. Here, evaporation of the metal started after the degree of vacuum reached 1×10⁻⁴ Pa or less.

When a voltage was applied to the light emitting device D10, EL emission as observed. Evaluation results at the emission luminance of 1000 cd/m² are shown in Table 8.

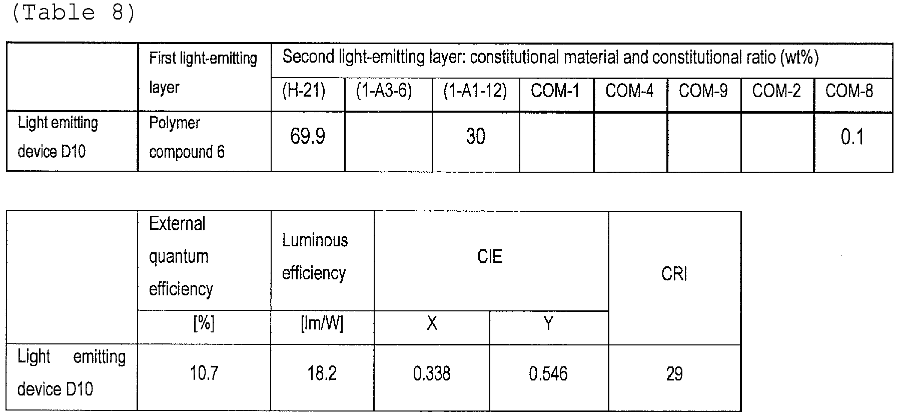

The external quantum efficiency of the light emitting device D10 is superior to the external quantum efficiency of the light emitting device CD4.

## Claims

1. A light emitting device comprising:
an anode;
a cathode;
a first light-emitting layer provided between the anode and the cathode; and
a second light-emitting layer provided between the anode and the cathode, wherein
the first light-emitting layer is a layer obtained by using a polymer compound comprising a constitutional unit having a cross-linkable group and a phosphorescent constitutional unit, and
the second light-emitting layer is a layer obtained by using a composition comprising a non-phosphorescent low molecular weight compound having a heterocyclic structure and at least two phosphorescent compounds.

2. The light emitting device according to claim 1, wherein the first light-emitting layer is provided between the anode and the second light-emitting layer.

3. The light emitting device according to claim 1 or 2, wherein the non-phosphorescent low molecular weight compound having a heterocyclic structure is a compound represented by formula (H-1): 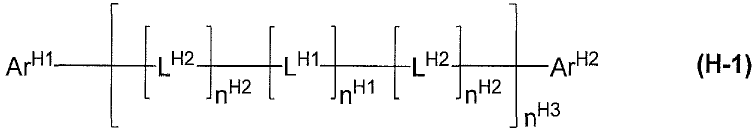 wherein,
Ar^{H1} and Ar^{H2} represent an aryl group or a monovalent heterocyclic group, and these groups optionally have a substituent,
n^{H1} and n^{H2} each independently represent 0 or 1; when a plurality of n^{H1} are present, they may be the same or different; the plurality of n^{H2} may be the same or different,
n^{H3} represents an integer of 1 or more,
L^{H1} represents an arylene group or a divalent heterocyclic group, and these groups optionally have a substituent; when a plurality of L^{H1} are present, they may be the same or different,
L^{H2} represents a group represented by -N(-L^{H3}-R^{HA}) - or a group represented by -[C(R^{HB})₂]n^{H4}-; R^{HA} represents an alkyl group, a cycloalkyl group, an aryl group or a monovalent heterocyclic group, and these groups optionally have a substituent; L^{H3} represents a single bond, an arylene group or a divalent heterocyclic group, and these groups optionally have a substituent; R^{HB} represents a hydrogen atom, an alkyl group, a cycloalkyl group, an alkoxy group, a cycloalkoxy group, an aryl group or a monovalent heterocyclic group, and these groups optionally have a substituent; the plurality of R^{HB} may be the same or different, and they may be combined together to form a ring together with the carbon atoms to which they are attached; n^{H4} represents an integer of 1 to 10; when a plurality of L^{H2} are present, they may be the same or different, and
at least one of Ar^{H1}, Ar^{H2}, L^{H1} and L^{H2} is a monovalent or divalent heterocyclic group or a group comprising a monovalent or divalent heterocyclic group.

4. The light emitting device according to any one of claims 1 to 3, wherein
the at least two phosphorescent compounds comprise at least one phosphorescent compound having an emission spectrum the maximum peak wavelength of which is between 400 nm or more and less than 480 nm (B), and at least one phosphorescent compound having an emission spectrum the maximum peak wavelength of which is between 480 nm or more and less than 680 nm (G).

5. The light emitting device according to claim 4, wherein the phosphorescent compound (B) is a phosphorescent compound represented by formula (1): 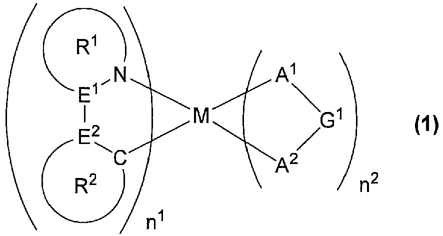 wherein,
M represents a ruthenium atom, a rhodium atom, a palladium atom, an iridium atom or a platinum atom,
n¹ represents an integer of 1 or more, n² represents an integer of 0 or more, and n¹+n² is 2 or 3; when M is a ruthenium atom, a rhodium atom or an iridium atom, n¹+n² is 3; when M is a palladium atom or a platinum atom, n¹+n² is 2,
E¹ and E² each independently represent a carbon atom or a nitrogen atom, and at least either E¹ or E² is a carbon atom,
the ring R¹ represents a five-membered or six-membered aromatic heterocyclic ring, and these rings optionally have a substituent; when a plurality of the substituents are present, they may be the same or different, and they may be combined together to form a ring together with the atoms to which they are attached; when a plurality of rings R¹ are present, they may be the same or different; E¹ is a carbon atom when the ring R¹ is a six-membered aromatic heterocyclic ring,
the ring R² represents a five-membered or six-membered aromatic carbon ring, or a five-membered or six-membered aromatic heterocyclic ring, and these rings optionally have a substituent; when a plurality of the substituents are present, they may be the same or different, and they may be combined together to form a ring together with the atoms to which they are attached; when a plurality of rings R² are present, they may be the same or different; E² is a carbon atom when the ring R² is a six-membered aromatic heterocyclic ring,
the ring R² has an electron-withdrawing group when the ring R¹ is a six-membered aromatic heterocyclic ring, and
A¹-G¹-A² represents an anionic bidentate ligand; A¹ and A² each independently represent a carbon atom, an oxygen atom or a nitrogen atom, and these atoms may be atoms constituting a ring; G¹ represents a single bond or an atomic group constituting the bidentate ligand together with A¹ and A²; when a plurality of A¹-G¹-A² are present, they may be the same or different.

6. The light emitting device according to claim 5, wherein the phosphorescent compound represented by formula (1) is a phosphorescent compound represented by formula (1-A) : 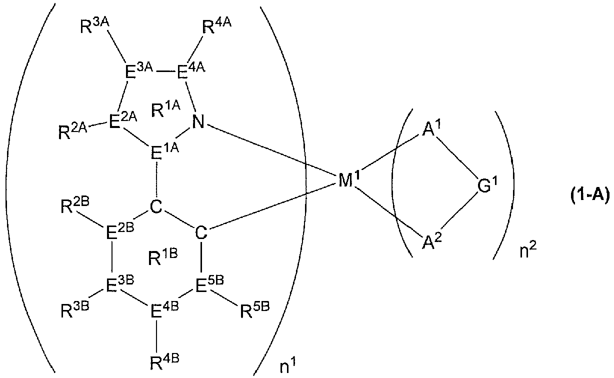 wherein,
n¹, n² and A¹-G¹-A² represent the same meaning as described above,
M¹ represents an iridium atom or a platinum atom,
E^{1A}, E^{2A}, E^{3A}, E^{4A}, E^{2B}, E^{3B}, E^{4B} and E^{5B} each independently represent a nitrogen atom or a carbon atom; when a plurality of E^{1A}, E^{2A}, E^{3A}, E^{4A}, E^{2B}, E^{3B}, E^{4B} and E^{5B} are present, they may be the same or different at each occurrence; when E^{2A}, E^{3A} and E^{4A} are nitrogen atoms, R^{2A}, R^{3A} and R^{4A} may be either present or not present; when E^{2B}, E^{3B}, E^{4B} and E^{5B} are nitrogen atoms, R^{2B}, R^{3B}, R^{4B} and R^{5B} are not present,
R^{2A}, R^{3A}, R^{4A}, R^{2B}, R^{3B}, R^{4B}, and R^{5B} each independently represent a hydrogen atom, an alkyl group, a cycloalkyl group, an alkoxy group, a cycloalkoxy group, an aryl group, an aryloxy group, a monovalent heterocyclic group, a halogen atom or dendron, and these groups optionally have a substituent; when a plurality of R^{2A}, R^{3A}, R^{4A}, R^{2B}, R^{3B}, R^{4B} and R^{5B} are present, they may be the same or different at each occurrence; R^{2A} and R^{3A}, R^{3A} and R^{4A}, R^{2A} and R^{2B}, R^{2B} and R^{3B}, R^{3B} and R^{4B}, and R^{4B} and R^{5B} may be combined together to form a ring together with the atoms to which they are attached,
the ring R^{1A} represents a triazole ring or an imidazole ring constituted of a nitrogen atom, E^{1A}, E^{2A}, E^{3A} and E^{4A}, and
the ring R^{1B} represents a benzene ring, a pyridine ring or a pyrimidine ring constituted of two carbon atoms, E^{2B}, E^{3B}, E^{4B}, and E^{5B}.

7. The light emitting device according to any one of claims 4 to 6, wherein the phosphorescent compound (G) is a phosphorescent compound represented by formula (2): 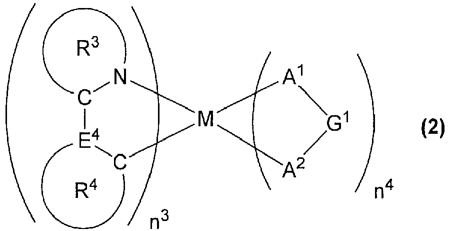 wherein,
M represents a ruthenium atom, a rhodium atom, a palladium atom, an iridium atom or a platinum atom,
n³ represents an integer of 1 or more, n⁴ represents an integer of 0 or more, n³+n⁴ is 2 or 3; when M is a ruthenium atom, a rhodium atom or an iridium atom, n³+n⁴ is 3; when M is a palladium atom or a platinum atom, n³+n⁴ is 2,
E⁴ represents a carbon atom or a nitrogen atom,
the ring R³ represents a six-membered aromatic heterocyclic ring, and this ring optionally has a substituent; when a plurality of the substituents are present, they may be the same or different, and they may be combined together to form a ring together with the atoms to which they are attached; when a plurality of rings R³ are present, they may be the same or different,
the ring R⁴ represents a five-membered or six-membered aromatic carbon ring, or a five-membered or six-membered aromatic heterocyclic ring, and these rings optionally have a substituent; when a plurality of the substituents are present, they may be the same or different, and they may be combined together to form a ring together with the atoms to which they are attached; when a plurality of rings R⁴ are present, they may be the same or different; E⁴ is a carbon atom when the ring R⁴ is a six-membered aromatic heterocyclic ring, and
A¹-G¹-A² represents an anionic bidentate ligand; A¹ and A² each independently represent a carbon atom, an oxygen atom or a nitrogen atom, and these atoms may be atoms constituting a ring; G¹ represents a single bond or an atomic group constituting the bidentate ligand together with A¹ and A²; when a plurality of A¹-G¹-A² are present, they may be the same or different.

8. The light emitting device according to any one of claims 1 to 7, wherein the constitutional unit having a cross-linkable group is a constitutional unit having at least one cross-linkable group selected from Group A of cross-linkable group:
(Group A of cross-linkable group) 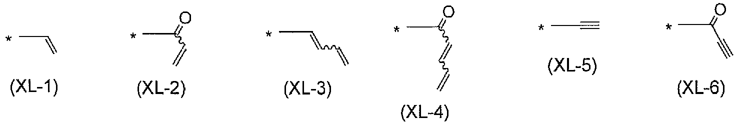 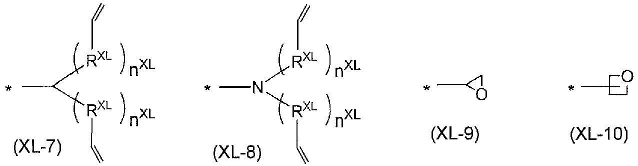 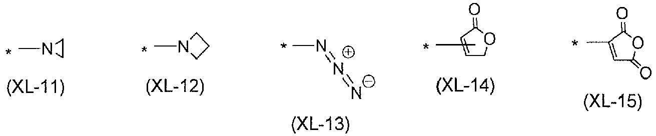 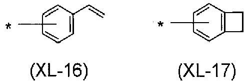 wherein,
R^{XL} represents a methylene group, an oxygen atom or a sulfur atom, and n^{XL} represents an integer of 0 to 5; when a plurality of R^{XL} are present, they may be the same or different; when a plurality of n^{XL} are present, they may be the same or different; * represents a bonding site, and these cross-linkable groups optionally have a substituent.

9. The light emitting device according to claim 8, wherein the constitutional unit having a cross-linkable group is at least one constitutional unit selected from the group consisting of a constitutional unit represented by formula (11) and a constitutional unit represented by formula (12): 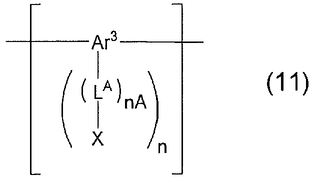 wherein,
nA represents an integer of 0 to 5, n represents 1 or 2; when a plurality of nA are present, they may be the same or different,
Ar³ represents a (n+2)-valent aromatic hydrocarbon group or a (n+2)-valent heterocyclic group, and these groups optionally have a substituent,
L^{A} represents an alkylene group, a cycloalkylene group, an arylene group, a divalent heterocyclic group, a group represented by -NR'-, an oxygen atom or a sulfur atom, and these groups optionally have a substituent; R' represents a hydrogen atom, an alkyl group, a cycloalkyl group, an aryl group or a monovalent heterocyclic group, and these groups optionally have a substituent; when a plurality of L^{A} are present, they may be the same or different, and
X represents a cross-linkable group selected from the Group A of cross-linkable group; when a plurality of X are present, they may be the same or different, 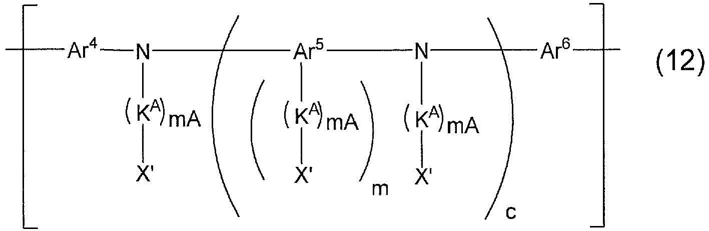
wherein,
mA represents an integer of 0 to 5, m represents an integer of 1 to 4, and c represents an integer of 0 or 1; when a plurality of mA are present, they may be the same or different,
Ar⁵ represents a (m+2)-valent aromatic hydrocarbon group, a (m+2)-valent heterocyclic group or a (m+2)-valent group in which at least one aromatic carbon ring and at least one heterocyclic ring are bonded directly to each other, and these groups optionally have a substituent,
Ar⁴ and Ar⁶ each independently represent an arylene group or a divalent heterocyclic group, and these groups optionally have a substituent,
each of Ar⁴, Ar⁵, and Ar⁶ may be bonded directly or via an oxygen atom or a sulfur atom to a group that is different from that group and that is attached to the nitrogen atom to which that group is attached, thereby forming a ring,
K^{A} represents an alkylene group, a cycloalkylene group, an arylene group, a divalent heterocyclic group, a group represented by -NR'-, an oxygen atom or a sulfur atom, and these groups optionally have a substituent; R' represents the same meaning as described above; when a plurality of K^{A} are present, they may be the same or different,
X' represents a cross-linkable group selected from the Group A of cross-linkable group, a hydrogen atom, an alkyl group, a cycloalkyl group, an aryl group or a monovalent heterocyclic group, and these groups optionally have a substituent; when a plurality of X' are present, they may be the same or different, and at least one X' is a cross-linkable group selected from the Group A of cross-linkable group.

10. The light emitting device according to any one of claims 1 to 9, wherein the phosphorescent constitutional unit is at least one constitutional unit selected from the group consisting of constitutional units represented by formula (1G), formula (2G), formula (3G) and formula (4G): 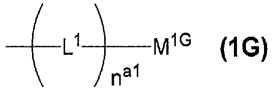 wherein,
M^{1G} represents a group obtained by removing from a phosphorescent compound one hydrogen atom bonded directly to a carbon atom or a hetero atom constituting the compound,
L¹ represents an oxygen atom, a sulfur atom, a group represented by -N(R^{A})-, a group represented by -C(R^{B})₂-, a group represented by C(R^{B})=C(R^{B})-, a group represented by-C≡C-, an arylene group or a divalent heterocyclic group, and these groups optionally have a substituent; R^{A} represents a hydrogen atom, an alkyl group, a cycloalkyl group, an aryl group or a monovalent heterocyclic group, and these groups optionally have a substituent; R^{B} represents a hydrogen atom, an alkyl group, a cycloalkyl group, an alkoxy group, a cycloalkoxy group, an aryl group or a monovalent heterocyclic group, and these groups optionally have a substituent; the plurality of R^{B} may be the same as or different, and they may be combined together to form a ring together with the carbon atoms to which they are attached; when a plurality of L¹ are present, they may be the same or different, and
n^{a1} represents an integer of 0 or more, 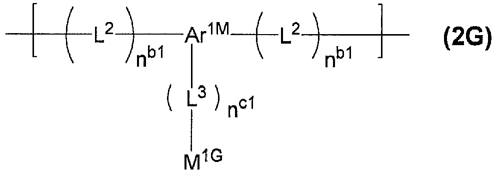
wherein,
M^{1G} represents the same meaning as described above,
L² and L³ each independently represent an oxygen atom, a sulfur atom, a group represented by -N(R^{A})-, a group represented by -C(R^{B})₂-, a group represented by - C(R^{B})=C(R^{B})-, a group represented by -C=C-, an arylene group or a divalent heterocyclic group, and these groups optionally have a substituent; R^{A} and R^{B} represent the same meaning as described above; when a plurality of L² and L³ are present, they may be the same or different at each occurrence,
n^{b1} and n^{c1} each independently represent an integer of 0 or more; the plurality of n^{b1} may be the same as or different, and
Ar^{1M} represents a trivalent aromatic hydrocarbon group or a trivalent heterocyclic group, and these groups optionally have a substituent, 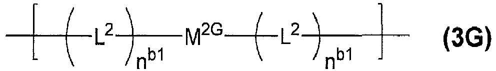
wherein,
L² and n^{b1} represent the same meaning as described above,
M^{2G} represents a group obtained by removing from a phosphorescent compound two hydrogen atoms bonded directly to carbon atoms or hetero atoms constituting the compound, 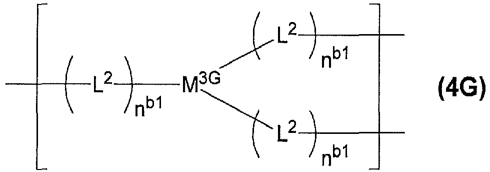
wherein,
L² and n^{b1} represent the same meaning as described above, and
M^{3G} represents a group obtained by removing from a phosphorescent compound three hydrogen atoms bonded directly to carbon atoms or hetero atoms constituting the compound.

11. The light emitting device according to any one of claims 1 to 10, wherein the polymer compound comprising the constitutional unit having a cross-linkable group and the phosphorescent constitutional unit further comprises at least one constitutional unit selected from the group consisting of a constitutional unit represented by formula (X) and a constitutional unit represented by formula (Y): 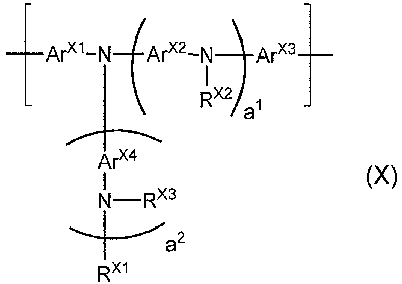 wherein,
a¹ and and a² each independently represent an integer of 0 or more,
Ar^{X1} and Ar^{X3} each independently represent an arylene group or a divalent heterocyclic group, and these groups optionally have a substituent,
Ar^{X2} and Ar^{X4} each independently represent an arylene group, a divalent heterocyclic group or a divalent group in which at least one arylene group and at least one divalent heterocyclic group are bonded directly to each other, and these groups optionally have a substituent; when a plurality of Ar^{X2} and Ar^{X4} are present, they be the same or different at each occurrence, and
R^{X1}, R^{X2} and R^{X3} each independently represent a hydrogen atom, an alkyl group, a cycloalkyl group, an aryl group or a monovalent heterocyclic group, and these groups optionally have a substituent; when a plurality of R^{X2} and R^{X3} are present, they may be the same or different at each occurrence, 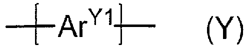
wherein,
Ar^{Y1} represents an arylene group, a divalent heterocyclic group or a divalent group in which at least one arylene group and at least one divalent heterocyclic group are bonded directly to each other, and these groups optionally have a substituent.

12. The light emitting device according to any one of claims 1 to 11, further comprising at least one layer selected from the group consisting of an electron transport layer and an electron injection layer between the second light-emitting layer and the cathode.

13. A polymer compound comprising a constitutional unit having a group represented by formula (13) and a phosphorescent constitutional unit: 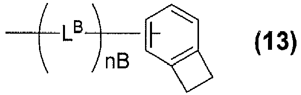 wherein,
nB represents an integer of 1 to 5,
L^{B} represents an alkylene group, a cycloalkylene group, an arylene group, a divalent heterocyclic group, a group represented by -NR'-, an oxygen atom or a sulfur atom, and these groups optionally have a substituent; R' represents a hydrogen atom, an alkyl group, a cycloalkyl group, an aryl group or a monovalent heterocyclic group, and these groups optionally have a substituent; when a plurality of L^{B} are present, they may be the same or different, and
the benzocyclobutene ring optionally has a substituent; when a plurality of the substituents are present, they may be the same or different, and they may be combined together to form a ring together with the carbon atoms to which they are attached.
